# EUROPEAN PATENT APPLICATION

(11) **EP 3 875 479 A1**
(43) Date of publication of application: **08.09.2021**
(21) Application number: 19879790.4
(22) Date of filing: 31.10.2019
(51) Int. Cl.: C07K 16/00, C07K 16/28, A61K 39/395, C12P 21/08

(54) **HOMODIMER-TYPE BISPECIFIC ANTIBODY TARGETING CD19 AND CD3, AND PREPARATION METHOD THEREFOR AND APPLICATION THEREOF**

(30) Priority: 01.11.2018 CN 201811294887
(71) Applicant: Ampsource Biopharma Shanghai Inc., Shanghai 201318 (CN)
(72) Inventor: LI, Qiang, Shanghai 201318 (CN); JIA, Shixiang, Shanghai 201318 (CN); MA, Xinlu, Shanghai 201318 (CN); ZHAO, Lili, Linyi, Shandong 273400 (CN); CUI, Xueyuan, Shanghai 201318 (CN); ZHANG, Yuhua, Shanghai 201318 (CN); LIU, Xuemei, Shanghai 201318 (CN); ZHANG, Guimin, Linyi, Shandong 273400 (CN)
(74) Representative: Prinz & Partner mbB
(86) International application number: PCT/CN2019/114808
(87) International publication number: WO 2020/088605

(57) **Abstract**

Provided is a tetravalent, homodimer-type bispecific antibody molecule that targets both immune effector cell antigen CD3 and tumor-related antigen CD19. The bispecific antibody molecule comprises first and second single-chain Fv and Fc fragments in sequence from the N-terminus to the C-terminus, wherein the first single-chain Fv can specifically bind to CD19, the second single-chain Fv can specifically bind to CD3, the first and second single-chains Fv are connected by means of a linker peptide, the second single-chain Fv and Fc fragments are directly connected to each other or connected by means of a linker peptide; and the Fc fragment does not have effector functions such as CDC, ADCC, and ADCP. The bispecific antibody can significantly inhibit or kill tumor cells, and has toxic and side effects that may be caused by excessive activation of effector cells; in addition, such bispecific antibody is of homodimer type, without the problem of heavy chain and light chain mismatch; the purification step is simple and efficient, the expression is high, and the physical and chemical properties as well as *in vivo* stability of the antibody are significantly improved.

## Description

### CROSS-REFERENCE TO RELATED APPLICATION(S)

This application claims priority to Chinese Patent Application No. 201811294887.4 filed Nov. 1, 2018, the disclosure of which is incorporated herein by reference in its entirety.

### TECHNICAL FIELD

The present disclosure relates to the field of immunology and, in particular, to an anti-CD3 bispecific antibody that mediates the killing of T cells and a use thereof, especially a use thereof for the treatment of cancer, autoimmune diseases and inflammatory diseases and immunological rejection.

### BACKGROUND

The body eliminates foreign matters through innate immunity and adaptive immunity, so as to maintain the stability of an internal environment, while adaptive immunity performs its immune function through lymphocytes, B cells and T cells. One function of B lymphocytes is to directly kill target cells through antibody-dependent cellular cytotoxicity (ADCC). Many membrane molecules on the surface of B cells, such as mlg, CD79a/CD79b, B cell receptors (BCRs), CD19, CD40, etc., play important roles in the antigen recognition, activation, proliferation, and antibody secretion of B cells. The BCR is especially necessary for the specific activation of a B cell antigen and rapidly activates a series of kinases and initiates the formation of a complex "signal body" which in turn activates downstream pathways such as controlling the growth, survival, and differentiation of the B cell. Thus, the imbalance of a steady state in BCR signal and the abnormal activation of B cells may lead to associated diseases. Many transmembrane proteins (e.g., CD19, CD20 and CD22) are specific elements that regulate BCR signal and thus become ideal targets for the treatment of functional diseases of B cells.

Cytotoxic T lymphocytes (CTLs), the most potent effector cells in an immune system, are produced after the proliferation and differentiation of initial T cells that are effectively activated after stimulated by an antigen. The antigen recognition by the initial T cells needs to be carried out through the processing and presentation of APCs, where a T-cell receptor (TCR)-CD3 complex and an MHC-antigen complex, which are distributed on the surfaces of the initial T cell and the APC respectively, bind to each other to constitute a first signal for effectively activating T cells. CD3 molecules are proteins expressed on the surface of all mature T lymphocytes and bind to TCRs to form TCR-CD3 complexes which initiate the humoral immune response to the stimulation by antigens. Thus, CD3 molecules become the most frequently used effector cells surface trigger molecules for bispecific antibodies which mediate the killing of immune cells (such as T cells and NK cells). CD3 can recruit CTL cells with killing effect. A bifunctional antibody that targets CD3 binds to a CD3 molecule on the surface of a T cell and an antigen on the surface of a target cell respectively so that the CTL is in direct contact with the target cell, so as to activate the T cell and induce the T cell to effectively kill the target cell. However, the first-generation anti-CD3 monoclonal antibody OKT3 (Kung P et al., Science, 206: 347-349, 1979) has been clinically associated with a severe "cytokine storm syndrome" due to the excessive activation of T cells and the extensive release of inflammatory factors (Hirsch R et al., J. Immunol., 142: 737-743, 1989). Therefore, how to weaken or avoid an excessive cytokine storm is the primary consideration for the development of a bifunctional antibody that targets CD3.

How to construct a product with a correct combination of heavy and light chains has always been the biggest challenge in the development of the bispecific antibody. One category of bispecific antibodies containing Fc domains has a longer half-life due to the cellular endocytosis and recycling processes mediated by FcRn; at the same time, it retains part or all of Fc-mediated effector functions such as ADCC, complement-dependent cytotoxicity (CDC) and antibody-dependent cellular phagocytosis (ADCP); and it has better solubility and stability. Therefore, such bispecific antibodies, including Triomabs, kih IgG, Crossmab, ortho-Fab IgG, DVD IgG, IgG scFv, scFv2-Fc, etc., have been widely applied to the development of bispecific antibody drugs. Current IgG-like anti-CD3 bispecific antibodies are generally monovalent in their designs mainly because bivalent anti-CD3 bispecific antibodies easily cause over-activation to induce the apoptosis of T cells and the transient release of numerous cytokines (Kuhn C et al., Immunotherapy, 8: 889-906, 2016) and may even trigger the antigen-independent activation of T cells to break an immune balance. However, while such heterodimers with asymmetric structures are expressed in hybridoma cells, generally many non-functional mismatched byproducts that are closely associated and difficult to remove are produced. Though "knobs-into-holes" and derivative techniques thereof are applied to partially solve the problem of mismatches between heavy chains of heterodimeric bispecific antibody molecules, the "mismatches between heavy chains/light chains" emerge as another challenge. A combination of "CrossMab+KiH" strategy can minimize mismatches, but a lot of genetic engineering such as mutations needs to be carried out for two antibody sequences, which cannot achieve simple and universal purposes.

In addition, an anti-CD3 bispecific antibody with an IgG-like structure may cause the unlimited activation of T cells due to its ability to bind to FcyR. Such activated T cells are found in any tissue (such as hematopoietic, lymphatic and reticuloendothelial systems) that expresses FcyR even if they are not bound to target antigens. This kind of systemic activation of T cells is accompanied by the release of numerous cytokines, leading to serious adverse effects during treatment. Therefore, the development of such anti-CD3 bispecific antibodies that mediate T-cell killing requires only the limited activation of immune effector cells in target cells.

Cluster of differentiation 19 (CD19), also known as B4 or Leu-12, is a specific marker protein on the surface of a B lymphocyte and expressed at all development stages of human B lymphocytes other than stem cells and plasma cells. CD19 forms B-cell co-receptor complexes with CD21 (CD2) and CD81 (TAPA-1), establishes B-cell signal thresholds by regulating BCR-dependent and BCR-independent signals, and plays an important role in regulating the proliferation, differentiation and signal transduction of B cells. The regulation of CD19 complexes on the activation and proliferation of B cells is accomplished by cross-linking another two B cell signal transduction complexes, and the corresponding enhancement or inhibitory effects are achieved depending on the amount of cross-linking. CD19 antigens are widely expressed in B lymphocytic malignant tumors which include, for example, acute/chronic lymphocytic leukemia (ALL/CLL), non-Hodgkin lymphoma (NHL), melanoma, myeloma (Nicholson IC et al., Mol. Immun., 34: 1157-1165, 1997; Nigel PM et al., eCancer Med. Sci., 11: 720, 2017; Grossbard ML et al, Br. J. Haematol., 102: 509-15, 1998). In addition, CD19 antigens are also found to be associated with immune system diseases (such as rheumatoid arthritis and multiple sclerosis) (Kunz M et al., Mediators Inflamm., 2009; 2009: 979258. doi: 10.1155/2009/979258) and immunological rejection (US 926, 0530), are exposed only on the surface of abnormal cells, and can be used as ideal targets for the diagnosis, prognosis, and treatment of associated diseases. Therefore, a bispecific antibody against CD19 and CD3 with superior performance in the aspects of a half-life, stability, safety and productibility is to be developed, which has therapeutic benefits in many diseases depending on B cell activation (such as autoimmune diseases or malignant tumors) and transplant rejection.

### SUMMARY

An object of the present disclosure is to provide a tetravalent, homodimer-type bispecific antibody molecule targeting immune effector cell antigen CD3 and tumor-associated antigen (TAA) CD19. The bispecific antibody can significantly inhibit or kill tumor cells *in vivo,* and has significantly reduced non-specific killing effect for normal cells with the low expression of TAA, controlled toxic side effects due to the overactivation of effector cells and significantly improved physicochemical and *in vivo* stability.

In an aspect of the present disclosure, there is provided a bispecific antibody against CD19, which is a tetravalent homodimer formed by two identical polypeptide chains that bind to each other by a covalent bond, wherein each of the polypeptide chains includes a first single-chain Fv that specifically binds to tumor-associated antigen CD19 (anti-TAA scFv), a second single-chain Fv that specifically binds to effector cell antigen CD3 (anti-CD3 scFv) and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions.

The first single-chain Fv has specificity to the tumor-associated antigen and includes a VH domain and a VL domain linked by a linker peptide (L1), wherein VH, L1 and VL are arranged in order of VH-L1-VL or VL-L1-VH and the linker peptide L1 has an amino acid sequence of (GGGGX)ₙ, wherein X includes Ser or Ala, preferably Ser; and n is a natural number from 1 to 5, preferably 3;
Exemplarily, the tumor-associated antigen is CD19, including, but not limited to, any variant, isotype, derivative and species homolog of CD19; preferably, CD19 is derived from a human, a cynomolgus monkey or a rhesus monkey.

For example, some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the first single-chain Fv against CD19 are listed in Table 1-1 of the present disclosure.

Preferably, the first single-chain Fv specifically binds to CD19 and contains CDR1, CDR2 and CDR3 selected from the group consisting of:
(i) HCDR1, HCDR2 and HCDR3, contained in a VH domain, that are as shown in SEQ ID NOs: 1, 2 and 3, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 1, 2 and 3; and LCDR1, LCDR2 and LCDR3, contained in a VL domain, that are as shown in SEQ ID NOs: 4, 5 and 6, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 4, 5 and 6;
(ii) HCDR1, HCDR2 and HCDR3, contained in a VH domain, that are as shown in SEQ ID NOs: 9, 10 and 11, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 9, 10 and 11; and LCDR1, LCDR2 and LCDR3, contained in a VL domain, that are as shown in SEQ ID NOs: 12, 13 and 14, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 12, 13 and 14;
(iii) HCDR1, HCDR2 and HCDR3, contained in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, contained in a VL domain, that are as shown in SEQ ID NOs: 20, 13 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 20, 13 and 21;
(iv) HCDR1, HCDR2 and HCDR3, contained in a VH domain, that are as shown in SEQ ID NOs: 24, 25 and 26, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 24, 25 and 26; and LCDR1, LCDR2 and LCDR3, contained in a VL domain, that are as shown in SEQ ID NOs: 27, 28 and 29, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 27, 28 and 29; and
(v) HCDR1, HCDR2 and HCDR3, contained in a VH domain, that are as shown in SEQ ID NOs: 17, 18 and 19, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, contained in a VL domain, that are as shown in SEQ ID NOs: 32, 33 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 32, 33 and 21.

More preferably, the first single-chain Fv specifically binds to CD19 and is selected from the group consisting of:
(i) a VH domain comprising an amino acid sequence as shown in SEQ ID NO: 7 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 7; and a VL domain comprising an amino acid sequence as shown in SEQ ID NO: 8 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 8;
(ii) a VH domain comprising an amino acid sequence as shown in SEQ ID NO: 15 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 15; and a VL domain comprising an amino acid sequence as shown in SEQ ID NO: 16 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 16;
(iii) a VH domain comprising an amino acid sequence as shown in SEQ ID NO: 22 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 22; and a VL domain comprising an amino acid sequence as shown in SEQ ID NO: 23 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 23;
(iv) a VH domain comprising an amino acid sequence as shown in SEQ ID NO: 30 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 30; and a VL domain comprising an amino acid sequence as shown in SEQ ID NO: 31 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 31;
(v) a VH domain comprising an amino acid sequence as shown in SEQ ID NO: 34 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 34; and a VL domain comprising an amino acid sequence as shown in SEQ ID NO: 35 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 35;
(vi) a VH domain comprising an amino acid sequence as shown in SEQ ID NO: 36 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 36; and a VL domain comprising an amino acid sequence as shown in SEQ ID NO: 37 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 37; and
(vii) a VH domain comprising an amino acid sequence as shown in SEQ ID NO: 38 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 38; and a VL domain comprising an amino acid sequence as shown in SEQ ID NO: 39 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 39.

The linker peptide (L2) that links the first single-chain Fv and the second single-chain Fv of the present disclosure consists of a flexible peptide and a rigid peptide.

Further, the flexible peptide includes two or more amino acids, and preferably selected from the following amino acids: Gly(G), Ser(S), Ala(A) and Thr(T). More preferably, the flexible peptide includes G and S residues. Most preferably, an amino acid composition structure of the flexible peptide has a general formula of GₓS_{y}(GGGGS)_{z}, wherein x, y and z are integers greater than or equal to 0 and x + y + z ≥ 1. For example, in a preferred embodiment, the amino acid sequence of the flexible peptide is G₂(GGGGS)₃.

Further, the rigid peptide is derived from a full-length sequence (as shown in SEQ ID NO: 48) consisting of amino acids 118 to 145 at carboxyl-terminus of natural human chorionic gonadotropin β-subunit or a truncated fragment thereof (hereinafter collectively referred to as CTP). Preferably, the CTP rigid peptide includes 10 amino acids at N-terminus of SEQ ID NO: 48, that is, SSSSKAPPPS (CTP¹); or the CTP rigid peptide includes 14 amino acids at C-terminus of SEQ ID NO: 48, that is, SRLPGPSDTPILPQ (CTP²); as another example, in another embodiment, the CTP rigid peptide includes 16 amino acids at the N-terminus of SEQ ID NO: 48, that is, SSSSKAPPPSLPSPSR (CTP³); for another example, in some other embodiments, the CTP rigid peptide includes 28 amino acids which begin at position 118 and end at position 145 of the human chorionic gonadotropin β-subunit, that is, SSSSKAPPPSLPSPSRLPGPSDTPILPQ (CTP⁴).

For example, some preferred amino acid sequences of the linker peptide L2 that links the first single-chain Fv and the second single-chain Fv are exemplarily listed in Table 1-2 of the present disclosure.

In a preferred embodiment of the present disclosure, the linker peptide has an amino acid sequence as shown in SEQ ID NO: 49, wherein the amino acid composition of the flexible peptide is G₂(GGGGS)₃, and the amino acid composition of the rigid peptide is SSSSKAPPPS (i.e. CTP¹).

The second single-chain Fv has specificity to immune effector cell antigen CD3 and includes a VH domain and a VL domain linked by a linker peptide (L3), wherein VH, L3 and VL are arranged in order of VH-L3-VL or VL-L3-VH and the linker peptide L3 has an amino acid sequence of (GGGGX)ₙ, wherein X includes Ser or Ala, preferably Ser; and n is a natural number from 1 to 5, preferably 3;

Preferably, the second single-chain Fv of the bispecific antibody binds to an effector cell at an EC₅₀ value greater than about 10 nM, or greater than 20 nM, or greater than 40 nM, or greater than about 50 nM in an *in vitro* FACS binding assay; more preferably, the second single-chain Fv of the bispecific antibody is capable of binding to human CD3 and specifically binding to CD3 of a cynomolgus monkey or a rhesus monkey. In a preferred embodiment of the present disclosure, the bispecific antibody specifically binds to effector cells at an EC₅₀ value of 15.69 nM.

Preferably, the second single-chain Fv specifically binds to CD3; the VH domain of the second single-chain Fv contains HCDR1, HCDR2 and HCDR3 as shown in SEQ ID NOs: 40, 41 and 42, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 40, 41 and 42; and the VL domain of the second single-chain Fv contains LCDR1, LCDR2 and LCDR3 as shown in SEQ ID NOs: 43, 44 and 45, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 43, 44 and 45.

More preferably, the second single-chain Fv specifically binds to CD3; the VH domain of the second single-chain Fv contains an amino acid sequence as shown in SEQ ID NO: 46 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 46; and the VL domain of the second single-chain Fv contains an amino acid sequence as shown in SEQ ID NO: 47 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 47.

The Fc fragment of the present disclosure is linked to the second single-chain Fv directly or by the linker peptide L4, and the linker peptide L4 includes 1-20 amino acids, and preferably selected from the following amino acids: Gly(G), Ser(S), Ala(A) and Thr(T); more preferably, the linker peptide L4 is selected from Gly(G) and Ser(S); further preferably, the linker peptide L4 consists of (GGGGS)n, wherein n = 1, 2, 3 or 4. In a preferred embodiment of the present disclosure, the Fc fragment is directly linked to the second single-chain Fv.

In another aspect, the Fc fragment of the present disclosure includes a hinge region, a CH2 domain and a CH3 domain from a human immunoglobulin heavy chain constant region. For example, in some embodiments, the Fc fragment of the present disclosure is selected from, for example, heavy chain constant regions of human IgG1, IgG2, IgG3, IgG4, IgM, IgA1, IgA2, IgD and IgE; particularly selected from heavy chain constant regions of human IgG1, IgG2, IgG3 and IgG4, and more particularly selected from a heavy chain constant region of human IgG1 or IgG4; and the Fc fragment has one or more amino acid substitutions, deletions or additions (for example, at most 20, at most 15, at most 10, or at most 5 substitutions, deletions, or additions) than a natural sequence from which the Fc fragment is derived.

In some preferred embodiments, the Fc fragment is changed, for example, mutated to modify the properties of the bispecific antibody molecule of the present disclosure (for example, to change one or more of the following properties: Fc receptor binding, antibody glycosylation, an effector cell function or a complement function).

For example, the bispecific antibody provided by the present disclosure includes an Fc variant containing amino acid substitutions, deletions or additions that change (for example, reduce or eliminate) effector functions. Fc region of the antibody mediates several important effector functions such as ADCC, ADCP and CDC. Methods for changing the affinity of the antibody to an effector ligand (such as FcyR or a complement C1q) by substituting amino acid residues in the Fc region of the antibody to change the effector functions are known in the art (see, for example, EP388151A1; US5648260; US5624821; Natsume A et al., Cancer Res., 68: 3863-3872, 2008; Idusogie EE et al., J. Immunol., 166: 2571-2575, 2001; Lazar GA et al., PNAS, 103: 4005-4010, 2006; Shields RL et al., JBC, 276: 6591-6604, 2001; Stavenhagen JB et al., Cancer Res., 67: 8882-8890, 2007; Stavenhagen JB et al., Advan. Enzyme. Regul., 48: 152-164, 2008; Alegre ML et al., J. Immunol., 148: 3461-3468, 1992; Kaneko E et al., Biodrugs, 25: 1-11, 2011). In some preferred embodiments of the present disclosure, amino acid L235 (EU numbering) in the constant region of the antibody is modified to change an interaction with an Fc receptor, such as L235E or L235A. In some other preferred embodiments, amino acids 234 and 235 in the constant region of the antibody are modified simultaneously, such as L234A and L235A (L234A/L235A) (EU numbering).

For example, the bispecific antibody provided by the present disclosure may include an Fc variant containing amino acid substitutions, deletions or additions that extend a circulating half-life. Studies show that M252Y/S254T/T256E, M428L/N434S or T250Q/M428L can extend the half-life of the antibody in primates. For more mutation sites included in the Fc variant with enhanced binding affinity to a neonatal receptor (FcRn), see Chinese invention patent CN 201280066663.2, US 2005/0014934A1, WO 97/43316, US 5,869,046, US 5,747,03 and WO 96/32478. In some preferred embodiments of the present disclosure, amino acid M428 (EU numbering) in the constant region of the antibody is modified to enhance the binding affinity to the FcRn receptor, such as M428L. In some other preferred embodiments, amino acids 250 and 428 (EU numbering) in the constant region of the antibody are modified simultaneously, such as T250Q and M428L (T250Q/M428L).

For example, the bispecific antibody provided by the present disclosure may also include an Fc variant containing amino acid substitutions, deletions or additions that can reduce or eliminate Fc glycosylation. For example, the Fc variant contains reduced glycosylation of the N-linked glycan normally present at amino acid site 297 (EU numbering). The glycosylation at position N297 has a great effect on the activity of IgG. If the glycosylation at this position is eliminated, the conformation of the upper half of CH2 of an IgG molecule is affected, thus losing the ability of binding to FcyRs and affecting the biological activity related to the antibody. In some preferred embodiments of the present disclosure, amino acid N297 (EU numbering) in the constant region of human IgG is modified to avoid the glycosylation of the antibody, such as N297A.

For example, the bispecific antibody provided by the present disclosure may also include an Fc variant containing amino acid substitutions, deletions or additions that eliminate charge heterogeneity. Various post-translational modifications during expression in engineered cells will cause the charge heterogeneity of monoclonal antibodies. The heterogeneity of lysine at C-terminus of an IgG antibody is one of the main reasons for charge heterogeneity. Lysine at C-terminus of a heavy chain may be deleted at a certain proportion during the production of the antibody, resulting in charge heterogeneity and affecting the stability, effectiveness, immunogenicity or pharmacokinetic of the antibody. In some preferred embodiments of the present disclosure, K447 (EU numbering) at the C-terminus of the IgG antibody is removed or deleted to eliminate the charge heterogeneity of the antibody and improve the homogeneity of the expressed product.

Some preferred amino acid sequences of the Fc fragment are exemplarily listed in Table 1-3 of the present disclosure. Compared with a bispecific antibody containing the Fc region of wild-type human IgG, the bispecific antibody provided by the present disclosure contains an Fc fragment that exhibits reduced affinity to at least one of human FcyRs (FcγRI, FcγRIIa, or FcγRIIIa) and C1q and has reduced effector cell functions or complement functions. For example, in a preferred embodiment of the present disclosure, the bispecific antibody includes an Fc fragment that is derived from human IgG1, has L234A and L235A substitutions (L234A/L235A), and exhibits reduced binding ability to FcγRI. In addition, the Fc fragment included in the bispecific antibody provided by the present disclosure may also contain amino acid substitutions that change one or more other characteristics (such as an ability of binding to the FcRn receptor, the glycosylation of the antibody or the charge heterogeneity of the antibody). For example, in a preferred embodiment of the present disclosure, the Fc fragment has an amino acid sequence as shown in SEQ ID NO: 54, which has amino acid substitutions L234A/L235A/T250Q/N297A/P331 S/M428L and a deleted or removed K447 compared with the natural sequence from which the Fc fragment is derived.

In a preferred embodiment of the present disclosure, the bispecific antibody binds to human CD19 and CD3 and has an amino acid sequence as follows:
(i) a sequence as shown in SEQ ID NO: 55;
(ii) a sequence having one or more substitutions, deletions or additions (such as 1, 2, 3, 4 or 5 substitutions, deletions or additions) relative to the sequence as shown in SEQ ID NO: 55; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 55.

In some preferred embodiments, the substitutions in (ii) are conservative substitutions.

Preferably, the bispecific antibody has an amino acid sequence as follows:
(i) a sequence as shown in SEQ ID NO: 57;
(ii) a sequence having one or more substitutions, deletions or additions (such as 1, 2, 3, 4 or 5 substitutions, deletions or additions) relative to the sequence as shown in SEQ ID NO: 57; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 57.

In some preferred embodiments, the substitutions in (ii) are conservative substitutions.

Preferably, the bispecific antibody has an amino acid sequence as follows:
(i) a sequence as shown in SEQ ID NO: 59;
(ii) a sequence having one or more substitutions, deletions or additions (such as 1, 2, 3, 4 or 5 substitutions, deletions or additions) relative to the sequence as shown in SEQ ID NO: 59; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 59.

In some preferred embodiments, the substitutions in (ii) are conservative substitutions.

Preferably, the bispecific antibody has an amino acid sequence as follows:
(i) a sequence as shown in SEQ ID NO: 61;
(ii) a sequence having one or more substitutions, deletions or additions (such as 1, 2, 3, 4 or 5 substitutions, deletions or additions) relative to the sequence as shown in SEQ ID NO: 61; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 61.

In some preferred embodiments, the substitutions in (ii) are conservative substitutions.

In another aspect of the present disclosure, there is provided a DNA molecule encoding the bispecific antibody as described above.

In a preferred embodiment of the present disclosure, the DNA molecule encoding the bispecific antibody as described above has a nucleotide sequence as shown in SEQ ID NO: 56. In a preferred embodiment of the present disclosure, the DNA molecule encoding the bispecific antibody as described above has a nucleotide sequence as shown in SEQ ID NO: 58.

In a preferred embodiment of the present disclosure, the DNA molecule encoding the bispecific antibody as described above has a nucleotide sequence as shown in SEQ ID NO: 60.

In a preferred embodiment of the present disclosure, the DNA molecule encoding the bispecific antibody as described above has a nucleotide sequence as shown in SEQ ID NO: 62.

In another aspect of the present disclosure, there is provided a vector including the DNA molecule as described above.

In another aspect of the present disclosure, there is provided a host cell including the vector as described above; the host cell includes a prokaryotic cell, a yeast or a mammalian cell, such as a CHO cell, an NS0 cell or another mammalian cell, preferably a CHO cell.

In another aspect of the present disclosure, there is provided a pharmaceutical composition including the bispecific antibody or the DNA molecule or the nucleotide sequence as described above and a pharmaceutically acceptable excipient, carrier or diluent.

Preferably, the pharmaceutical composition further includes an additional pharmaceutically active agent.

Preferably, the additional pharmaceutically active agent is a drug for treating an immune-related disease.

Preferably, the additional pharmaceutically active agent is a drug having antitumor activity.

Preferably, the additional pharmaceutically active agent is a drug for treating an autoimmune disease or an inflammatory disease.

Preferably, the additional pharmaceutically active agent is a drug for treating a disease or disorder related to transplant rejection.

Preferably, the bispecific antibody or the DNA molecule or the nucleotide sequence described above and the additional pharmaceutically active agent are provided as separate components or as components of the same composition.

Preferably, a pharmaceutically active agent is administered before, after or while the pharmaceutical composition is administered to a subject, wherein the pharmaceutically active agent is selected from an antibody, an antibody fragment, a drug, an enzyme, a cytotoxic agent, a toxin, an antibiotic, a hormone, an immunomodulator, a cytokine, a chemokine or a radioisotope.

Preferably, the drug in the pharmaceutical composition is selected from the group consisting of cyclophosphamide, nimustine, amethopterin, fluorouracil, capecitabine, gemcitabine, tigio (tegafur-gimeracil-oteracil potassium), pemetrexed, fludarabine, doxorubicin, bleomycin, vinorelbine, paclitaxel, docetaxel, irinotecan, tamoxifen, letrozole, exemestane, fulvestrant, goserelin, medroxyprogesterone, cisplatin, carboplatin, oxaliplatin, nedaplatin, oxaliplatin, asparaginase, lobaplatin, etoposide, vincristine, irinotecan, tegafur, dacarbazine, mitomycin, teniposide, pirarubicin, mitoxantrone, vindesine, raltitrexed, methotrexate, cisplatin bleomycin sulfate, carmustine, chlorambucil, cyclophosphamide hydroxyurea and daunomycin.

Preferably, the toxin in the pharmaceutical composition is selected from the group consisting of ricin, abrin, α toxin, saponin, ribonuclease (RNase), DNase I, staphylococcal enterotoxin-A, pokeweed antiviral protein, gelonin, diphtheria toxin, Pseudomonas exotoxin and Pseudomonas endotoxin.

Preferably, the immunomodulator in the pharmaceutical composition is selected from the group consisting of a cytokine, a chemokine, a stem cell growth factor, a lymphotoxin, a colony-stimulating factor (CSF), an interleukin (IL), an erythropoietin, a platelet growth factor, a tumor necrosis factor (TNF), a granulocyte-colony stimulating factor (G-CSF), a granulocyte macrophage-colony stimulating factor (GM-CSF), interferon-a, interferon-β, interferon-γ or interferon-A, TGF-α, TGF-β, interleukin-1 (IL-1), IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-23, IL-25, LIF, FLT-3, a vascular endothelial growth factor, a thrombospondin and an endostatin.

Preferably, the pharmaceutical composition may be applied in combination with one or more additional therapies, wherein the additional therapies are selected from the group consisting of a surgery, chemotherapy, radiotherapy, immunotherapy, gene therapy, DNA therapy, RNA therapy, nano-therapy, viral therapy, adjuvant therapy and a combination thereof.

In another aspect of the present disclosure, there is provided a method for enhancing or stimulating an immune response or function, comprising administering a therapeutically effective amount of the bispecific antibody or the DNA or the nucleotide sequence or the pharmaceutical composition to an individual.

In another aspect of the present disclosure, there is provided a method for treating, preventing or ameliorating an immune disorder or disease in a cell, tissue, organ or animal, comprising administering a therapeutically effective amount of the bispecific antibody or the DNA or the nucleotide sequence or the pharmaceutical composition to an individual.

In another aspect of the present disclosure, there is provided a method for preventing/treating, delaying development of, or reducing/inhibiting recurrence of a disease including an immune-related disease, a tumor, an autoimmune disease, an inflammatory disease or a transplant rejection-related disease or disorder, comprising administering an effective amount of the bispecific antibody or the DNA or the nucleotide sequence or the pharmaceutical composition to an individual suffering from the disease or disorder.

Preferably, the method may be used in combination with one or more additional therapies, wherein the additional therapies are selected from the group consisting of a surgery, chemotherapy, radiotherapy, immunotherapy, gene therapy, DNA therapy, RNA therapy, nanotherapy, viral therapy, adjuvant therapy and a combination thereof.

In another aspect of the present disclosure, there is further provided a method for preparing the bispecific antibody of the present disclosure, comprising: (a) obtaining a fusion gene of the bispecific antibody, and constructing an expression vector of the bispecific antibody; (b) transfecting the expression vector into a host cell by a genetic engineering method; (c) culturing the host cell under conditions that allow the bispecific antibody to be produced; (d) separating and purifying the generated antibody.

The expression vector in step (a) is one or more selected from a plasmid, a bacterium and a virus, preferably the expression vector is PCDNA3.1.

The host cell into which the constructed vector is transfected by the genetic engineering method in step (b) includes a prokaryotic cell, a yeast or a mammalian cell, such as a CHO cell, an NS0 cell or another mammalian cell, preferably a CHO cell.

The bispecific antibody is separated and purified in step (d) by a conventional immunoglobulin purification method including protein A affinity chromatography and ion exchange, hydrophobic chromatography or molecular sieve.

In another aspect of the present disclosure, there is provided use of the bispecific antibody or the DNA or the nucleotide sequence for preparing a drug for treating, preventing or alleviating a tumor; wherein examples of the tumor include, but are not limited to, acute myeloid leukemia (AML), chronic myeloid leukemia (CML), B acute lymphocytic leukemia (B-ALL), B chronic lymphocytic leukemia (B-CLL), B-cell lymphoma (BCL), T-cell lymphoma (TCL) (such as skin), myelodysplastic syndrome (MDS), small lymphocytic lymphoma (SLL), hairy cell leukemia (HCL), marginal zone lymphoma (MZL) (such as extranodal or splenic), follicular lymphoma (FL) (such as pediatric or gastrointestinal), B-cell prolymphocytic leukemia (B-PLL), mantle cell lymphoma (MCL), lymphoplasmacytic lymphoma (LPL)/Waldenstrom's macroglobulinemia (WM), lymphoblastic leukemia (ALL) (such as B cell), lymphoblastic lymphoma (LBL) (such as B cell), plasmablastic lymphoma (PBL) (such as B cell), Hodgkin's lymphoma, non-Hodgkin's lymphoma, diffuse large B-cell lymphoma (DLBCL) (for example, primary or inflammation-related), Burkitt's lymphoma (BL), multiple myeloma, anaplastic large-cell lymphoma and HIV-related lymphoma.

In another aspect of the present disclosure, there is provided use of the bispecific antibody or the DNA or the nucleotide sequence for preparing a drug for treating, preventing or alleviating an autoimmune or inflammatory disease, wherein the autoimmune or inflammatory disease is selected from rheumatoid arthritis (RA), osteoarthritis, reactive arthritis, systemic lupus erythematosus (SLE), Crohn's disease, multiple sclerosis, scleroderma, psoriasis, psoriatic arthritis, ulcerative colitis (such as chronic), insulin-dependent diabetes (such as juvenile), thyroiditis (such as chronic), hyperthyroidism, asthma, allergic diseases, sarcoidosis, autoimmune hemolytic anemia, pernicious anemia, graft-versus-host disease, dermatomyositis, chronic hepatitis, microscopic renal vasculitis, chronic active hepatitis, uveitis, intestinal synovitis, autoimmune intestinal disease, idiopathic leukopenia, autoimmune glomerulonephritis, autoimmune hemolytic anemia, autoimmune hepatitis, interstitial pneumonia, chronic pemphigus, pemphigus vulgaris, arteritis, polyarteritis nodosa and ankylosing spondylitis.

In another aspect of the present disclosure, there is provided use of the bispecific antibody or the DNA or the nucleotide sequence for preparing a drug for treating, preventing or alleviating a disease or disorder associated with transplant rejection, where the transplant rejection includes acute, hyperacute or chronic transplant rejection and the transplant rejection includes rejection to an organ, tissue or cell transplant which includes, but is not limited to, blood transfusion, a vascular transplant, an epithelial transplant, an endothelial transplant, a muscle transplant, a connective tissue transplant, a joint transplant, a heart transplant, a lung transplant, a liver transplant, a kidney transplant, a pancreas transplant, a skin transplant, an intestinal transplant, a corneal transplant, a bone transplant, a bone marrow transplant, a graft-versus-host disease or a host-versus-graft disease.

In another aspect of the present disclosure, there is provided a method for detecting the presence of cancer in a mammal, comprising: (a) contacting a sample including one or more cells derived from the mammal with the bispecific antibody or the DNA or the nucleotide sequence to form a complex; and
(b) detecting the complex, where the presence of the complex indicates the presence of cancer in the mammal.

In another aspect of the present disclosure, there is provided a kit including the bispecific antibody or the DNA or the nucleotide sequence of the present disclosure.

In another aspect of the present disclosure, there is further provided a monoclonal antibody binding to CD19 or an antigen-binding fragment thereof, wherein a variable region of the monoclonal antibody is derived from the first single-chain Fv of the bispecific antibody and CDR1, CDR2 and CDR3 contained therein are selected from the group consisting of:
(i) HCDR1, HCDR2 and HCDR3, contained in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, contained in a VL domain, that are as shown in SEQ ID NOs: 32, 33 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 32, 33 and 21; and
(ii) HCDR1, HCDR2 and HCDR3, contained in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, contained in a VL domain, that are as shown in SEQ ID NOs: 20, 13 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 20, 13 and 21.

Preferably, the monoclonal antibody is a humanized antibody or a fully human antibody.

Preferably, the antigen-binding fragment is selected from scFv, Fab, Fab', (Fab')2, an Fv fragment or Fv linked by a disulfide bond (dsFv).

More preferably, the monoclonal antibody binding to CD19 or the antigen-binding fragment thereof comprises a VH domain and a VL domain selected from the group consisting of:
(i) a VH domain comprising an amino acid sequence as shown in SEQ ID NO: 36 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 36; and a VL domain comprising an amino acid sequence as shown in SEQ ID NO: 37 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 37; and
(ii) a VH domain comprising an amino acid sequence as shown in SEQ ID NO: 38 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 38; and a VL domain comprising an amino acid sequence as shown in SEQ ID NO: 39 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 39.

The technical solutions provided by the present disclosure have beneficial effects summarized as follows:
1. The bispecific antibody provided by the present disclosure includes anti-TAA scFvs located at the N-terminus of the bispecific antibody and having changed spatial conformation, so that the bispecific antibody has reduced binding ability to the TAA under some conditions, especially that the bispecific antibody is difficult to bind to normal cells with the weak expression or low expression of TAAs, thereby exhibiting reduced non-specific killing effect. However, the binding specificity to cells with over-expression or high expression of TAAs is not significantly reduced, and the bispecific antibody exhibits a good killing effect *in vivo.* It can be known that when a target antigen is merely expressed on tumor cells or the bispecific antibody of the present disclosure specifically binds to only tumor cells over-expressing the target antigen, immune effector cells are activated restrictively and merely in target cell tissues, which can minimize the non-specific killing of the bispecific antibody on normal cells and the accompanying release of cytokines and reduce the toxic side effects of the bispecific antibody in clinical treatment.
2. The anti-CD3 scFv selected by the bispecific antibody provided by the present disclosure specifically binds to effector cells with weak binding affinity (EC₅₀ value greater than about 10 nM, or greater than 20 nM, or greater than 40 nM, or greater than about 50 nM). In addition, the anti-CD3 scFv is embedded between the anti-TAA scFv and Fc and the CTP rigid peptide contained in the linker peptide L3 at its N-terminus and the Fc fragment located at its C-terminus partially "cover" or "shield" the antigen-binding domain of the anti-CD3 scFv. Such steric hindrance effect makes the anti-CD3 scFv bind to CD3 with weaker binding affinity (for example, greater than 1 µM), which reduces its ability to activate and stimulate T cells, limits the excessive release of cytokines, and provides higher safety. In addition, the anti-CD3 scFv used in the present disclosure can bind to CD3 natural antigens from human and cynomolgus monkeys and/or rhesus monkeys at the same time so that no alternative molecule needs to be constructed for preclinical toxicology evaluation and the effective dose, toxic dose and toxic side effects obtained are more objective and accurate and can be directly converted into a clinical dose to reduce the risk of clinical studies. Further, the bispecific antibody provided by the present disclosure creatively adopts a divalent anti-CD3 scFv, which avoids the asymmetric structure of a heterodimer (including a monovalent anti-CD3 scFv) commonly used in the existing art in terms of the configuration design of the bispecific antibody, and solves the problem of heavy chain mismatches, thereby simplifying downstream purification steps. Moreover, unexpectedly, the non-specific binding of the anti-CD3 scFv to T cells is not observed in an *in vitro* cell binding assay, and the degree of cell activation (the release of cytokines such as IL-2) is controlled within a safe and effective range. That is, the bivalent anti-CD3 scFv structure used in the present disclosure has not induced over-activation of T cells in a non-antigen-dependent manner, whereas for other bispecific antibodies including bivalent anti-CD3 domains, the uncontrollable over-activation of T cells is common and thus anti-CD3 bispecific antibodies are generally designed to avoid the introduction of a bivalent anti-CD3 structure.
3. The modified Fc fragment included in the bispecific antibody provided by the present disclosure has no ability of binding to FcyR, avoiding the systemic activation of T cells mediated by FcyR and allowing immune effector cells to be activated restrictively and merely in target cell tissues.
4. The bispecific antibody provided by the present disclosure is homodimeric without mismatches of heavy chains and light chains. The bispecific antibody is produced by a stable downstream process and purified by simple and efficient steps, with a homogeneous expression product and significantly improved physicochemical and *in vivo* stability.
5. The bispecific antibody provided by the present disclosure has a relatively long *in vivo* circulating half-life due to the inclusion of the Fc fragment.

### Detailed description

### Abbreviations and definitions

BiAb Bispecific antibody
CDR Complementarity determining region in a variable region of an immunoglobulin, defined by a Kabat numbering system
EC₅₀ A concentration at which 50% efficacy or binding is generated
ELISA Enzyme-linked immunosorbent assay
FR Framework region of an antibody: a variable region of an immunoglobulin excluding CDRs
HRP Horseradish peroxidase
IL-2 Interleukin 2
IFN Interferon
IC₅₀ A concentration at which 50% inhibition is generated
IgG Immunoglobulin G
Kabat Immunoglobulin comparison and numbering system advocated by Elvin A Kabat
mAb Monoclonal antibody
PCR Polymerase chain reaction
V region IgG chain fragment whose sequence is variable for different antibodies; the V region extends to Kabat residue 109 of a light chain and residue 113 of a heavy chain.
VH Heavy chain variable region of an immunoglobulin
VK κ light chain variable region of an immunoglobulin
K_{D} Equilibrium dissociation constant
kₐ Association rate constant
k_{d} Dissociation rate constant

In the present disclosure, unless otherwise specified, the scientific and technical terms used herein have meanings generally understood by those skilled in the art. The antibody or fragments thereof used in the present disclosure may be further modified using conventional techniques known in the art alone or in combination, such as amino acid deletion, insertion, substitution, addition, and/or recombination and/or other modification methods. A method for introducing such modifications into a DNA sequence of an antibody according to the amino acid sequence of the antibody is well known to those skilled in the art. See, for example, Sambrook, Molecular Cloning: A Laboratory Manual, Cold Spring Harbor Laboratory (1989), N.Y. Such modifications are preferably performed at a nucleic acid level. Meanwhile, for a better understanding of the present disclosure, the definitions and explanations of related terms are provided below.

"CD19", as known as Cluster of Differentiation 19 polypeptide, is a single channel Type I transmembrane glycoprotein with two C2-set Ig-like (immunoglobulin-like) domains and a relatively large cytoplasmic tail that is highly conserved among mammalian species. CD19 is expressed in almost all B lineage cells and follicular cells and plays an indispensable role in B lymphocyte differentiation. It works with CD21, CD81, and CD225 as the B cell key co-receptor. Therefore, CD19 acts as a biomarker for B lymphocyte development, B-cell lymphoma diagnosis, and B-lymphoblastic leukemia diagnosis. In addition, mutations in CD19 are associated with severe immunodeficiency syndromes. Indications for CD19 targets also include other related diseases or disorders found in the existing art or about to be discovered in the future. The term also includes any variants, isotypes, derivatives, and species homologues of CD19 that are naturally expressed by cells including tumor cells or expressed by cells transfected with CD19 genes or cDNA.

CD3 molecule is an important differentiation antigen on the T cell membrane and a characteristic marker of mature T cells. It is composed of six peptide chains, and these chains are associated with the T cell antigen receptor (TCR) with a non-covalent bond to constitute a TCR-CD3 complex. CD3 molecule not only participates in the intracytoplasmic assembly of the TCR-CD3 complex but also transmits antigen stimulation signals through the immunoreceptor tyrosine-based activation motif (ITAM) of the cytoplasmic regions of polypeptide chains. The main functions of CD3 molecule are to stabilize TCR structure and transmit T cell activation signal. When TCR specifically recognizes and binds to the antigen, CD3 is involved in signal transduction into T cell cytoplasm as the first signal to induce T cell activation and plays a very important role in T cell antigen recognition and immune response generation.

"CD3" refers to a part of a T-cell receptor complex and consists of three different chains CD3ε, CD3δ and CD3γ. CD3 is clustered on T cells by, for example, being immobilized by an anti-CD3 antibody, leading to the activation of T cells, which is similar to T cell receptor-mediated activation but independent of the specificity of TCR clones. Most anti-CD3 antibodies recognize the chain CD3ε. The second functional domain that specifically recognizes the T cell surface receptor CD3 in the present disclosure is not specifically limited as long as it can specifically recognize CD3, for example, but not limited to, CD3 antigens mentioned in the following patents: U.S. 7,994,289; U.S. 6,750,325; U.S. 6,706,265; U.S. 5,968,509; U.S. 8,076,459; U.S. 7,728,114; and U.S. 20100183615. Preferably, the antibody against human CD3 used in the present disclosure is cross-reactive with cynomolgus monkeys and/or rhesus monkeys, for example, but not limited to, CD3 antigens mentioned in the following patents: WO 2016130726, U.S. 20050176028, WO 2007042261, or WO 2008119565. The term also includes any variants, isotypes, derivatives, and species homologues of CD3 that are naturally expressed by cells or expressed by cells transfected with a gene or cDNA encoding the preceding chains.

The term "antibody" generally refers to proteinaceous binding molecules with immunoglobulin-like functions. Typical examples of the antibody are immunoglobulins and derivatives or functional fragments thereof as long as they exhibit the desired binding specificity. Techniques for preparing the antibody are well known in the art. The "antibody" includes different classes of natural immunoglobulins (such as IgA, IgG, IgM, IgD and IgE) and subclasses (such as IgG1, IgG2, IgA1 and IgA2). The "antibody" further includes non-natural immunoglobulins which include, for example, a single-chain antibody, a chimeric antibody (e.g., a humanized murine antibody), and a heterologous conjugated antibody (e.g., a bispecific antibody) as well as antigen-binding fragments thereof (e.g., Fab', F(ab'), Fab, Fv and rlgG). See, for example, Pierce Catalog and Handbook, 1994-1995, Pierce Chemical Co., Rockford, IL; Kuby, J., Immunology, 3rd Ed., 1998, NY; W.H. Freeman&Co..

The antibody can bind to one antigen to be "monospecific"; or bind to two different antigens to be "bispecific"; or bind to more than one different antigens to be "multispecific". The antibody may be monovalent, bivalent, or multivalent, i.e. the antibody may bind to one, two or multiple antigen molecules at one time.

The antibody "monovalently" binds to a particular protein, that is, one molecular of an antibody binds to merely one molecular of a protein, but the antibody may also bind to different proteins. When the antibody binds to merely one molecular of two different proteins, the antibody binds to each protein "monovalently" and the antibody is "bispecific" and "monovalently" binds to each of the two different proteins. The antibody may be "monomeric", that is, the antibody contains a single polypeptide chain. The antibody may contain multiple polypeptide chains ("multimeric") or may contain two ("dimeric"), three ("trimeric") or four ("tetrameric") polypeptide chains. If the antibody is multimeric, the antibody may be a homomultimer, that is, the antibody contains more than one molecular of only one type of polypeptide chain, including a homodimer, a homotrimer or a homotetramer. Alternatively, the multimeric antibody may be a heteromultimer, that is, the antibody contains more than one type of polypeptide chains that are different, including a heterodimer, a heterotrimer or a heterotetramer.

The term "hypervariable region", "CDR" or "complementarity determining region" refers to amino acid residues of an antibody, which are responsible for antigen binding, and is a discontinuous amino acid sequence. CDR sequences are amino acid residues in the variable region that may be defined by the IMGT, Kabat, Chothia or AbM method or identified by any CDR sequence determination method well known in the art. For example, the hypervariable region includes the following amino acid residues: amino acid residues from a "complementarity determining region" or "CDR" defined by sequence comparison, for example, residues at positions 24-34 (L1), 50-56 (L2) and 89-97 (L3) in a light chain variable domain and residues at positions 31-35 (H1), 50-65 (H2) and 95-102 (H3) in a heavy chain variable domain (see Kabat et al., 1991, Sequences of Proteins of Immunological Interest (5th edition), Public Health Service, National Institutes of Health, Bethesda, Md.), and/or amino acid residues from a "hypervariable loop" (HVL) defined according to the structure, for example, residues at positions 26-32 (L1), 50-52 (L2) and 91-96 (L3) in the light chain variable domain and residues at positions 26-32 (H1), 53-55 (H2) and 96-101 (H3) in the heavy chain variable domain (see Chothia and Leskl, J. Mol Biol, 196: 901-917, 1987). "Framework" residues or "FR" residues refer to variable domain residues other than the hypervariable region residues as defined in the present disclosure. In some embodiments, the antibody or the antigen-binding fragment thereof in the present disclosure is preferably determined through the Kabat, Chothia or IMGT numbering system. Those skilled in the art may explicitly assign each system to any variable domain sequence without relying on any experimental data beyond the sequence itself. For example, the Kabat residue numbering method of a given antibody may be determined by comparing the sequence of the given antibody to each "standard" numbered sequence. Based on the numbers of the sequences provided herein, the numbering scheme of determining any variable region sequence in the sequence table is entirely within the conventional technical scope of those skilled in the art.

The term "single-chain Fv antibody" (or "scFv antibody") refers to an antibody fragment comprising VH and VL domains of an antibody. It is a fusion protein of the variable regions of the heavy (VH) and light chains (VL) connected with a linker. The linker enables these two domains to be cross-linked to form an antigen-binding site, and the sequence of the linker generally consists of a flexible peptide, for example, but not limited to, G₂(GGGGS)₃. The size of scFv is generally 1/6 of an intact antibody. The single-chain antibody is preferably an amino acid chain sequence encoded by a nucleotide chain. For the review of scFv, reference may be made to Pluckthun (1994), The Pharmacology of Monoclonal Antibodies, Vol. 113, edited by Rosenburg and Moore, Springer-Verlag, New York, pages 269-315. Reference may also be made to International Patent Application Publication No. WO 88/01649 and U.S. Patent Nos. 4,946,778 and 5,260,203.

The term "Fab fragment" consists of a light chain and a CH1 and a variable domain of each of a heavy chain. The heavy chain of the Fab molecule cannot form a disulfide bond with another heavy chain molecule. The size of "Fab antibody" is 1/3 of an intact antibody, and "Fab antibody" includes only one antigen-binding site.

The term "Fab' fragment" contains a light chain, and a VH domain and a CH1 domain of a heavy chain, and a constant region between CH1 and CH2 domains.

The term "F(ab')₂ fragment" contains two light chains, VH domains and CH1 domains of two heavy chains, and constant regions between CH1 and CH2 domains, so that an inter-chain disulfide bond is formed between the two heavy chains. Therefore, the F(ab')₂ fragment is composed of two Fab' fragments held together by the disulfide bond between the two heavy chains.

The term "Fc" region refers to the antibody heavy chain constant region fragment, including at least a hinge region and CH2 and CH3 domains.

The term "Fv region" includes variable regions from the heavy chain and the light chain but lacks the constant regions, and is the minimum fragment containing a complete antigen recognition and binding site.

The term "Fd fragment" consists of CH1 and variable regions of a heavy chain and is the heavy chain part of a Fab fragment excluding the light chain.

The term "antibody fragment" and "antigen-binding fragment" refers to an antigen-binding fragment of the antibody that retains a specific binding ability to an antigen (e.g., CD19), as well as antibody analogs. It generally includes at least part of an antigen-binding region or a variable region of a parental antibody. The antibody fragment retains at least part of the binding specificity of the parental antibody. Generally, when the activity is represented in moles, the antibody fragment retains at least 10% of parental binding activity. Preferably, the antibody fragment retains at least 20%, 50%, 70%, 80%, 90%, 95% or 100% of the binding affinity of the parental antibody to a target. The antibody fragments include, but are not limited to, Fab fragments, Fab' fragments, F(ab')2 fragments, Fv fragments, Fd fragments, complementarity determining region (CDR) fragments, disulfide-stabilized variable fragments (dsFv); linear antibodies, single-chain antibodies (e.g., scFv monoclonal antibodies) (technology from Genmab), bivalent single-chain antibodies, single-chain phage antibodies, single domain antibodies (e.g., VH domain antibodies), domain antibodies (technology from Ablynx); multispecific antibodies formed from antibody fragments (e.g., triabodies and tetrabodies); and engineered antibodies such as chimeric antibodies (e.g., humanized mouse antibodies) and heteroconjugate antibodies. These antibody fragments can be obtained using any conventional technologies known to those skilled in the art, and the utility of these fragments can be screened in the same way as the intact antibody.

The term "linker peptide" refers to a peptide linking two polypeptides, wherein the linker peptide may be two immunoglobulin variable regions or one variable region. The length of the linker peptide may be 0 to 30 amino acids or 0 to 40 amino acids. In some embodiments, the linker peptide may be in the length of 0 to 25, 0 to 20, or 0 to 18 amino acids. In some embodiments, the linker peptide may be a peptide having no more than 14, 13, 12, 11, 10, 9, 8, 7, 6, or 5 amino acids. In other embodiments, the linker peptide may include 0 to 25, 5 to 15, 10 to 20, 15 to 20, 20 to 30, or 30 to 40 amino acids. In other embodiments, the linker peptide may have about 0, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids. The linker peptide is known to those skilled in the art. The linker peptide may be prepared by any method in the art. For example, the linker peptide may be originated from synthesis.

The term "heavy chain constant region" includes an amino acid sequence from the immunoglobulin heavy chain. The polypeptide comprising the heavy chain constant region includes at least one of: a CH1 domain, a hinge domain (e.g., an upper hinge region, an intermediate hinge region, and/or a lower hinge region), a CH2 domain, a CH3 domain, or a variant or fragment thereof. For example, the antigen-binding polypeptide used herein may include a polypeptide chain having a CH1 domain; a polypeptide having a CH1 domain, at least part of a hinge domain, and a CH2 domain; a polypeptide chain having a CH1 domain and a CH3 domain; a polypeptide chain having a CH1 domain, at least part of a hinge domain, and a CH3 domain; or a polypeptide chain having a CH1 domain, at least part of a hinge domain, a CH2 domain, and a CH3 domain. In another embodiment, the polypeptide of the present application includes a polypeptide chain having a CH3 domain. In addition, the antibody used in the present application may lack at least part of a CH2 domain (e.g., all or part of a CH2 domain). As described above, it is appreciated by those of ordinary skill in the art that heavy chain constant regions may be modified such that they differ in amino acid sequence from naturally immunoglobulin molecules.

The term "VH domain" includes the variable domain at the amino terminus of an immunoglobulin heavy chain. The term "CH1 domain" includes the first constant region (generally at the amino terminus) of an immunoglobulin heavy chain. The CH1 domain is adjacent to the VH domain and at the amino terminus to the hinge region of an immunoglobulin heavy chain molecule.

The term "hinge region" includes the portion of a heavy chain molecule that links the CH1 domain to the CH2 domain. The hinge region contains about 25 residues and is flexible so that two N-terminal antigen-binding regions move independently. The hinge region may be divided into three different domains: upper, middle and lower hinge domains (Roux KH et al., J. Immunol., 161: 4083, 1998).

The term "disulfide bond" includes a covalent bond formed between two sulfur atoms. The amino acid cysteine contains a sulfhydryl group which can form a disulfide bond or a bridge with a second sulfhydryl group. In most naturally occurring IgG molecules, CH1 and CK regions are linked by a disulfide bond and two heavy chains are linked by two disulfide bonds at positions 239 and 242 according to the Kabat numbering system (position 226 or 229, EU numbering system).

The term *"in vivo* half-life" refers to the biological half-life of the polypeptide of interest in the circulation of a given animal and is expressed as the time it takes to clear half of the amount present in the circulation of the animal from the circulation and/or other tissues in the animal.

The term "identity" refers to the matching of sequences between two polypeptides or between two nucleic acids. When a certain position in each of two sequences for comparison is occupied by the same base group or amino acid monomer subunit (e.g., a certain position in each of the two DNA molecules is occupied by adenine, or a certain position in each of the two polypeptides is occupied by lysine), then the molecules are identical at that position. The "percentage identity" between two sequences is a function of the number of matching positions of the two sequences divided by the number of positions to be compared and then multiplied by 100. For example, if 6 of the 10 positions in two sequences are matched, the identity between the two sequences is 60%. For example, DNA sequences CTGACT and CAGGTT have a total identity of 50% (three of a total of six positions are matched). Generally, the comparison is made when two sequences are aligned to produce maximum identity. Such alignment may be implemented, for example, through a computer program, such as an Align program (DNAstar, Inc.) to conveniently perform the method described by Needleman et al. Mol. Biol., 48: 443-453. The percentage identity between the two amino acid sequences may also be determined by using the algorithm proposed by E. Meyers and W. Miller (Comput. Appl Biosci., 4: 11-17) which has been incorporated into ALIGN program (version 2), using the PAM 120 weight residue table with a gap length penalty score of 12 and a gap penalty score of 4. In addition, the percentage identity between the two amino acid sequences may also be determined by using the algorithm proposed by Needleman and Wunsch (J. Mol. Biol., 48: 444-453) which has been incorporated into the GAP program in the GCG software package (available on www.gcg.com), using Blossum 62 matrix or PAM 250 matrix with a gap weight of 16, 14, 12, 10, 8, 6 or 4 and a length weight of 1, 2, 3, 4, 5 or 6.

The term "Fc region" or "Fc fragment" refers to the C-terminal regions of the immunoglobulin heavy chain, which includes at least part of a hinge region, a CH2 domain and a CH3 domain. It mediates the binding of immunoglobulins to host tissues or factors, including the binding of immunoglobulins to Fc receptors located on various cells (e.g., effector cells) of the immune system or the binding of immunoglobulins to the first component (C1q) of the classical complement system. It includes the native sequence Fc region and the variant Fc region.

Generally, the human IgG heavy chain Fc region is a segment from the amino acid residue at the position Cys226 or Pro230 of the human IgG heavy chain Fc region to the carboxy terminus, but its boundaries may vary. The C-terminal lysine of the Fc region (residue 447, according to the EU numbering system) may or may not be present. Fc may also refer to this region in isolation, or in the case of a protein polypeptide comprising Fc, for example, "binding protein comprising an Fc region", and is also referred to as "Fc fusion protein" (e.g., antibody or immunoadhesin). The native Fc region of the antibody of the present disclosure includes mammalian (e.g., human) IgG1, IgG2 (IgG2A, IgG2B), IgG3, and IgG4. Among human IgG1 Fc regions, at least two allotypes are known. In some embodiments, there is a single amino acid substitution, insertion, and/or deletion of about 10 amino acids per 100 amino acids in the amino acid sequences of two Fc polypeptide chains relative to the sequence of the amino acid sequence of the mammalian Fc polypeptide. In some embodiments, the difference may be changes in Fc that extend the half-life, changes that increase FcRn binding, changes that inhibit Fcγ receptor (FcyR) binding, and/or changes that decrease or remove ADCC and CDC.

In IgG, IgA and IgD antibody isotypes, the Fc region contains the CH2 and CH3 constant domains of each of the two heavy chains of the antibody; and in IgM and IgE, the Fc region contains three heavy chain constant domains (CH domains 2 to 4) in each polypeptide chain.

The term "Fc receptor" or "FcR" refers to a receptor that binds to the Fc region of an immunoglobulin. FcR may be a native sequence human FcR, or may be an FcR that binds to the IgG antibody (a γ receptor), as well as allelic variants and alternatively spliced forms of these receptors. The FcyR family is composed of three activating receptors (FcγRI, FcγRIII and FcyRIV in mice; FcyRIA, FcyRIIA and FcγRIIIA in humans) and one inhibitory receptor (FcγRIIb or equivalent FcyRIIB). The FcγRII receptor includes FcyRIIA ("activating receptor") and FcγRIIB ("inhibitory receptor") which have similar amino acid sequences. The cytoplasmic domain of FcyRIIA includes an immunoreceptor tyrosine-based activation motif (ITAM). The cytoplasmic domain of FcγRIIB contains an immunoreceptor tyrosine-based inhibitory motif (ITIM) (see M. Annu. Rev. Immunol., 15: 203-234 (1997)). Most native effector cell types co-express one or more activating FcyRs and inhibitory FcγRIIbs, while NK cells selectively express one activating Fc receptor (FcγRIII in mice and FcγRIIIA in humans) but do not express inhibitory FcγRIIb in mice and humans. Human IgG1 binds to most human Fc receptors and is considered equivalent to murine IgG2a in terms of the type of activating Fc receptor to which Human IgG1 binds. The term "FcR" herein covers other FcRs, including those which will be identified in the future. Methods of measuring the binding to FcRn are known (see, e.g., Ghetie V et al., Immunol Today, 18: 592-8, 1997); Ghetie V et al., Nature Biotechnology, 15: 637-40, 1997)). The *in vivo* binding and serum half-life of the human FcRn high-affinity binding polypeptide to FcRn can be determined, for example, in transgenic mice expressing human FcRn or transfected human cell lines. The term "Fc receptor" or "FcR" also includes the neonatal receptor FcRn which is responsible for transferring maternal IgG to the fetus (Guyer RL et al., J. Immunol., 117: 587, 1976) and Kim YJ et al., J. Immunol., 24: 249, 1994)).

The term "target cell" is a cell that binds to an antibody and participates in mediating disease. In some cases, the target cell may be a cell that generally mediates an immune response and also mediates a disease. For example, in B-cell lymphoma, B cells that generally mediate immune responses may be target cells. In some embodiments, the target cell is a cancer cell, a pathogen-infected cell, or a cell that mediates an auto-immunological disease or inflammatory disease (such as fibrosis). The antibody may bind to the target cell by binding to an antigen on a "target cell protein" which is a protein displayed on the surface of the target cell and may be a highly expressed protein.

The term "toxin" used herein refers to a substance that inhibits or prevents the function of a cell and/or causes damages to the cell. The term is intended to include radioisotopes (such as I¹³¹, I¹²⁵, Y⁹⁰ and Re¹⁸⁶), chemotherapeutic agents, toxins (such as toxins having enzymatic activity and derived from bacteria, fungi, plants or animals), or fragments thereof.

The term "cytokine" generally refers to a protein released by a population of cells, and acting as an intercellular mediator on another cell or having an autocrine effect on the cell that produces the protein. Examples of such cytokines include lymphokines, mononuclear factors; interleukins ("IL") such as IL-2, IL-6 and IL-17A-F; tumor necrosis factors such as TNF-α or TNF-β; and other polypeptide factors such as a leukemia inhibitory factor (LIF).

The term "chemotherapy" refers to the administration of any chemical agent for therapeutic purposes in the treatment of diseases characterized by the growth of abnormal cells. Such diseases include, for example, tumors, neoplasms, cancers and diseases characterized by proliferative growth. A "chemotherapeutic agent" specifically targets cells involved in cell division rather than cells not involved in the cell division. The chemotherapeutic agent directly interferes with processes closely related to cell division, such as DNA replication, RNA synthesis, protein synthesis, or the assembly, decomposition, or function of mitotic spindles, and/or the synthesis or stability of molecules (such as nucleotides or amino acids) that function in these processes. Therefore, the chemotherapeutic agent has cytotoxicity or cytostatic effects on cancer cells and other cells involved in cell division. Combined chemotherapy refers to the administration of more than one agent for the treatment of cancer.

The term "immunobinding" and "immunobinding property" refers to a non-covalent interaction between an immunoglobulin molecule and an antigen (to the antigen, the immunoglobulin is specific). The strength or affinity of the immunobinding interaction may be represented by the equilibrium dissociation constant (K_{D}) of the interaction, where the smaller the K_{D} value, the higher the affinity. The immunobinding property of the selected polypeptide may be quantified using a method known in the art. One method relates to the measurement of rates at which an antigen-binding site/antigen complex is formed and dissociated. Both the "binding rate constant" (Kₐ or Kₒₙ) and the "dissociation rate constant" (K_{d} or K_{off}) may be calculated according to the concentration and actual rates of association and dissociation (see Malmqvist M et al., Nature, 361: 186-187, 1993). The ratio of k_{d}/kₐ is the dissociation constant K_{D} (generally see Davies et al., Annual Rev Biochem., 1990, 59: 439-473). Any effective method may be used for measuring values of K_{D}, kₐ and k_{d}.

The term "cross-reaction" refers to the ability of the antibody described herein to bind to tumor-associated antigens from different species. For example, the antibody described herein that binds to human TAA may also bind to TAAs from other species (e.g., cynomolgus monkey TAA). Cross-reactivity may be measured by detecting specific reactivity with purified antigens in binding assays (e.g., SPR, ELISA), or detecting the binding to cells physiologically expressing TAA or the interaction with the function of cells physiologically expressing TAA. Examples of assays known in the art for determining the binding affinity include surface plasmon resonance (e.g., Biacore) or similar techniques (e.g., Kinexa or Octet).

The term "immune response" refers to the action of immune system cells (such as T lymphocytes, B lymphocytes, NK cells, antigen-presenting cells, macrophages, eosinophils, mast cells, DCs, or neutrophils) and soluble macromolecules produced by any one of the immune cells or the liver (including antibodies, cytokines and complements) that results in selective injuries to, damages to, or elimination from the human body of invading pathogens, cells or tissues infected with pathogens, cancer cells, or normal human cells or tissues in the context of pathological inflammation. The immune response includes, for example, the activation or inhibition of T cells (e.g., effector T cells or Th cells such as CD⁸⁺ or CD⁴⁺ T cells) or the inhibition or depletion of NK cells or Treg cells.

"Effector cell" refers to a cell of the immune system, which expresses one or more FcRs and mediates one or more effector functions. Preferably, the cell expresses at least one type of activating Fc receptors such as human FcγRIII and performs ADCC effector function. Examples of human leukocytes which mediate ADCC include peripheral blood mononuclear cells (PBMCs), natural killer (NK) cells, monocytes, macrophages, neutrophils, and eosinophils. Effector cells also include, for example, T cells. They may be derived from any organism including, but not limited to, humans, mice, rats, rabbits or monkeys.

The term "effector function" refers to biological activities that can be attributed to the biological activities of the antibody Fc region (a native sequence Fc region or amino acid sequence variant Fc region) and that vary with antibody isotypes. Examples of antibody effector functions include, but are not limited to, Fc receptor binding affinity, ADCC, ADCP, CDC, downregulation of cell surface receptors (e.g., B cell receptors), B cell activation, cytokine secretion, and half-life/clearance rate of antibodies and antigen-antibody complexes. Methods of altering the effector function of antibodies are known in the art, for example, the effector function of antibodies may be altered by introducing mutations in the Fc region.

The term "antibody-dependent cell-mediated cytotoxicity (ADCC)" refers to a cytotoxic form in which Ig binds to FcRs on cytotoxic cells (e.g., NK cells, neutrophils or macrophages) to enable these cytotoxic effector cells to specifically bind to antigen-attached target cells and then secret cytotoxins to kill the target cells. Methods for detecting the ADCC activity of an antibody are known in the art, for example, the ADCC activity may be detected by measuring the binding activity between a to-be-tested antibody and FcR (e.g., CD16a).

The term "antibody-dependent cell-mediated phagocytosis (ADCP)" refers to a cell-mediated reaction in which a non-specific cytotoxic active cell expressing FcyR recognizes a bound antibody on a target cell and subsequently causes phagocytosis of the target cell.

The term "complement-dependent cytotoxicity (CDC)" refers to a cytotoxic form that activates the complement cascade by binding the complement component C1q to the antibody Fc. Methods for detecting the CDC activity of an antibody are known in the art, for example, the CDC activity may be detected by measuring the binding activity between a to-be-tested antibody and an Fc receptor (e.g., C1q).

The term "pharmaceutically acceptable carrier and/or excipient and/or diluent" refers to a carrier and/or excipient and/or stabilizer which is pharmacologically and/or physiologically compatible with the subject and the active ingredient and which is non-toxic to the cell or mammal exposed to such a carrier and/or excipient and/or stabilizer at the dosage and concentration employed. Examples include, but are not limited to, pH regulators, surfactants, adjuvants, ionic strength enhancers, diluents, reagents to maintain osmotic pressure, reagents to delay absorption, and preservatives. For example, pH adjusting agents include, but are not limited to, phosphate buffers. Surfactants include, but are not limited to, cationic surfactant, anionic surfactant or nonionic surfactants, for example, Tween-80. Ionic strength enhancers include, but are not limited to, sodium chloride. Preservatives include, but are not limited to, various antibacterial reagent and antifungal reagent, such as parabens, chlorobutanol, phenol, and sorbic acid. Reagents to maintain osmotic pressure include, but are not limited to, sugars, NaCl, and analogs thereof. Reagents to delay absorption include, but are not limited to, monostearate and gelatin. Diluents include, but are not limited to, water, aqueous buffers (e.g., buffered saline), alcohols, and polyols (e.g., glycerol). Preservatives include, but are not limited to, various antibacterial reagent and antifungal reagent, such as thiomersal, 2-phenoxyethanol, parabens, chlorobutanol, phenol, and sorbic acid. Stabilizers have the meaning as commonly understood by those of ordinary skill in the art. Stabilizers are those capable of stabilizing the desired activity of the active ingredient in a drug, including, but not limited to, sodium glutamate, gelatin, SPGA, sugars (e.g., sorbitol, mannitol, starch, sucrose, lactose, dextran or glucose), amino acids (e.g., glutamic acid or glycine), proteins (e.g., dry whey, albumin or casein), or degradation products thereof (e.g., lactalbumin hydrolysate).

The terms "patient" and "subject", "individual" or "object" refer to any human or non-human animal, in particular human, that receives prophylactic or therapeutic treatment. For example, the method and composition described herein may be used to treat a subject with cancer. The term "non-human animal" includes all vertebrates, for example, mammals and non-mammals such as non-human primates, sheep, dogs, cattle, chickens, amphibians, reptiles and the like.

The "treatment" or "therapy" for a subject means any type of intervention or treatment on the subject or administration of an active agent to the subject for the purpose of reversing, alleviating, ameliorating, inhibiting, slowing down or preventing the occurrence, progression, development, severity or recurrence of symptoms, complications, conditions or biochemical indicia related to a disease.

The term "autoimmunization" refers to that in an immune-tolerant state, a certain amount of autoreactive T cells and autoantibodies are ubiquitous in the whole peripheral immune system of an individual, which helps to eliminate aging and degenerated auto components and has important physiological significance for maintaining the autoimmune stability of the immune system.

The terms "mutated", "mutant" and "mutation" in reference to nucleic acids and polypeptides refer to the replacement, deletion or insertion of one or more nucleotides or amino acids, respectively, relative to natural nucleic acids or polypeptides (i.e., reference sequences that may be used for defining wild types).

The term "T-cell receptor (TCR)" is a specific receptor present on the surface of T cells, i.e., T lymphocytes. *In vivo,* the T-cell receptor is present as a complex of several proteins. The T-cell receptor generally has two separate peptide chains, typically T-cell receptors α and β (TCRα and TCRβ) chains, and in some T cells, they are T-cell receptor γ and δ (TCRγ and TCRδ). The other proteins in the complex are CD3 proteins: CD3εγ and CD3εδ heterodimers, most importantly, CD3ζ homodimers having six ITAMs. The ITAMs on CD3ζ can be phosphorylated by Lck, which in turn recruit ZAP-70. Lck and/or ZAP-70 may also phosphorylate tyrosine on many other molecules, particularly CD28, LAT, and SLP-76, which allows aggregation of signal transduction complexes around these proteins.

The term "bispecific antibody" refers to the bispecific antibody of the present disclosure, for example, an anti-CD19 antibody or an antigen-binding fragment thereof, which may be derivatized or linked to another functional molecule, for example, another peptide or protein (e.g., TAA, a cytokine and a cell surface receptor), to generate a bispecific antibody that binds to at least two different binding sites or target molecules. To produce the bispecific molecule of the present disclosure, the antibody of the present disclosure may be functionally linked (e.g., by chemical coupling, gene fusion, non-covalent binding or other means) to one or more other binding molecules, for example, another antibody, antibody fragment, peptide or binding mimetic, to produce the bispecific molecule. For example, the "bispecific antibody" refers to one including two variable domains or ScFv units such that the antibody obtained recognizes two different antigens. Various different forms and uses of the bispecific antibody are known in the art (Chames P et al., Curr. Opin. Drug Disc. Dev., 12:.276, 2009; Spiess C et al., Mol. Immunol., 67: 95-106, 2015).

The term "hCG-β carboxy terminal peptide (CTP)" is a short peptide from the carboxy terminus of a human chorionic gonadotropin (hCG) β-subunit. Four reproduction-related polypeptide hormones, follicle-stimulating hormone (FSH), luteinizing hormone (LH), thyroid-stimulating hormone (TSH), and human chorionic gonadotropin (hCG), each contain the same α-subunit and their respective specific β-subunits. The *in vivo* half-life of hCG is significantly longer than those of the other three hormones, mainly due to the specific carboxy terminal peptide (CTP) on the β-subunit of hCG. The CTP includes 37 amino acid residues and four O-glycosylation sites, in which sugar side chain terminals are sialic acid residues. The electronegative highly-sialyl CTP can resist renal clearance and thus extend the half-life *in vivo* (Fares F A et al., Proc Natl Acad. Sci. USA, 1992, 89: 4304-4308, 1992).

The term "glycosylation" means that an oligosaccharide (a carbohydrate containing two or more monosaccharides that are linked together, e.g., a carbohydrate containing 2 to about 12 monosaccharides that are linked together) is attached to form a glycoprotein. The oligosaccharide side chains are generally linked to the backbone of the glycoprotein via *N*- or *O*-linkages. The oligosaccharides of the antibodies disclosed herein are generally CH2 domains linked to the Fc region as *N*-linked oligosaccharides. "*N*-linked glycosylation" refers to carbohydrate moiety attachment to an asparagine residue of a glycoprotein chain. For example, the skilled artisan can recognize a single site useful for *N*-linked glycosylation at residue 297 of each of CH2 domains of murine IgG1, IgG2a, IgG2b and IgG3 and human IgG1, IgG2, IgG3, IgG4, IgA and IgD.

### Homologous antibody

In another aspect, amino acid sequences included in the heavy and light chain variable regions of the antibody of the present disclosure are homologous with amino acid sequences of the preferred antibody described herein, and the antibody retains desired functional properties of the bispecific antibody of the present disclosure, for example, Her2×CD3 bispecific antibody.

### Antibody with conservative modifications

The term "conservative modification" is intended to mean that an amino acid modification does not significantly affect or change the binding characteristics of the antibody containing an amino acid sequence. Such conservative modifications include amino acid substitutions, additions and deletions. A modification may be introduced into the antibody of the present disclosure by using a standard technology known in the art, such as a site-directed mutagenesis and a PCR-mediated mutagenesis. A conservative amino acid substitution refers to the substitution of an amino acid residue with an amino acid residue with a similar side chain. Families of amino acid residues with similar side chains have been described in detail in the art. These families include amino acids with basic side chains (such as lysine, arginine and histidine), amino acids with acidic side chains (such as aspartic acid and glutamic acid), amino acids with uncharged polar side chains (such as glycine, asparagine, glutamine, serine, threonine, tyrosine, cysteine and tryptophan), amino acids with non-polar side chains (such as alanine, valine, leucine, isoleucine, proline, phenylalanine and methionine), amino acids with β-branched side chains (such as threonine, valine and isoleucine) and amino acids with aromatic side chains (such as tyrosine, phenylalanine, tryptophan and histidine). Therefore, one or more amino acid residues in the CDR of the antibody of the present disclosure may be substituted with other amino acid residues from the same side chain family.

### Fc variant with altered binding affinity for the neonatal receptor (FcRn)

"FcRn" used herein refers to a protein that binds to at least part of the Fc region of the IgG antibody and that is encoded by the FcRn gene. FcRn may be derived from any organism including, but not limited to, humans, mice, rats, rabbits or monkeys. The functional FcRn protein includes two polypeptides that often referred to as heavy and light chains, in which the light chain is β-2-microgiobuiin and the heavy chain is encoded by the FcRn gene.

The present disclosure relates to an antibody whose binding to FcRn is regulated (the regulation includes to increase or decrease the binding). For example, in some cases, increased binding may result in cell recirculating antibodies, and thus extends, for example, the half-life of the therapeutic antibody. Sometimes, it is desirable to decrease the FcRn binding, for example, when the antibody is used as a diagnostic or therapeutic antibody including a radiolabel. In addition, antibodies exhibiting increased binding to FcRn and altered binding to other Fc receptors such as Fcγ Rs may be used in the present disclosure.

The present application relates to an antibody including an amino acid modification that regulates the binding to FcRn. Of particular interest is that at lower pH, the binding affinity for FcRn exhibits an increase, while at higher pH, the binding basically does not exhibit an altered antibody that minimally includes the Fc region or functional variants thereof.

### Fc variant with enhanced binding affinity for the neonatal receptor (FcRn)

The plasma half-life of IgG depends on its binding to FcRn, where IgG generally binds to FcRn at a pH of 6.0 and dissociates from FcRn at a pH of 7.4 (the pH of plasma). Through studies on the binding site, a binding site of IgG to FcRn is modified to increase the binding capacity at the pH of 6.0. It has been proved that mutations of some residues of a human Fcγ domain, which are essential to the binding to FcRn, can increase the serum half-life. It has been reported that mutations at T250, M252, S254, T256, V308, E380, M428, and N434 (EU numbering) can increase or decrease the binding affinity for FcRn (Roopenian et al., Nat. Rev. Immunol., 7: 715-725, 2007). Korean Patent No. KR 10-1027427 discloses trastuzumab (Herceptin, Genentech) variants with enhanced binding affinity for FcRn, where these variants include one or more amino acid modifications selected from 257C, 257M, 257L, 257N, 257Y, 279Q, 279Y, 308F, and 308Y. Korean Patent Publication No. KR 2010-0099179 provides bevacizumab (Avastin, Genentech) variants, where these variants exhibit an increased *in vivo* half-life through amino acid modifications included in N434S, M252Y/M428L, M252Y/N434S, and M428L/N434S. In addition, Hinton et al. have found that T250Q and M428L mutants increase the binding to FcRn threefold and sevenfold, respectively. At the same time, the mutation of two sites increases the binding 28-fold. In rhesus monkeys, the M428L or T250QM/428 L mutant exhibits the plasma half-life increased twofold (Hinton P.R. et al., J. Immunol., 176: 346-356, 2006). For more mutational sites included in the Fc variant with the enhanced binding affinity for the neonatal receptor (FcRn), see Chinese invention patent CN 201280066663.2. In addition, studies have shown that through the T250Q/M428L mutation on Fc fragments of five humanized antibodies, the interaction between Fc and FcRn is improved, and in subsequent *in vivo* pharmacokinetic assays, the pharmacokinetic parameters of the Fc-mutation antibody are improved compared with the wild-type antibody through subcutaneous administration, for example, the *in vivo* exposure is increased, the clearance rate is reduced, and the subcutaneous bioavailability is increased (Datta-Mannan A et al., MAbs. Taylor&Francis, 4: 267-273, 2012).

Other mutational sites capable of enhancing the affinity of the antibody of the present disclosure for FcRn include, but are not limited to, the following amino acid modifications: 226, 227, 230, 233, 239, 241, 243, 246, 259, 264, 265, 267, 269, 270, 276, 284, 285, 288, 289, 290, 291, 292, 294, 298, 299, 301, 302, 303, 305, 307, 309, 311, 315, 317, 320, 322, 325, 327, 330, 332, 334, 335, 338, 340, 342, 343, 345, 347, 350, 352, 354, 355, 356, 359, 360, 361, 362, 369, 370, 371, 375, 378, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 433, 438, 439, 440, 443, 444, 445, and 446, where the numbers of the amino acids in the Fc region is numbers of the EU indexes in Kabat.

Fc variants with enhanced binding affinity for FcRn also include all other known amino acid modification sites as well as amino acid modification sites that have not yet been found.

In an optional embodiment, the IgG variant can be optimized to gain increased or decreased affinity for FcRn and increased or decreased affinity for human FcyR including, but not limited to, FcγRI, FcγRIIa, FcγRIIb, FcγRIIc, FcγRIIIa and FcγRIIIb, including allelic variants thereof.

Preferentially, the Fc ligand specificity of an IgG variant determines its therapeutic application. The given IgG variant for therapeutic purposes depends on the epitope or form of the target antigen as well as the to-be-treated disease or indication. Enhanced FcRn binding may be more preferred for most targets and indications because enhanced FcRn binding may result in extended serum half-life. A relatively long serum half-life allows administration at relatively low frequencies and doses during treatment.

This property may be particularly preferred when the therapeutic agent is administered in order to respond to indications requiring repeated administration. For some targets and indications, the reduced affinity for FcRn may be particularly preferred when the variant Fc is required to have an increased clearance or reduced serum half-life, for example, when the Fc polypeptide is used as an imaging agent or a radiotherapy agent.

The affinity of the polypeptide for FcRn can be evaluated by methods well known in the art.

FcRn may be derived from any organism including, but not limited to, humans, mice, rats, rabbits or monkeys.

### Alterations to inhibit FcγR binding

As used herein, "alterations to inhibit FcyR binding" refers to one or more insertions, deletions or substitutions in the Fc polypeptide chain that inhibit binding of FcyRIIA, FcγRIIB and/or FcγRIIIA, in which the binding is determined, for example, by a competitive binding assay (PerkinElmer, Waltham, MA). These alterations may be included in the Fc polypeptide chain as part of the bispecific antibody. More specifically, alterations that inhibit binding of the Fcγ receptor (FcyR) include L234A, L235A, or any alteration that inhibits glycosylation at the position N297, including any substitution at N297. In addition, along with the alterations that inhibit glycosylation of the position N297, additional alterations to stabilize the dimer Fc region by establishing additional disulfide bridges are also expected. Further examples of alterations that inhibit FcyR binding include D265A alterations in one Fc polypeptide chain and A327Q alterations in another Fc polypeptide chain. Some of the above mutations are described, for example, in Xu D et al., Cellular Immunol., 200: 16-26, 2000, of which the section about the above mutations and the activity evaluation thereof is incorporated herein by reference. The above numbers are based on EU numbering.

For example, the Fc fragment included by the bispecific antibody provided by the present disclosure in the alterations that inhibit FcyR binding exhibits reduced affinity for at least one of human FcγR (FcγRI, FcγRIIa or FcγRIIIa) or C1q, and has reduced effector cell functions or complement functions.

Other alterations that inhibit FcyR binding include sites and modifications thereof that are well known in the art or may be discovered in the future.

FcyR may be derived from any organism including, but not limited to, humans, mice, rats, rabbits or monkeys.

### Fc alterations to extend the half-life

As used herein, "Fc alterations to extend the half-life" refers to an alteration to extend the *in vivo* half-life of a protein that includes an altered Fc polypeptide in the Fc polypeptide chain as compared with the half-life of a protein that includes the same Fc polypeptide but does not include any altered but similar Fc. These alterations may be included in the Fc polypeptide chain as part of the bispecific antibody. Alterations T250Q, M252Y, S254T and T256E (alteration of threonine at position 250 to glutamine; alteration of methionine at position 252 to tyrosine; alteration of serine at position 254 to threonine; and alteration of threonine at position 256 to glutamic acid; where numbers are based on EU numbering) is an Fc alteration to extend half-life and may be used jointly, alone or in any combination. These and other alterations are described in detail in U.S. Pat. No. 7,083,784. The section about this alteration described in U.S. Pat. No. 7,083,784 is incorporated herein by reference.

Similarly, M428L and N434S are Fc alterations that extend the half-life and can be used jointly, alone or in any combination. These alterations and other alterations are described in detail in U.S. Patent Application Publication No. 2010/0234575 and U.S. Pat. No. 7,670,600. Sections of such alterations described in U.S. Patent Application Publication No. 2010/0234575 and U.S. Pat. No. 7,670,600 are incorporated herein by reference.

In addition, according to the meaning herein, any substitution at one of the following positions can be considered to be an Fc alternation that extends the half-life: 250, 251, 252, 259, 307, 308, 332, 378, 380, 428, 430, 434, and 436. Each of these alterations or a combination of these alterations may be used to extend the half-life of the bispecific antibody described herein. Other alternations that can be used to extend the half-life are described in detail in International Application No. PCT/US2012/070146 (Publication No. WO 2013/096221) filed December 17, 2012. The section about the above alterations of this application is incorporated herein by reference.

Fc alternations that extend the half-life also include sites and modifications thereof that are well known in the art or may be discovered in the future.

Fc may be derived from any organism including, but not limited to, humans, mice, rats, rabbits or monkeys.

### Nucleic acid encoding the bispecific antibody

Using the therapeutic agent, the antibody or the antibody fragment described herein, those skilled in the art may easily construct multiple clones containing functionally equivalent nucleic acids (e.g., nucleic acids having different sequences but encoding the same effector moiety or antibody sequence). Therefore, the present disclosure provide bispecific antibodies, nucleic acids encoding the antibodies, antibody fragments and conjugates and fusion protein thereof, and variants, derivatives and species homologues of the nucleic acids .

Many nucleic acid sequences encoding immunoglobulin regions including VH, VL, hinge, CH1, CH2, CH3 and CH4 regions are known in the art. See, for example, Kabat et al., Sequences of Proteins of Immunological Interest, Public Health Service N.I.H., Bethesda, MD, 1991. According to the teachings provided herein, those skilled in the art may combine the nucleic acid sequences and/or other nucleic acid sequences known in the art to construct a nucleic acid sequence encoding the bispecific antibody of the present disclosure. An exemplary nucleotide encoding the bispecific antibody of the present disclosure includes (1) SEQ ID NO: 56, (2) SEQ ID NO: 58, (3) SEQ ID NO: 60 and (4) SEQ ID NO: 62.

In addition, based on the amino acid sequences provided herein and elsewhere and the common knowledge in the art, those skilled in the art may determine the nucleic acid sequence encoding the bispecific antibody of the present disclosure. In addition to more conventional methods for producing cloned DNA fragments encoding particular amino acid sequences, companies such as DNA 2.0 (Menlo Park, CA, USA) and Blue Heron (Bothell, WA, USA) generally employ chemical synthesis to produce DNA that has any size of genes arranged in a desired order, thus simplifying the process of producing the DNA.

### Method for preparing a bispecific antibody

The bispecific antibody of the present disclosure may be prepared by any method known in the art. Early methods for constructing the bispecific antibody include chemical cross-linking or hybridoma heterozygosis or quadroma method (e.g., Staerz UD et al., Nature, 314: 628-31, 1985; Milstein C et al., Nature, 305: 537-540, 1983; Karpovsky B et al., J. Exp. Med., 160: 1686-1701, 1984). The chemical coupling method is to connect two different monoclonal antibodies by chemical coupling to prepare a bispecific monoclonal antibody. For example, two different monoclonal antibodies chemically bind to each other, or two antibody fragments, for example, two Fab fragments chemically bind to each other. The heterozygosis-hybridoma method is to prepare a bispecific monoclonal antibody by a cell hybridization method or a ternary hybridoma method, where the cell hybridoma or the ternary hybridoma is obtained by the fusion of constructed hybridomas or the fusion of a constructed hybridoma and lymphocytes derived from mice. Although these techniques are used to manufacture BiAb, various generation problems make such complexes difficult to use, such as the generation of mixed populations containing different combinations of antigen-binding sites, difficulties in protein expression, the need for purifying the target BiAb, low yields, and high production costs.

Recent methods utilize genetically engineered constructs that can produce a single homogeneous product of BiAb so that there is no need for thorough purification to remove unwanted by-products. Such constructs include tandem scFv, diabodies, tandem diabodies, double variable domain antibodies, and heterdimeric antibodies using the Ch1/Ck domain or DNLTM motifs (Chames & Baty, Curr. Opin. Drug. Discov. Devel., 12: 276-83, 2009; Chames & Baty, mAbs, 1: 539-47). Related purification techniques are well known.

The antibody may also be produced by cloning and expressing immunoglobulin variable region cDNAs produced from a single lymphocyte selected to produce the specific antibody using a single lymphocyte antibody method, for example, by a method described in Babcook J et al., Proc. Natl. Acad. Sci. USA. 93: 7843-7848, 1996; WO92/02551; WO2004/051268 and WO2004/106377.

Antigen polypeptides for producing, for example, antibodies for immunizing hosts or for screening, for example, antibodies for phage display (or the expression on the surface of yeast cells or bacterial cells) may be prepared from genetically engineered host cells including expression systems by methods well known in the art, or they may be recycled from natural biological sources. For example, nucleic acids encoding one or two polypeptide chains of the bispecific antibody may be introduced into cultured host cells by a variety of known methods (such as transformation, transfection, electroporation and bombardment with nucleic acid-coated microparticles, etc.). In some embodiments, the nucleic acids encoding the bispecific antibody may be inserted into a vector suitable to be expressed in the host cell before introduced into the host cell. A typical vector may include sequence elements that enable the inserted nucleic acids to be expressed at RNA and protein levels.

The vector is well known in the art and many vectors are commercially available. The host cell containing the nucleic acids may be cultured under conditions that enable the cell to express the nucleic acids, and the resulting BiAb may be collected from a cell population or a culture medium. Optionally, the BiAb may be produced *in vivo,* for example, in a plant leaf (see, for example, Scheller J et al., Nature Biotechnol., 19: 573-577, 2001 and references cited therein), in a bird egg (see, for example, Zhu L et al., Nature Biotechnol., 23: 1159-1169, 2005 and references cited therein) or in milk of a mammal (see, for example, Laible G et al., Reprod. Fertil. Dev., 25: 315, 2012).

Various cultured host cells that may be used include, for example, prokaryotic cells, eukaryotic cells, bacterial cells (such as *Escherichia coli* or *Bacilis stearothermophilus),* fungal cells (such as *Saccharomyces cerevisiae* or *Pichia pastoris),* insect cells (such as lepidopteran insect cells including Spodoptera frugiperda cells), or mammalian cells (such as Chinese hamster ovary (CHO) cells, NS0 cells, baby hamster kidney (BHK) cells, monkey kidney cells, Hela cells, human hepatocellular carcinoma cells or 293 cells).

The bispecific antibody may be prepared by immunizing an appropriate subject (such as a rabbit, a goat, a mouse, or other mammals including transgenic and knocked-out mammals) with an immunogenic preparation of the bispecific antigen. An appropriate immunogenic preparation may be, for example, a chemically synthesized or recombinantly expressed bispecific antigen. The preparation may further include adjuvants such as Freund's complete or incomplete adjuvants or similar immunostimulatory compounds. Furthermore, the bispecific antigen of the present disclosure may be used alone or preferably used as a conjugate with a vector protein when used for preparing antibodies, in particular by *in vivo* immunization. Such methods of enhancing an antibody response are well known in the art. Depending on different antibodies required, different animal hosts may be used for *in vivo* immunization. A host that expresses an endogenous antigen of interest itself may be used, or a host that has been caused deficient in an endogenous antigen of interest may be used.

The bispecific antibody may be prepared by a combination of the methods described above.

The bispecific antibody molecule of the present disclosure may act as a monoclonal antibody (MAb) for each target. In some embodiments, the antibody is chimeric, humanized or fully human.

The monoclonal antibody may be prepared by any method known in the art, such as a hybridoma technique (Kohler&Milstein, Nature, 256: 495-497, 1975), a trioma technique, a human B-cell hybridoma technique (Kozbor D et al., Immunology Today, 4: 72, 1983), and an EBV-hybridoma technique (Cole SPC et al., Monoclonal Antibodies and Cancer Therapy, pp 77-96, Alan R Liss, Inc., 1985).

The bispecific antibody or a portion thereof in the present disclosure may be used for detecting any or all of these antigens (for example, in biological samples such as serum or plasma) by conventional immunological analysis methods such as an enzyme-linked immunosorbent assay (ELISA), a radioimmunoassay (RIA) or tissue immunohistochemistry. The present disclosure provides a method for detecting an antigen in a biological sample, comprising: contacting a biological sample with the bispecific antibody or the antibody portion of the present disclosure that specifically recognizes the antigen, and detecting an antigen-bound antibody (or antibody portion) or a non-bound antibody (or antibody portion), so as to detect the antigen in the biological sample. The antibody is directly or indirectly labeled with a detectable substance to facilitate the detection of the bound or non-bound antibody. Suitable detectable substances include various enzymes, repair groups, fluorescent substances, luminescent substances and radioactive substances. Examples of suitable enzymes include horseradish peroxidase, alkaline phosphatase, β-galactosidase and acetylcholinesterase; examples of suitable repair group complexes include streptavidin/biotin and avidin/biotin; examples of suitable fluorescent substances include 7-hydroxycoumarin, fluorescein, fluorescein isothiocyanate, rhodamine, dichlorotriazinylamine fluorescein, dansyl chloride or phycoerythrin; an example of the luminescent substance includes 3-aminophthalhydrazide; examples of suitable radioactive substances include I¹²⁵, I¹³¹, ³⁵S or ³H.

### Immune effector cell and effector cell protein

The bispecific antibody can bind to a molecule expressed on the surface of an immune effector cell (herein referred to as an "effector cell protein") and to another molecule expressed on the surface of a target cell (herein referred to as a "target cell protein"). The immune effector cell may be a T cell, an NK cell, a macrophage or a neutrophil. In some embodiments, the effector cell protein is a protein contained in a T-cell receptor (TCR)-CD3 complex. The TCR-CD3 complex includes a homodimer which includes TCRα and TCRβ or TCRγ and TCRδ and a variety of CD3 chains selected from CD3ζ chains, CD3ε chains, CD3γ chains and CD3δ chains, etc. In some embodiments, the effector cell protein may be a human CD3ε chain, which may be a portion of a dimer protein. Alternatively, the effector cell protein may be TCRα, TCRβ, TCRδ, TCRγ, CD3β, CD3γ, CD3δ or CD3ζ of a human and/or a cynomolgus monkey and/or a rhesus monkey.

Additionally, in some embodiments, the bispecific antibody may also bind to a CD3ε chain of a non-human species (such as mice, rats, rabbits, New World monkeys and/or Old World monkeys). The species includes, but is not limited to, the following mammal species: Mus musculus; Rat tusrattus; Rattus norvegicus; Cynomolgus monkeys; hamadryas baboon, Papio hamadryas; Guinea baboon, Papio papio; olive baboon, Papio anubis; yellow baboon, Papio cynocephalus; Chacma baboon, Papio ursinus; Callithrix jacchus; Saguinus Oedipus and Saimiri sciureus. It can be known from the field of development of protein therapeutics, therapeutics with similar activity in humans and species commonly used in preclinical researches (such as mice and monkeys) can simplify and accelerate the development of drugs, which is extremely important for bringing drugs to market.

In some preferred embodiments, a partial fragment of the above CD3 chain may be bound to the homodimer of the present disclosure. The CD3 may be a human CD3ε chain or a CD3ε chain derived from a different species, preferably one of the mammal species listed above. The epitopes to which the antibody binds may be determined through alanine scanning and other known techniques. The alanine scanning is described in, for example, U.S. Patent Application Publication No. 2010/183615, the relevant portion of which is incorporated herein by reference.

When a T cell is the immune effector cell, the effector cell protein to which the bispecific antibody can bind includes, but is not limited to, CD3ε, CD3γ, CD3δ, CD3ζ, TCRα, TCRβ, TCRγ and TCRδ. When an NK cell or a cytotoxic T cell is the immune effector cell, the effector cell protein may be, for example, NKG2D, CD352, NKp46 or CD16a. When a CD⁸⁺T cell is the immune effector cell, the effector cell protein may be, for example, 4-1BB or NKG2D. Alternatively, the bispecific antibody may bind to other effector cell proteins expressed on T cells, NK cells, macrophages or neutrophils.

### Target cell and target cell protein expressed on the target cell

As described above, the bispecific antibody can bind to effector cell proteins and target cell proteins. For example, the target cell protein may be expressed on the surface of cancer cells, cells infected by pathogen, or cells mediating diseases (e.g., inflammatory and autoimmune diseases). In some embodiments, the target cell protein can be highly expressed on the surface of target cells, although such high-level expression is not required. In some embodiments, the target cell protein is not expressed or low-expressed on the surface of target cells.

When the target cell is a cancer cell, the homodimeric bispecific antibody as described herein can bind to the cancer cell antigen as described above. The cancer cell antigen may be a human protein or a protein derived from other species.

In some embodiments, the target cell protein may be a protein that is selectively expressed or overexpressed or not expressed on the surface of tumor cells.

In some embodiments, the target cell protein may be a protein on the surface on cells that mediate lymphatic system-related diseases.

In other aspects, the target cell may be a cell that mediates autoimmune diseases or inflammatory diseases. For example, human eosinophils in asthma may be the target cell, and in this case, for example, the EGF-like module-containing mucin-like hormone receptor (EMR1) may be the target cell protein. Optionally, excess human B cells in patients suffering from systemic lupus erythematosus may be the target cell, and in this case, for example, CD19 or CD20 may be the target cell protein. In other autoimmune diseases, excess human Th2T cells may be the target cell, and in this case, for example, CCR4 may be the target cell protein. Similarly, the target cell may be a fibrotic cell that mediates, for example, atherosclerosis, chronic obstructive pulmonary disease (COPD), liver cirrhosis, scleroderma, renal transplantation fibrosis, renal allograft nephropathy or pulmonary fibrosis (including idiopathic pulmonary fibrosis and/or idiopathic pulmonary hypertension). For the fibrosis, for example, fibroblast activation protein α (FAPα) may be the target cell protein.

In some embodiments, the target cell may be a cell that mediates other related diseases, including diseases related to transplant rejection.

The bispecific antibody may bind to target cell proteins from species of mice, rats, rabbits, New World monkeys, and/or Old World monkeys. The species include, but are not limited to, the following species: Mus musculus, Rat tusrattus, Rattus norvegicus, Cynomolgus monkeys, Macaca fascicularis, Hamadryas baboon, Papio hamadryas, Guinea baboon, Papio papio, Olive baboon, Papio anubis, Yellow baboon, Papio cynocephalus, Chacma baboon, Papio ursinus, Callithrix jacchus, Saguinus Oedipus and Saimiri sciureus.

### Cancer

The term "cancer" refers to a broad class of diseases characterized by uncontrolled growth of abnormal cells *in vivo.* "Cancer" includes benign and malignant cancers as well as dormant tumors or micrometastases. Cancer includes primary malignant cells or tumors (e.g., tumors in which cells have not migrated to a site other than the site of the original malignant disease or tumor in the subject) and secondary malignant cells or tumors (e.g., tumors resulting from metastasis in which cells are metastasized to malignant cells or tumor cells and then migrate to a secondary site different from the original tumor site). Cancers also include hematologic malignancies. "Hematologic malignancies" include lymphomas, leukemias, myelomas or lymphoid malignancies, as well as spleen cancer and lymph node tumors.

In a preferred embodiment, the bispecific antibody of the present disclosure or nucleic acids or polynucleotides encoding the antibody of the present disclosure or immunoconjugates or pharmaceutical compositions or combination therapies are useful for the treatment, prevention or alleviation of cancer. Examples of cancers include, but are not limited to, carcinomas, lymphomas, glioblastomas, melanomas, sarcomas, leukemia, myelomas or lymphoid malignancies. More specific examples of such cancers are described below and include: squamous cell carcinoma (e.g., epithelial squamous cell carcinoma), Ewing's sarcoma, Wilms' tumor, astrocytoma, lung cancer (including small-cell lung cancer, non-small-cell lung cancer, lung adenocarcinoma, and lung squamous-cell carcinoma), peritoneal carcinoma, hepatocellular carcinoma, stomach or gastric cancer (including gastrointestinal cancer), pancreatic cancer, glioblastoma multiforme, cervical cancer, ovarian cancer, liver cancer, bladder cancer, hepatoma, hepatocellular carcinoma, neuroendocrine tumor, medullary thyroid cancer, differentiated thyroid cancer, breast cancer, ovarian cancer, colon cancer, rectal cancer, endometrial or uterine carcinoma, salivary gland tumors, kidney or renal carcinoma, prostate cancer, vaginal cancer, anal cancer, penile cancer, and head and neck cancer.

Other examples of cancers or malignancies include, but are not limited to, childhood acute lymphoblastic leukemia, acute lymphoblastic leukemia, acute lymphocytic leukemia, acute myelogenous leukemia, adrenocortical carcinoma, adult (primary) hepatocellular carcinoma, adult (primary) liver cancer, adult acute lymphocytic leukemia, adult acute myelogenous leukemia, adult Hodgkin's lymphoma, adult lymphocytic lymphoma, adult non-Hodgkin's lymphoma, adult primary liver cancer, adult soft tissue sarcoma, AIDS-related lymphoma, AIDS-related malignancies, anal cancer, astrocytoma, cholangiocarcinoma, bladder cancer, bone cancer, brain stem glioma, brain tumor, breast cancer, renal pelvis and ureter cancer, central nervous system (primary) lymphoma, central nervous system lymphoma, cerebellar astrocytoma, cerebral astrocytoma, cervical cancer, childhood (primary) hepatocellular carcinoma, childhood (primary) liver cancer, childhood acute lymphoblastic leukemia, childhood acute myelogenous leukemia, childhood brain stem glioma, childhood cerebellar astrocytoma, childhood cerebral astrocytoma, childhood extracranial blastoma, childhood Hodgkin's disease, childhood Hodgkin's lymphoma, childhood hypothalamic and visual pathway glioma, childhood lymphoblastic leukemia, childhood medulloblastoma, childhood non-Hodgkin's lymphoma, childhood pineal and supratentorial primitive neuroectodermal tumors, childhood primary liver cancer, childhood rhabdomyosarcoma, childhood soft-tissue sarcoma, childhood visual pathway and hypothalamic glioma, chronic lymphocytic leukemia, chronic myelogenous leukemia, colon cancer, cutaneous T-cell lymphoma, endocrine pancreas islet cell carcinoma, endometrial cancer, ependymoma, epithelial cancer, esophageal cancer, Ewing's sarcoma and related tumors, exocrine pancreatic cancer, extracranial blastoma, extragonadal blastoma, cholangiocarcinoma, retinoblastoma, female breast cancer, Gaucher's disease, gallbladder carcinoma, gastric cancer, gastrointestinal benign tumor, gastrointestinal tumors, blastoma, gestational trophoblastic tumor, hairy cell leukemia, head and neck cancer, hepatocellular carcinoma, Hodgkin's lymphoma, hypergammaglobulinemia, hypopharyngeal cancer, intestinal cancers, intraocular melanoma, islet cell carcinoma, islet cell pancreatic cancer, Kaposi's sarcoma, kidney cancer, laryngeal cancer, lip and oral cavity cancer, liver cancer, lung cancer, lymphoproliferative disorders, macroglobulinemia, male breast cancer, malignant mesothelioma, malignant thymoma, medulloblastoma, melanoma, mesothelioma, metastatic primary latent squamous neck cancer, metastatic primary squamous neck cancer, metastatic squamous neck cancer, multiple myeloma, multiple myeloma/plasma cell neoplasm, myelodysplastic syndrome, myelogenous leukemia, myeloid leukemia, myeloproliferative disorders, nasal cavity and paranasal sinus cancer, nasopharyngeal cancer, neuroblastoma, non-Hodgkin's lymphoma, non-melanoma skin cancer, non-small-cell lung cancer, metastatic primary latent metastatic squamous neck cancer, oropharyngeal cancer, osteosarcoma/malignant fibrous sarcoma, osteosarcoma/malignant fibrous histiocytoma, osteosarcoma/malignant fibrous histiocytoma of bone, ovarian epicytoma, ovarian blastoma, ovarian low malignant potential tumor, pancreatic cancer, paraproteinemias, polycythemia vera, parathyroid cancer, penile cancer, pheochromocytoma, pituitary tumor, primary central nervous system lymphoma, primary liver cancer, prostate cancer, rectal cancer, renal cell cancer, renal pelvis and ureter cancer, retinoblastoma, rhabdomyosarcoma, salivary gland cancer, sarcoidosis sarcomas, Sezary syndrome, skin cancer, small-cell lung cancer, small intestine cancer, soft-tissue sarcoma, squamous neck cancer, stomach cancer, supratentorial primitive neuroectodermal and pineal tumors, T-cell lymphoma, testicular cancer, thymoma, thyroid cancer, transitional cell cancer of the renal pelvis and ureter, transitional renal pelvis and ureter cancer, trophoblastic tumors, ureter and renal pelvis cell cancer, urethral cancer, uterine cancer, uterine sarcoma, vaginal cancer, visual pathway and hypothalamic glioma, vulvar cancer, Waldenstrom's macroglobulinemia, Wilm's tumor, and any other hyperproliferative disease, besides neoplasia, located in an organ system listed above.

The bispecific antibody, the nucleic acid encoding the antibody of the present application, the pharmaceutical composition or the combination therapy of the present disclosure may be used for treating malignant or premalignant conditions and preventing the development into neoplastic or malignant conditions, which include, but are not limited to, those disorders described above. Such uses are indicated for conditions that are known or suspected to have previously progressed to neoplasms or cancer. Specifically, non-neoplastic cell growth consists of hyperplasia and metaplasia. Most specifically, developmental abnormalities occur (for the summary of such abnormal growth conditions, see Robbins and Angell, Basic Pathology., 2nd Edition, W.B. Saunders Co., Philadelphia, pp 68-79, 1976).

### Lymphatic system disease

In preferred embodiments, the bispecific antibody, the nucleic acid or polynucleotide encoding the antibody of the present application, the pharmaceutical composition or the combination therapy of the present disclosure is useful for treating, preventing or alleviating lymphatic system diseases. Examples of the lymphatic system diseases include, but are not limited to, what is described below.

### B-cell lymphoma

B-cell lymphoma is a solid neoplasm of B cells and includes Hodgkin's lymphoma (HL) and non-Hodgkin's lymphoma (NHL). The Hodgkin's lymphoma mainly includes classical Hodgkin's lymphoma and nodular lymphocytoma. Diffuse large B-cell lymphoma, follicular lymphoma, mucosa-associated lymphoid tissue (MALT) lymphoma, small lymphocytic lymphoma/chronic lymphocytic leukemia and mantle cell lymphoma (MCL) are five most common types of B-cell non-Hodgkin's lymphoma and account for 3/4 of non-Hodgkin's lymphoma.

B-cell non-Hodgkin's lymphoma includes, but is not limited to, (a) precursor lymphatic neoplasms: B lymphoblastic leukemia/lymphoma and concomitant conditions. (b) Mature B-cell tumors include, but are not limited to, chronic lymphocytic leukemia/small lymphocytic lymphoma, precursor B lymphocytic leukemia, splenic marginal zone lymphoma, hairy cell leukemia, splenic lymphoma/leukemia, lymphoplasmacytic lymphoma, heavy chain disease, plasma cell myeloma/plasmacytoma, extranodal marginal zone B-cell lymphoma of mucosa-associated lymphoid tissue (MALT lymphoma), primary cutaneous follicular center lymphoma, follicular lymphoma (gastrointestinal follicular lymphoma, childhood follicular lymphoma, and "in situ" follicular lymphoma), intranodal marginal zone B-cell lymphoma, mantle cell lymphoma, diffuse large B-cell lymphoma, T-cell/histiocyte rich large B-cell lymphoma, EBV-positive diffuse large B-cell lymphoma of the elderly, diffuse large B-cell lymphoma associated with chronic inflammation, pyothorax-associated lymphoma, chronic osteomyelitis-associated lymphoma, implant-associated lymphoma, primary central nervous system diffuse large B-cell lymphoma, lymphomatoid granulomatosis, primary mediastinal (thymic) large B-cell lymphoma, intravascular large B-cell lymphoma, primary cutaneous large B-cell lymphoma, leg-type plasmablastic lymphoma, primary effusion lymphoma, ALK-positive diffuse large B-cell lymphoma, large B-cell lymphoma arising in HHV8-positive multicentric Castleman disease, Burkitt lymphoma, unclassifiable B-cell lymphoma between diffuse large B-cell lymphoma and Burkitt lymphoma, and unclassifiable B-cell lymphoma between diffuse large B-cell lymphoma and classical Hodgkin's lymphoma.

Some B-cell lymphomas are caused by infection. Non-limiting examples of infectious viruses include Kaposi's sarcoma-associated herpesvirus (primary effusion lymphoma), Epstein-Barr virus (lymphomatoid granulomatosis, post-transplant lymphoproliferative disorder), HIV (AIDS-associated lymphoma) and Helicobacter pylori (mucosa-associated lymphoma).

In addition, B-cell lymphoma is divided into indolent lymphoma and aggressive lymphoma depending on differences in clinical behavior. Indolent lymphoma generally develops slowly, retains stability for many years and long-term survival, and cannot be cured. Aggressive lymphoma generally requires more intensive treatment but is likely to be cured. The prognosis and treatment of B-cell lymphoma depends on the specific types and stages of lymphomas.

### T-cell lymphoma

T-cell lymphoma includes four types of lymphomas that affect T cells: angiocentric lymphoma, cutaneous T cell lymphoma such as Sézary's disease and mycosis fungoides, anaplastic large-cell lymphoma and angioimmunoblastic T-cell lymphoma.

Non-limiting examples of T-cell lymphoma include: acute lymphocytic leukemia (such as acute precursor T lymphocytic leukemia/lymphoma), prolymphocytic leukemia (such as T-cell prolymphocytic leukemia), CD30⁺ landmark leukemia (such as anaplastic large-cell lymphoma and lymphomatoid papulosis), skin diseases (such as mycosis fungoides and Sézary's disease), liver and spleen diseases, vascular diseases, intestinal-related diseases and infectious diseases (such as human T-lymphotropic virus type (e.g., adult T-cell leukemia/lymphoma)).

### B lymphocytic leukemia

Exemplary examples include: acute B lymphocytic leukemia (precursor B-cell lymphocytic leukemia), common B cell leukemia (B chronic lymphocytic leukemia), B-cell prolymphocytic leukemia and CD11c hairy cell leukemia.

### T lymphocytic leukemia

Exemplary examples include: acute T lymphocytic leukemia, acute precursor T lymphocytic leukemia/lymphoma, T-cell prolymphocytic leukemia and adult T-cell leukemia/lymphoma.

### Other lymphoid-related diseases

Examples include: a lymphatic system and a bone marrow system (such as acute biphenotypic leukemia), lymphocytosis such as lymphoproliferative disorders (X-linked recessive progressive combined variable immune deficiency and autoimmune lymphoproliferative syndrome), leukemia-like reactive disorders and pseudolymphoma.

### Autoimmune disease and inflammatory disease

The bispecific antibody, the nucleic acid or polynucleotide encoding the antibody of the present application, the pharmaceutical composition or the combination therapy plays a key role in the pathology associated with a variety of diseases including immune and inflammatory factors. The above diseases include, but are not limited to, rheumatoid arthritis, osteoarthritis, juvenile chronic arthritis, Lyme arthritis, psoriatic arthritis, reactive arthritis, spondyloarthropathy, systemic lupus erythematosus, Crohn's disease, ulcerative colitis, inflammatory bowel disease, insulin-dependent diabetes, thyroiditis, asthma, allergic diseases, psoriasis, scleroderma dermatitis, graft-versus-host disease, organ transplant rejection, acute or chronic immune diseases associated with an organ transplant, sarcoidosis, atherosclerosis, disseminated intravascular coagulation, Kawasaki's disease, Grave's disease, nephrotic syndrome, chronic fatigue syndrome, orbital necrotizing granulomatosis, Henoch-Schonlein purpura, microscopic renal vasculitis, chronic active hepatitis, uveitis, septic shock, toxic shock syndrome, sepsis syndrome, cachexia, infectious diseases, parasitic disease, acquired immunodeficiency syndrome, acute transverse myelitis, chronic chorea, Parkinson's disease, Alzheimer's disease, stroke, Charcot's cirrhosis, hemolytic anemia, malignancy, heart failure, myocardial infarction, Addison's disease, sporadic polyglandular deficiency type I and type II, Schmidt's syndrome, adult (acute) dyspnea syndrome, alopecia, alopecia areata, seronegative arthropathy, arthropathy, Laterl's disease, psoriatic arthropathy, ulcerative oolitic arthropathy, intestinal synovitis, chlamydia, arthropathy associated with Yersinia and Salmonella, arthropathy of spinal tuberculosis, atherosclerosis/arteriosclerosis, specific reactive allergy, autoimmune bowel disease, chronic pemphigus, pemphigus foliaceus, pemphigoid, linear IgA disease, autoimmune hemolytic anemia, Coombs positive hemolytic anemia, acquired pernicious anemia, juvenile pernicious anemia, myalgic encephalitis/Royal Free disease, chronic cutaneous mucosal candidiasis, giant cell arteritis, primary sclerosing hepatitis, cryptogenic autoimmune hepatitis, acquired immunodeficiency syndrome, acquired immunodeficiency-related diseases, hepatitis C, common varied immunodeficiency (few commom variable gamma immunoglobulins in the blood), dilated cardiomyopathy, female infertility, ovarian failure, early decline of ovarian function, fibrotic lung disease, cryptogenci fibrosing alveolitis, postinflammatory interstitial lung disease, interstitial pneumonia, interstitial lung disease associated with connective tissue disease, lung disease associated with mixed connective tissue disease, interstitial lung disease associated with systemic sclerosis, interstitial lung disease associated with rheumatoid arthritis, lung disease associated with systemic lupus erythematosus, lung disease associated with dermatomyositis/polymyositis, disease-associated lung disease, lung disease associated with ankylosing spondylitis, vasculitis diffuse lung disease, hemosiderosis-associated lung disease, drug-induced interstitial lung disease, radiation fibrosis, bronchitis obliterans, chronic eosinophilic pneumonia, lymphocytic infiltrate lung disease, post-infection interstitial lung disease, gouty arthritis, autoimmune hepatitis, type I autoimmune hepatitis (typical autoimmune or lupus hepatitis), type II autoimmune hepatitis (anti-LKM antibody hepatitis), autoimmune-mediated hypoglycemia, type B anti-insulin acanthosis nigricans, hypoparathyroidism, acute immune disease associated with an organ transplant, chronic immune disease associated with an organ transplant, osteoarthropathy, primary sclerosing cholangitis, psoriasis type I, psoriasis type II, spontaneous leucopaenia, autoimmune neutral leukopenia, renal disease NOS, glomerulonephritides, microcosmic renal vasulitis, Lyme disease, discoid lupus erythematosus, spontaneous or NOS male sterility, sperm autoimmunity, multiple sclerosis (all subtypes), sympathetic ophthalmitis, pulmonary hypertension secondary to connective tissue disease, Goodpasture's syndrome, pulmonary manifestations of periarteritis nodosa, acute rheumatic fever, rheumatoid spondylitis, Still's disease, systemic sclerosis, Sjorgren's syndrome, Takayasu's disease/arteritis, autoimmune thrombocytopathy, spontaneous thrombocytopathy, autoimmune thyroid disease, hyperthyroidism, autoimmune hypothyroidism (Hashimoto's disease) that causes goiter, atrophic autoimmune hypothyroidism, initial myxedema, lens-related uveitis, initial vasculitis, vitiligo, central nervous system's disease (e.g., depression, schizophrenia, Alzheimer's disease, Parkinson's disease, etc.), acute and chronic pain and lipid disorders.

### Transplant rejection-related disease

Transplant rejection refers to hyperacute, acute or chronic rejection of a cell, tissue or solid organ to an allograft or xenograft. According to conventional usage, a graft may include one or more organs (such as kidney transplantation or heart lung transplantation), a portion of an organ (such as skin graft), a cell (such as bone marrow transplantation, islet cells, other endocrine or exocrine cells) or a tissue (such as the skin, or a connective tissue such as a cartilage, ligament or tendon). More specific grafts include, but are not limited to, stem cells, corneal tissues, hearts, lungs, heart-lung associations, kidneys, livers, intestines (or other parts of the gastrointestinal tract), pancreases (especially pancreatic islets), tracheae or esophagi, blood vessels, nerves, bones, bone marrows, cartilages, joints, tendons, ligaments, muscles, fat, breasts, gastrointestinal linings, skins, epithelia, endothelia, connective tissues, etc. Similarly, in addition, the present disclosure may be used in combination with body parts including a variety of tissue types, such as the replacements of eyes, ears, nose, fingers (fingers or toes), joints, blood vessels, nerves or muscles or other surgical changes or reconstruction of limbs or other body parts.

Indications include conditions associated with transplant rejection. For example, the following cases are treated (for example, causes or symptoms are ameliorated, reduced, eliminated, or cured) or prevented (substantially or completely) or avoided: (a) acute transplant rejection such as the treatment of a receptor of a heart, lung, heart-lung, liver, kidney, pancreas, skin, bowel or corneal transplant, in particular the prevention and/or treatment of T cell-mediated rejection and a graft-versus-host disease such as bone marrow transplantation; (b) chronic transplant rejection, for example, especially the prevention of graft vascular diseases such as graft artery stenosis due to intimal thickening under smooth muscle cell proliferation and related effects; (c) xenograft transplant rejection including acute, hyperacute, or chronic rejection of an organ when a donor of the organ is a different species from a receptor of the organ, most particularly B cell-mediated rejection or antibody-mediated rejection. The indications further include, for example, adverse reactions after blood transfusion, which include, but are not limited to, fever reactions, allergic reactions, hemolytic reactions, a graft-versus-host disease after blood transfusion, complications after massive blood transfusion (circulatory overload and bleeding tendency), blood transfusion reactions due to bacterial contamination and transfusion transmitted diseases.

A drug may be administered to a patient who has just received or is ready to receive a transplant by any conventional path including, but not limited to, intravenous, intramuscular, oral, subcutaneous, intradermal and parenteral administration.

Since transplant rejection is often accompanied by autoimmune diseases, inflammatory diseases and other related diseases or disorders, a subject to be administered with the CD19 bispecific antibody or the nucleic acid or polynucleotide encoding the antibody of the present application or the pharmaceutical composition or the combination therapy of the present disclosure includes any animal that may exhibit such related diseases or disorders as autoimmune diseases, inflammatory diseases and transplant rejection-related diseases and symptoms. Ideally, the subject is a mammal. More specifically, the mammal includes at least humans, apes, rodents, sheep, cattle, ruminants, lagomorphs, pigs, goats, horses, dogs, rabbits, feline animals and birds, etc. The mammal is most preferably a human. In other embodiments, a host may be a commercially important mammal, or a companion animal or other valuable animals such as a member of an endangered species. According to a preferred embodiment of the present disclosure, a non-human animal is preferably murine, such as guinea pigs, hamsters, gerbils, rats and mice. Therefore, both human physicians and veterinarians may use the bispecific antibody of the present disclosure.

The CD19 bispecific antibody or the nucleic acid or polynucleotide encoding the antibody of the present application or the pharmaceutical composition of the present disclosure may be used in combination with any type of tissue transplant or transplant procedure, in particular a procedure in which the donor (transplanted) tissue is subjected to or at risk of the failure or rejection of an immune system of the receptor host. In particular, the CD19 bispecific antibody or the nucleic acid or polynucleotide encoding the antibody of the present application or the pharmaceutical composition of the present disclosure may be used in any environment where the donor tissue is not histocompatible with the receptor host. A transplant donor may be a non-homologous member of the same phylogenetic species as a transplant receptor (i.e., an allogeneic donor that provides an allograft tissue) or a member of a different phylogenetic species from the transplant receptor (i.e., a xenogeneic donor that provides a xenograft tissue). The donor tissue may come from, for example, a volunteer or other living donors by conventional means. Preferably, the donor can achieve histocompatibility with the receptor host. Thus, autologous and allogeneic donor tissues are preferred when the receptor host is the human. If a xenograft is used as the source of the transplanted tissue, the donor is preferably compatible with the receptor host with respect to MHC; for example, baboons or chimpanzees are preferably used as donors whose tissues are transplanted into humans.

The CD19 bispecific antibody of the present disclosure or the nucleic acid or polynucleotide encoding the antibody of the present application or the pharmaceutical composition may be used for promoting the transplantation of any type of body tissue or organ no matter whether the donor (graft) tissue is an entire organ, a portion of an organ or a tissue or an isolated cell. In some embodiments, the donor tissue includes organs or body parts. In other embodiments, the donor tissue includes a portion or a biopsy of a donor organ or tissue. In other embodiments, the donor tissue includes a cell, especially an isolated or suspended cell, which includes cells taken out or removed from donor hosts, cells maintained in primary culture or immortalized cell lines. Optionally, the donor tissue may include cells carrying exogenous genetic substances, such as transfected or transformed host cells, which include (or are derived from ancestral cells that have been modified) genetic substances necessary to produce a polypeptide of therapeutic value. In other embodiments, the donor tissue may be derived from a transgenic mammal that has been engineered to include genetic substances necessary to produce a polypeptide of therapeutic value to the receptor host in some or all body tissues of the transgenic mammal. Exemplary polypeptides of therapeutic value to receptors include hormones such as insulin or growth hormone; cytokines; growth and differentiation factors; enzymes; and structural proteins; etc.

An effective dose for inhibiting transplant rejection is about 0.001 mg to 10.0 mg per kilogram in body weight. The dose selected within the range of about 0.001-10.0 mg/kg in body weight is effective and non-toxic. The dose used will be administered to a patient once to six times daily.

The present disclosure provides a method for inhibiting transplant rejection. The method includes administering an effective amount of the CD19 bispecific antibody or the nucleic acid or polynucleotide encoding the antibody of the present application or the pharmaceutical composition defined herein for inhibiting the transplant rejection.

### Pharmaceutical composition

The bispecific antibody (such as CD19xCD3) of the present disclosure or the nucleic acid or polynucleotide encoding the antibody of the present application may be applied to the preparation of the pharmaceutical composition or a sterile composition. For example, the bispecific antibody is mixed with a pharmaceutically acceptable carrier, excipient or stabilizer. The pharmaceutical composition may include one bispecific antibody or a combination of (for example, two or more different) bispecific antibodies of the present disclosure. For example, the pharmaceutical composition of the present disclosure may include a combination of antibodies or antigen-binding fragments that have complementary activity and bind to different epitopes on a target antigen. Formulations of therapeutic and diagnostic agents may be prepared by mixing the antibody with the pharmaceutically acceptable carrier, excipient or stabilizer in the form of, for example, lyophilized powder, slurry, an aqueous solution or a suspension.

The term "pharmaceutically acceptable" means that a molecule, molecule fragment or composition does not produce unfavorable, allergic or other adverse effects when properly administered to animals or humans. Specific examples of some substances that may act as the pharmaceutically acceptable carriers or components thereof include sugars (such as lactose), starch, cellulose and its derivatives, vegetable oils, gelatin, polyols (such as propylene glycol), alginic acid and the like.

In some preferred embodiments, the pharmaceutical composition of the present disclosure is used for treating, preventing or alleviating diseases including, but not limited to, immune-related diseases, cancer, autoimmune diseases, inflammatory diseases and transplant rejection-related diseases or conditions.

The bispecific antibody of the present disclosure or the nucleic acid or polynucleotide encoding the antibody of the present application may be used alone or in combination with one or more other pharmaceutically active agents such as antibodies, antibody fragments, drugs, enzymes, cytotoxic agents, toxins, antibiotics, hormones, immunomodulators, cytokines, chemokines or radioisotopes.

The drugs include, but are not limited to, cyclophosphamide, nimustine, amethopterin, fluorouracil, capecitabine, gemcitabine, tigio, pemetrexed, fludarabine, doxorubicin, bleomycin, vinorelbine, paclitaxel, docetaxel, irinotecan, tamoxifen, letrozole, exemestane, fulvestrant, goserelin, medroxyprogesterone, cisplatin, carboplatin, oxaliplatin, nedaplatin, oxaliplatin, asparaginase, lobaplatin, etoposide, vincristine, irinotecan, tegafur, dacarbazine, mitomycin, teniposide, pirarubicin, mitoxantrone, vindesine, raltitrexed, methotrexate, cisplatin bleomycin sulfate, carmustine, chlorambucil, cyclophosphamide hydroxyurea and daunomycin. These therapeutic agents themselves are only effective when they are toxic or subtoxic to patients. The above therapeutic agents are well known in the art. Therapeutic agents that may be developed in the future are also included.

The toxins include, but are not limited to, ricin, abrin, α toxin, saponin, ribonuclease (RNase), DNase I, staphylococcal enterotoxin-A, pokeweed antiviral protein, gelonin, diphtheria toxin, Pseudomonas exotoxin and Pseudomonas endotoxin.

The bispecific antibody of the present disclosure or the nucleic acid or polynucleotide encoding the antibody of the present application may be administered along with, for example, effector cell co-stimulatory molecules, effector cells, DC cell surface molecules, DC cells and molecules activating T cells (Hutloff A et al., Nature, 397: 262-266, 1999). The effector cells may be human leukocytes such as macrophages, neutrophils or monocytes. Other cells include eosinophils, NK cells and other cells with IgG or IgA receptors. If required, effector cells may be obtained from subjects to be treated. Target-specific effector cells may be administered as a suspension of cells in a physiologically acceptable solution. The number of cells administered may be within a range of 10⁸ to 10⁹ and may vary depending on the purpose of treatment. In general, this number is sufficient to locate target cells (such as tumor cells expressing CD19) and kill the target cells. In the presence of a complement, the composition of the present disclosure with complement binding sites may also be used. The complement binding sites are from, for example, IgG1, IgG2, IgG4 or IgM moieties bound to the complement. The composition of the present disclosure may also be administered with a complement such as C1q.

The bispecific antibody of the present disclosure or the nucleic acid or polynucleotide encoding the antibody of the present application may be administered in combination with, for example, immunomodulators or immunogenic agents such as cancer cells, purified tumor antigens (including recombinant proteins, peptides and carbohydrate molecules) or cells transfected with genes encoding immunostimulatory cytokines (He YF et al., J. Immunol., 173: 4919-28, 2004).

The immunomodulators may include, but are not limited to, cytokines, chemokines, a stem cell growth factor, lymphotoxins, hematopoietic factors, colony-stimulating factors (CSFs), interleukins (ILs), erythropoietin, platelet growth factors, tumor necrosis factor-α (TNF-α), TNF-β, a granulocyte-colony stimulating factor (G-CSF), a granulocyte macrophage-colony stimulating factor (GM-CSF), interferon-a, interferon-β, interferon-γ, interferon-A, a stem cell growth factor known as "factor S1", human growth hormone, N-methionyl human growth hormone, bovine growth hormone, parathyroid hormone, thyroxine, insulin, proinsulin, relaxin, prorelaxin, follicle stimulating hormone (FSH), thyroid stimulating hormone (TSH), luteinizing hormone (LH), a liver growth factor, prostaglandin, fibroblast growth factor, prolactin, placental prolactin, OB protein, mullerian inhibiting substance, mouse gonadotropin-associated peptide, inhibin, activin, a vascular endothelial growth factor, integrin, NGF-β, platelet growth factors, TGF-α, TGF-β, insulin-like growth factor-I, insulin-like growth factor-II, a macrophage CSF (M-CSF), IL-1, IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-23, IL-25, LIF, FLT-3, the vascular endothelial growth factor, thrombospondin and endostatin. In some embodiments, the bispecific antibody or the antibody fragment may be linked to an immunomodulator such as a cytokine. Cytokine complexes are disclosed, for example, in U.S. Pat. Nos. 7906118 and 8,034, 3522, examples of each of which are incorporated herein by reference in part. Preferred immunomodulators include interferon-a, interferon-β and interferon-A.

The bispecific antibody of the present disclosure or the nucleic acid or polynucleotide encoding the antibody of the present application may also be administered in combination with, for example, standard cancer therapies (such as a surgery, radiation and chemotherapy). For example, anti-tumor therapy using the compositions of the present disclosure and/or effector cells equipped with these compositions is used in combination with chemotherapy. Non-limiting examples of antibody combination therapies of the present disclosure include surgery, chemotherapy, radiotherapy, immunotherapy, gene therapy, DNA therapy, RNA therapy, nanotherapy, viral therapy, adjuvant therapy and combinations thereof.

The bispecific antibody or the nucleic acid or polynucleotide encoding the antibody of the present application may be linked to agents (as immune complexes) or administered separately from the agents. In the latter case, the bispecific antibody or the nucleic acid or polynucleotide encoding the antibody of the present application may be administered before, after or together with the agents or may be administered with other known therapies (such as anticancer therapies or radiation).

The pharmaceutical composition of the present application includes a nucleic acid or polynucleotide encoding a protein. The nucleic acid may be administered *in vivo* to promote the expression of the protein encoded by the nucleic acid or administered by constructing the nucleic acid as a portion of an appropriate nucleic acid expression vector so that the nucleic acid enters a cell. For example, the nucleic acid is administered by using a retroviral vector (see U.S. Pat. No. 4980286), through direct injection, through particle bombardment (such as a gene gun, Biolistic, Dupont), by coating the nucleic acid with a lipid, cell surface receptor or transfection agent, or by linking the nucleic acid to a homeobox peptide. The homeobox peptide is known to enter a nucleus (see, for example, Joliot A et al., Proc. Natl. Acad. Sci. 88: 1864-1868, 1991). Alternatively, the nucleic acid may be introduced intracellularly and bound to DNA of a host cell to be expressed through homologous recombination.

The compositions of the present disclosure may be in various forms. The forms include, for example, liquid, semi-solid and solid dosage forms such as liquid solutions (e.g. injectable and non-molten solutions), dispersants, suspensions tablets, pills, powders, liposomes and suppositories. A preferred manner depends on the mode of administration and the therapeutic use. Preferred typical compositions are injectable or non-molten solutions, such as those compositions that passively immunize humans like other antibodies. The compositions of the present disclosure may be administered through various routes including oral, rectal, transmucosal, trans-intestinal, parenteral; intramuscular, subcutaneous, intradermal, intramedullary, intrathecal, direct intraventricular, intravenous, intraperitoneal, intranasal, intraocular, inhalation, insufflation, topical, skin, transdermal, or intraarterial. A preferred mode of administration is parenteral (e.g., intravenous, subcutaneous, intraperitoneal, intramuscular). In a preferred embodiment, the antibody is administered by intravenous infusion or injection. In another preferred embodiment, the antibody is injected intramuscularly or subcutaneously.

The above combination methods, treatment methods and administration methods are well known. Combination, treatment and administration methods that may be developed in the future are also included.

An appropriate dose is determined by a clinician, for example, by using parameters or factors known or suspected to affect treatment in the art. Generally, the dose begins with a dose slightly smaller than an optimal dose and then increases by small increments until the desired or optimal effect is achieved relative to any negative side effects. Important diagnostic measures include, for example, symptoms of inflammation or levels of produced inflammatory cytokines.

The present disclosure provides a container (such as a plastic or glass vial having, for example, a cover, a chromatography column, a hollow needle or a syringe cylinder) that includes any antibody of the present disclosure or the nucleic acid or polynucleotide encoding the antibody of the present application, and instructions. The present disclosure further provides an injection device including any antibody of the present disclosure or the nucleic acid or polynucleotide encoding the antibody of the present application.

### Combination therapy

The present disclosure relates to uses of a combination of the bispecific antibody or nucleic acids or polynucleotides encoding the antibody of the present disclosure or immunoconjugates or pharmaceutical compositions and one or more active therapeutic agents (e.g., chemotherapeutic agents) or other prophylactic or therapeutic modes (e.g., radiation). In such combination therapies, the various active agents often have different complementary mechanisms of action, and the combination therapy may lead to synergistic effects. The combination therapy includes therapeutic agents that affect immune responses (e.g., an enhanced or activated response) and therapeutic agents that affect (e.g., inhibit or kill) tumor/cancer cells. The combination therapy may reduce the likelihood of drug-resistant cancer cells. The combination therapy may allow the dose reduction of one or more reagents to reduce or eliminate adverse effects associated with the one or more reagents. Such combination therapies may have synergistic therapeutic or prophylactic effects on underlying diseases, disorders or symptoms.

The "combination" includes therapies that can be administered separately, for example, separate formulations for individual administration (e.g., which may be provided in a kit), and therapies that can be administered together in a single formulation (i.e., "co-formulation"). In some embodiments, the bispecific antibody of the present disclosure or nucleic acids or polynucleotides encoding the antibody of the present disclosure or immunoconjugates or pharmaceutical compositions may be administered sequentially. In some embodiments, the bispecific antibody of the present disclosure or nucleic acids or polynucleotides encoding the antibody of the present disclosure or immunoconjugates or pharmaceutical compositions may be administered simultaneously. The bispecific antibody or nucleic acids or polynucleotides encoding the antibody of the present disclosure or immunoconjugates or pharmaceutical compositions may be used in any manner in combination with at least one other (active) agent.

Treatment with the bispecific antibody of the present disclosure may be combined with other treatments that are effective against the to-be-treated disease. Non-limiting examples of antibody combination therapies of the present disclosure include surgery, chemotherapy, radiation therapy, immunotherapy, gene therapy, DNA therapy, RNA therapy, nanotherapy, viral therapy, and adjuvant therapy.

Combination therapies also include all other combination therapies known in the art or developed in the future.

### Detection method and kit

In some aspects, the present disclosure provides a method for detecting (e.g., *in vitro* or *in vivo)* the presence or level of a TAA in a sample (such as a biological sample, e.g., blood, serum, semen, urine or tissue biopsy samples (from, for example, a hyperproliferative or cancerous lesion)). The method may be used for evaluation (e.g., monitoring the treatment or progress, diagnosis and/or staging of the disease (e.g., immune disorders or cancer) as described in the present disclosure in a subject). The method may include: (i) incubating the sample with an antibody specific for the TAA; (ii) detecting a TAA complex using a detectable probe; (iii) comparing the amount of (ii) with a standard curve obtained from a reference sample containing a known amount of TAAs; and (iv) calculating the amount of TAAs in the sample based on the standard curve. The formation of the complex indicates the presence of the TAA and may indicate the suitability of or need for the treatment described herein. The method may involve, for example, immunohistochemistry, immunocytochemistry, flow cytometry, antibody molecule complexed magnetic beads, an ELISA and a PCR technology (e.g., RT-PCR). Generally, the bispecific antibody molecule or the nucleic acid or polynucleotide encoding the bispecific antibody used in *in vivo* and *in vitro* diagnostic methods is directly or indirectly labelled with a detectable substance to output a detection signal. Appropriate detectable substances include, but are not limited to, various biologically active enzymes, prosthetic groups, fluorescent substances, luminescent substances and radioactive substances.

The present disclosure further provides a reagent and a kit for diagnosis or detection including one or more bispecific antibodies or nucleic acids or polynucleotides encoding the bispecific antibodies of the present disclosure. In one embodiment, the bispecific antibody or the nucleic acid or polynucleotide encoding the bispecific antibody and a pharmaceutically acceptable carrier are optionally combined with one or more therapeutic agents and optionally formulated together in a pharmaceutical composition. The reagent and the kit are used in a variety of assays including, for example, immunoassays such as ELISA (in a sandwich or competitive form). The kit may include other additives such as stabilizers and buffers (such as blocking buffers or lysis buffers). Specifically, the reagent may be provided as dry powder which is generally lyophilized, includes an excipient, and will provide a solution of reagent with an appropriate concentration after dissolved.

Components of the kit may be pre-attached to a solid support or may be applied to the surface of the solid support when the kit is used. In some embodiments of the present disclosure, a signal generation device may be pre-associated with the bispecific antibody or the nucleic acid or polynucleotide encoding the bispecific antibody of the present disclosure or combined with one or more components before used, such as buffers, antibody-enzyme conjugates and enzyme substrates. In a specific aspect, an enzyme that catalyzes the formation of a chemiluminescent or chromogenic product or the reduction of a chemiluminescent or chromogenic substrate is a component of the signal generation device. An enzyme label is generally detected by providing the enzyme with a substrate and detecting the reaction product produced by the action of the enzyme on the substrate, and a calorimetric label is detected by simply visualizing the colored label. In some embodiments, the detectable label as described above may be linked to a recombinant protein of the present disclosure through linkers of different lengths to reduce potential steric hindrance. Such enzymes are well known in the art.

The kit may also include an additional reagent such as a blocking reagent for reducing the non-specific binding to a solid phase surface, a washing reagent or an enzyme substrate. The solid phase surface may be in the form of tubes, beads, microtiter plates, microspheres or other materials suitable for immobilizing proteins, peptides or polypeptides.

The components of the kit may be packaged together or divided into two or more containers. In some embodiments, the container may be a vial containing a sterile lyophilized formulation of a composition suitable for recombination. Other containers that may be used include, but are not limited to, bags, trays, boxes and tubes, etc. The kit may also contain one or more buffers suitable for restoring and/or diluting other reagents.

In one embodiment, the kit includes the bispecific antibody or the nucleic acid or polynucleotide encoding the antibody of the present application packaged in the container.

In one embodiment, the kit includes the bispecific antibody or the nucleic acid or polynucleotide encoding the antibody of the present application packaged in the container and one or more pharmaceutically acceptable carriers.

In one embodiment, the kit includes a combination of the present disclosure, which includes the bispecific antibody of the present disclosure or the nucleic acid or polynucleotide encoding the antibody of the present application and one or more pharmaceutically acceptable carriers in a single common container and is optionally combined with one or more therapeutic agents and formulated in a pharmaceutical composition.

In one embodiment, the kit includes the bispecific antibody of the present disclosure or the nucleic acid or polynucleotide encoding the antibody of the present application or the pharmaceutical composition in one container and the pharmaceutical composition and/or a therapeutic agent in another container.

The concomitant administration of two therapeutic agents does not require that the agents be administered concurrently or by the same path, as long as there is an overlap between time periods during which the agents exert respective therapeutic effects.

In another aspect, the above kit includes a tag attached to or packaged with a container. The tag describes contents in the container and provides indications and/or a method of using the contents in the container. The tag may be used as instructions for treating, preventing and/or diagnosing one or more disease states as described herein.

Optionally, the kit may further include a syringe for parenteral administration, such as intravenous administration. The kit also includes a device for tightly accommodating a vial for commercial sale and/or easy packaging and delivery. The kit also includes a device or equipment for performing the detection or monitoring method as described herein.

### Use

As described herein, the bispecific antibody, the nucleic acid or polynucleotide encoding the antibody of the present application, the pharmaceutical composition or the combination therapy of the present application may be used in the treatment and diagnosis of immune-related diseases, cancer, autoimmune diseases, inflammatory diseases and transplant rejection-related diseases or conditions described herein. The treatment includes administering the bispecific antibody, the nucleic acid or polynucleotide encoding the bispecific antibody, the pharmaceutical composition or the combination therapy of the present application to a patient such as an animal, a mammal and a human, for the treatment or diagnosis of one or more diseases or symptoms described herein. Therapeutic compounds of the present application include, but are not limited to, the antibody (including variants, isotypes, derivatives and species homologues thereof as described herein) of the present application or the nucleic acid or polynucleotide (including variants, isotypes, derivatives and species homologues thereof as described herein) encoding the antibody of the present application.

The bispecific antibody, the nucleic acid or polynucleotide encoding the antibody of the present application or the pharmaceutical composition of the present disclosure may be used for diagnostic purposes. In one embodiment, the antibody of the present disclosure is used for diagnostic tests *in vitro* such as laboratory tests for detecting antigens of interest or care tests for detecting antigens of interest. Established *in vitro* tests using an antibody include ELISAs, RIAs, Western blotting, etc. In another embodiment, the antibody of the present disclosure is used for diagnostic tests *in vivo* such as imaging tests *in vivo.* For example, the antibody is labeled with a detectable substance that can be detected *in vivo,* the labelled antibody may be applied to a subject, and the labelled antibody may be detected *in vivo,* whereby *in vivo* imaging may be performed. Related diagnostic techniques include other well-known techniques and techniques that may be developed.

The bispecific antibody of the present disclosure or the nucleic acid or polynucleotide encoding the antibody of the present application may be used for preparing a drug. The pharmaceutical composition includes, but is not limited to, pharmaceutical compositions described above. The bispecific antibody of the present disclosure or the nucleic acid or polynucleotide encoding the antibody of the present application may be used alone or in combination with one or more other therapeutic agents. The therapeutic agents include, but are not limited to, those described above. In some preferred embodiments, the bispecific antibody or the nucleic acid or polynucleotide encoding the antibody of the present application and other pharmaceutically active agents are provided as separate components or components of the same composition, or the bispecific antibody or the nucleic acid or polynucleotide encoding the antibody of the present application may be administered with other known therapies. Other known therapies include, but are not limited to, those described above. Combination, treatment and administration methods of the pharmaceutical composition include, but are not limited to, those described above. Further provided are a container that includes the bispecific antibody or the nucleic acid or polynucleotide encoding the antibody of the present application or the pharmaceutical composition of the present disclosure, and instructions.

The present disclosure provides a combination therapy that includes the bispecific antibody of the present disclosure or the nucleic acid or polynucleotide encoding the antibody of the present application or the pharmaceutical composition. The combination therapy includes, but is not limited to, those described above.

The present disclosure further provides a detection method and a kit that include the bispecific antibody of the present disclosure or the nucleic acid or polynucleotide encoding the antibody of the present application or the pharmaceutical composition. Related content includes, but is not limited to, those described above.

The bispecific antibody or the nucleic acid or polynucleotide encoding the antibody of the present application or the pharmaceutical composition of the present application may be used for diagnosing, treating, inhibiting, or preventing diseases or symptoms, which include malignant diseases, disorders, or conditions related to such diseases or disorders, such as diseases related to increased cell survival or inhibited apoptosis, such as, but not limited to, immune-related diseases, cancer, autoimmune diseases, inflammatory diseases and transplant rejection-related diseases or conditions described above. For example, such diseases involve unregulated and/or inappropriate proliferation of cells, sometimes accompanied by destruction of adjacent tissues and new vascular growth, which may allow cancer cells to invade new regions, i.e. metastases.

The bispecific antibody of the present application or the nucleic acid or polynucleotide encoding the antibody of the present application or the pharmaceutical composition may be used for treating, preventing, diagnosing and/or predicting other diseases or symptoms associated with increased cell survival, which include, but are not limited to, the development and/or metastasis of malignancies and the development and/or metastasis of the other related diseases or symptoms (such as inflammatory diseases or lymphatic system diseases) described above.

The form of the bispecific antibody or the nucleic acid or polynucleotide encoding the antibody of the present application or the pharmaceutical composition includes, but is not limited to, those described above. The method for administering the bispecific antibody of the present disclosure or the nucleic acid or polynucleotide encoding the antibody of the present application or the pharmaceutical composition includes, but is not limited to, those described above. Treatment methods and combination therapies include, but are not limited to, those described above.

For example, the bispecific antibody or the nucleic acid or polynucleotide encoding the antibody of the present application or the pharmaceutical composition may be intravenously administered through, for example, bolus injection or continuous infusion or even administered to mammals by other parenteral means such as intravenous, intramuscular, intraperitoneal or intravascular administration. Preferably, the bispecific antibody or the nucleic acid or polynucleotide encoding the antibody of the present application or the pharmaceutical composition is injected continuously for less than about 4 hours, and more preferably, for less than about 3 hours. An injectable formulation may be provided in unit dosages, for example, in ampoules or multi-dosage containers where preservatives are added. The composition may be in the form of for example, a suspension, solution, or emulsion in an oily or aqueous vehicle and may contain formulations such as suspensions, stabilizers and/or dispersants. Alternatively, active components may be in the form of powder reconstituted with an appropriate vehicle (e.g. sterile pyrogen-free water) before used.

Various delivery systems are known and may be used for administering the antibody of the present application or the nucleic acid or polynucleotide encoding the antibody of the present application or the pharmaceutical composition, for example, encapsulation in liposomes, microparticles, microcapsules, recombinant cells capable of expressing the compound, or receptor-mediated endocytosis (see Wu GY et al., J. Biol. Chem., 262: 4429-4432, 1987), construction of the nucleic acid as a retrovirus or other vectors. The delivery systems also include all other well-known techniques and parts that may develop in the future.

Additional pharmaceutical methods may be used for controlling the duration of the action of the therapeutic composition, such as a controlled release system. Non-limiting examples of the controlled release system are pumps (see, for example, Sefton, CRC, Crit. Ref. Biomed. Eng., 14: 201, 1987; Buchwald H et al., Surgery, 88: 507, 1980), polymeric materials (see, for example, Medical Applications of Controlled Release, Langer and Wise, CRC Pres. Boca Raton, Fla., 1974) or the like. Such pharmaceutical methods also include all other well-known techniques and parts that may develop in the future.

A therapeutically effective dose of the bispecific antibody or the nucleic acid or polynucleotide encoding the antibody of the present application or the pharmaceutical composition may be administered. The therapeutically effective dose of the bispecific antibody or the polynucleotide encoding the antibody of the present application or the pharmaceutical composition may vary depending on an indication being treated, the weight of a patient and the calculated surface area of the skin of the patient. The administration may be adjusted in order to achieve desired effects. In many cases, repeated administration may be required. For example, the administration may be conducted three times, twice or once every week, once every two, three, four, five, six, seven, eight, nine or ten weeks, or once every two, three, four, five or six months. The bispecific antibody of the present application or the nucleic acid or polynucleotide encoding the antibody of the present application is generally administered to the patient at a dosage of about 0.001-100 mg/kg, for example, about 0.1-50 mg/kg, about 0.1-20 mg/kg, about 0.1-10 mg/kg, about 0.5 mg/kg, about 0.3 mg/kg, about 1 mg/kg, or about 3 mg/kg. Optionally, the dosage at which the antibody is administered may be adjusted based on the estimated surface area of the skin of the patient or based on the weight of the patient. The administration schedule may optionally be repeated at other intervals, and the dosage may be administered by various parenteral means with the appropriate adjustment of the dosage and a schedule.

In general, a human antibody has a longer half-life in humans than an antibody from other species, due to the immune response to exogenous polypeptides. Therefore, a low dose of human antibodies may be usually allowed to be administered at a lower frequency. In addition, the administration dosage and frequency of the antibody of the present application may be reduced by enhancing the absorption and tissue penetration (e.g., into the brain) of the antibody, which is achieved through modifications, e.g., lipidation.

The bispecific antibody of the present disclosure or the nucleic acid or polynucleotide encoding the antibody of the present application may be used for preparing a diagnostic or therapeutic kit which includes the bispecific antibody of the present disclosure or the nucleic acid or polynucleotide encoding the antibody of the present application and/or instructions for use. The pharmaceutical composition of the present disclosure may be used for preparing a diagnostic or therapeutic kit which includes the pharmaceutical composition of the present disclosure and/or instructions for use.

### BRIEF DESCRIPTION OF DRAWINGS

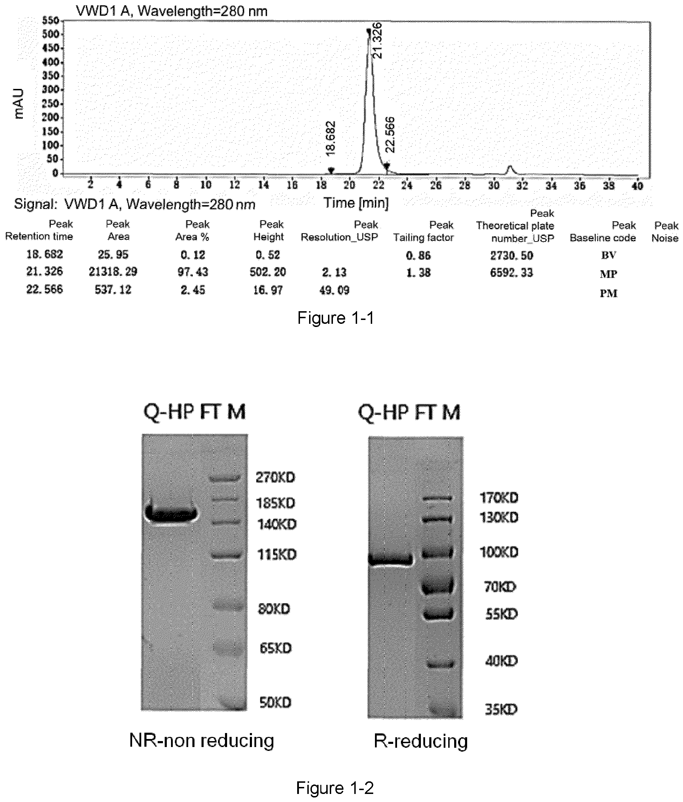

FIG. 1-1 illustrates SEC-HPLC test results of purified samples of a bispecific antibody against CD19.
FIG. 1-2 illustrates SDS-PAGE electrophoresis results of purified samples of a bispecific antibody against CD19.
FIG. 2-1 illustrates an ability detected through an FACS of a bispecific antibody AB23P7 to bind to tumor cells Raji.
FIG. 2-2 illustrates an ability detected through an FACS of a bispecific antibody AB23P8 to bind to tumor cells Raji.

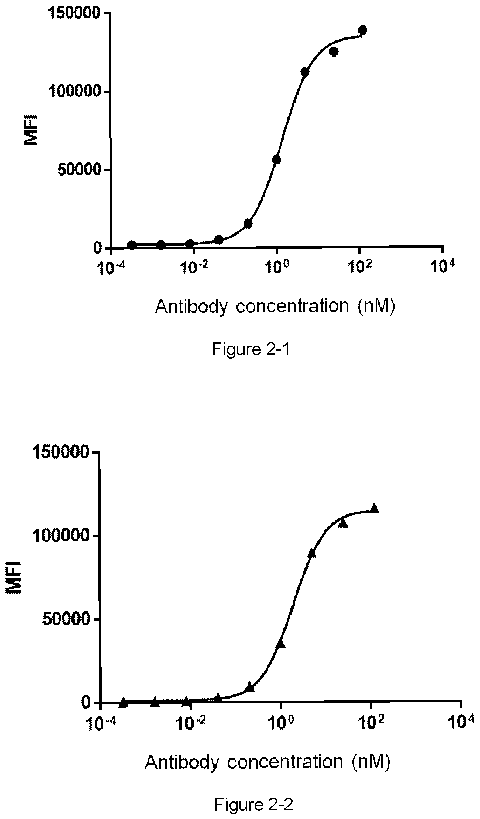

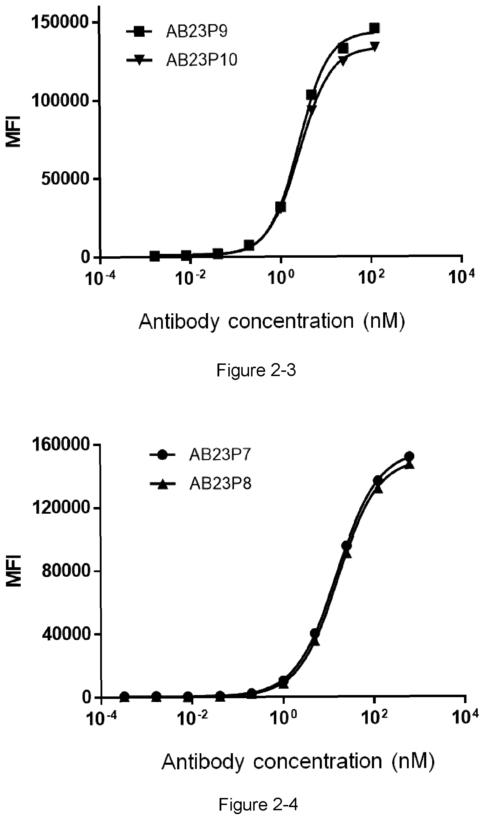

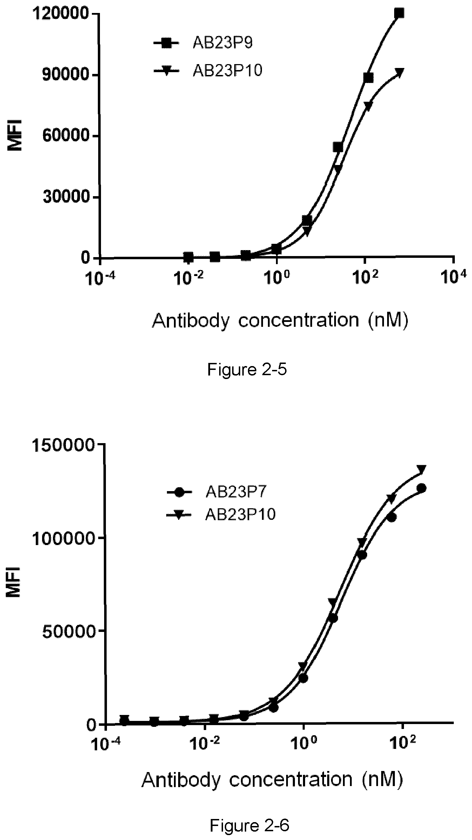

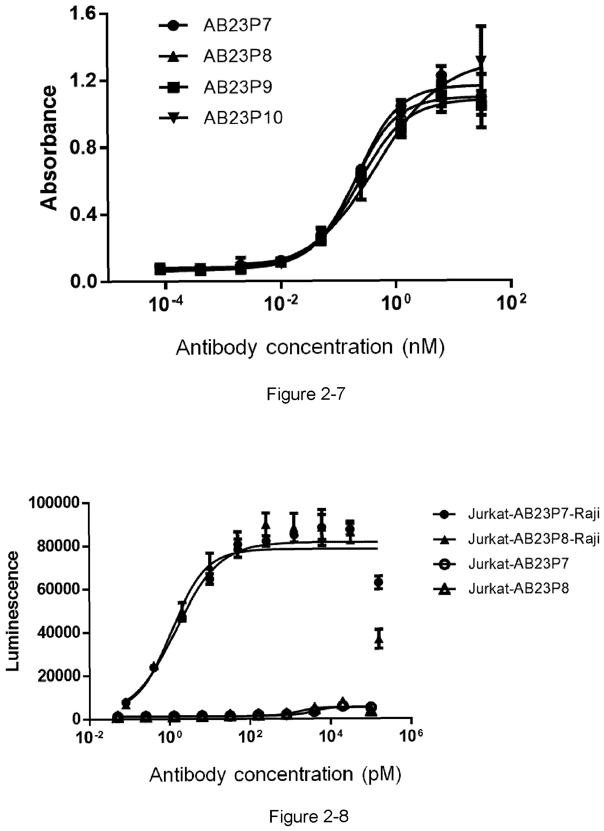

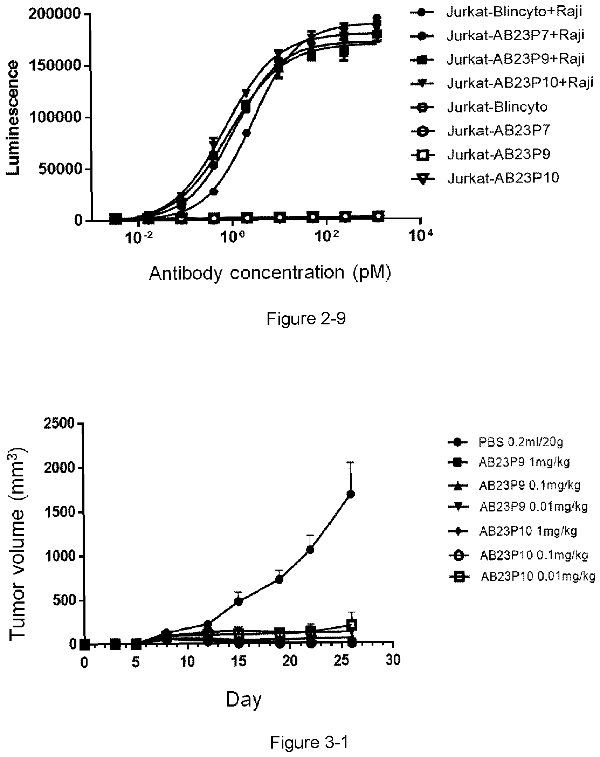

FIG. 2-3 illustrates abilities detected through an FACS of bispecific antibodies AB23P9 and AB23P10 to bind to tumor cells Raji.
FIG. 2-4 illustrates abilities detected through an FACS of bispecific antibodies AB23P7 and AB23P8 to bind to effector cells CIK.
FIG. 2-5 illustrates abilities detected through an FACS of bispecific antibodies AB23P9 and AB23P10 to bind to effector cells CIK.
FIG. 2-6 illustrates abilities detected through an FACS of bispecific antibodies AB23P7 and AB23P10 to bind to T cells of a cynomolgus monkey.
FIG. 2-7 illustrates abilities detected through an ELISA of four anti-CD19×CD3 bispecific antibodies to bind to CD3 molecules and CD19 molecules.
FIG. 2-8 illustrates abilities detected through a microplate reader of bispecific antibodies AB23P7 and AB23P8 to activate Jurkat T cells of a reporter gene cell strain.
FIG. 2-9 illustrates abilities detected through a microplate reader of four anti-CD19xCD3 bispecific antibodies to activate Jurkat T cells of a reporter gene cell strain.
FIG. 3-1 illustrates *in vivo* antitumor effects of bispecific antibodies AB23P9 and AB23P10 in NPG mouse models of transplanted tumor constructed by co-inoculating subcutaneously human CIK cells and Raji cells .

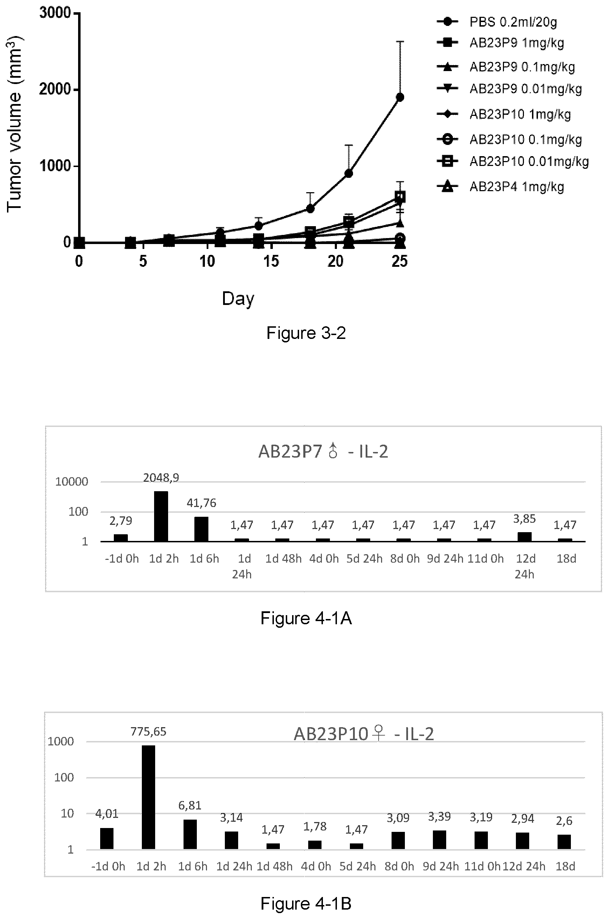

FIG. 3-2 illustrates *in vivo* antitumor effects of bispecific antibodies, Blincyto, AB23P9 and AB23P10 in NPG mouse models of transplanted tumor constructed by co-inoculating subcutaneously human CIK cells and Daudi cells.

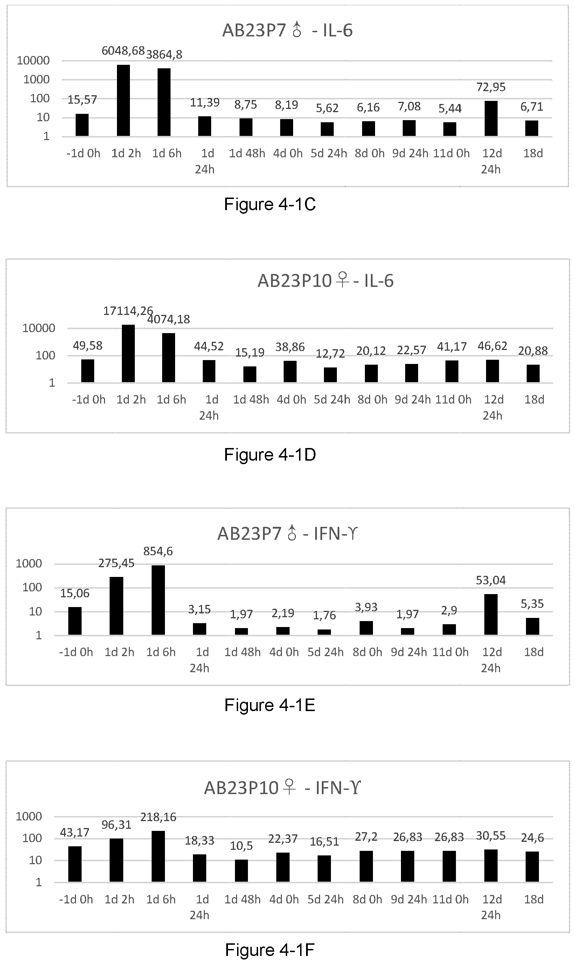

FIG. 4-1A illustrates the determination of content of cytokine IL-2 in serum of a normal male cynomolgus monkey administered with a bispecific antibody AB23P7.
FIG. 4-1B illustrates the determination of content of cytokine IL-2 in serum of a normal female cynomolgus monkey administered with a bispecific antibody AB23P10.
FIG. 4-1C illustrates the determination of content of cytokine IL-6 in serum of a normal male cynomolgus monkey administered with a bispecific antibody AB23P7.
FIG. 4-1D illustrates the determination of content of cytokine IL-6 in serum of a normal female cynomolgus monkey administered with a bispecific antibody AB23P10.
FIG. 4-1E illustrates the determination of content of cytokine IFN-γ in serum of a normal male cynomolgus monkey administered with a bispecific antibody AB23P7.
FIG. 4-1F illustrates the determination of content of cytokine IFN-γ in serum of a normal female cynomolgus monkey administered with a bispecific antibody AB23P10.

### DETAILED DESCRIPTION

The present disclosure is further described through examples which should not be construed as further limitations. All drawings, all reference documents, and the contents of patents and published patent applications cited in the entire application are expressly incorporated herein by reference.

### Example 1 Design and preparation of anti-CD19xCD3 bispecific antibody

### 1.1 Design of bispecific antibody

In Chinese Patent Application No. 201811294887.4 to which the present application claims priority, six different configurations were designed for an exemplary bispecific antibody against Her2 and CD3. One configuration similar to AB7K7 was selected and optimized herein so that anti-CD19xCD3 bispecific antibodies with the following structure were derived. Specifically, the configuration of the bispecific antibodies and their composition characteristics from N-terminus to C-terminus are described below.

Seven antibodies including bispecific antibodies AB23P7, AB23P8, AB23P9 and AB23P10 were each composed of an anti-CD19 scFv, a linker peptide L2, an anti-CD3 scFv and an Fc fragment in sequence, and VH and VL within the anti-CD19 scFv and VH and VL within the anti-CD3 scFv were linked by a linker peptide L1 and a linker peptide L3, respectively. In the amino acid sequences of first five anti-CD19 scFvs contained in the bispecific antibodies listed in Table 1-1, VH domains and VL domains thereof are sequentially made reference to: a bispecific antibody Blinatumomab (whose sequence was from an antibody that binds to CD19 in U.S. Pat. No. US10/554852 and whose heavy chain variable region and light chain variable region are as shown in SEQ ID NOs: 7 and 8, respectively), an antibody conjugate Coltuximab (whose sequence was from an antibody HuB4 that binds to CD19 in U.S. Pat. No. US14/117806 and whose heavy chain variable region and light chain variable region are as shown in SEQ ID NOs: 15 and 16, respectively), an antibody conjugate Denintuzumab (whose sequence was from an antibody hBU12 that binds to CD19 in Chinese patent No. 201580017717.X and whose heavy chain variable region and light chain variable region are as shown in SEQ ID Nos: 22 and 63, respectively), a monoclonal antibody Inebilizumab (whose sequence was from an antibody that binds to CD19 in U.S. Pat. No. US15/863144 and whose heavy chain variable region and light chain variable region are as shown in SEQ ID Nos: 30 and 31, respectively) and a bispecific antibody Duvortuxizumab (whose sequence was from an antibody that binds to CD19 in Chinese patent No. 201580051198.9 and whose heavy chain variable region and light chain variable region are as shown in SEQ ID Nos: 34 and 35, respectively). Fc fragments contained in the bispecific antibodies were all from human IgG1 and had multiple amino acid substitutions L234A, L235A, T250Q, N297A, P331S and M428L (EU numbering) and a deleted or removed K447 (EU numbering) at the C-terminus of the Fc fragment. Each linker peptide (L2) consisted of a flexible peptide and a rigid peptide, where the flexible peptide was G₂(GGGGS)₃ and the rigid peptide was SSSSKAPPPS. The linker peptide L1 or L3 inside each scFv consisted of (GGGGS)₃.

Amino acid sequences of the VH domain and its complementarity determining regions (HCDR1, HCDR2 and HCDR3) and amino acid sequences of the VL domain and its complementarity determining regions (LCDR1, LCDR2 and LCDR3) of the anti-CD19 scFv in each of seven bispecific antibodies are listed in Table 1-1, where amino acid residues contained in the CDRs are defined according to a Kabat rule. The amino acid composition of the linker peptide between VH and VL of the anti-CD19 scFv is (GGGGS)n, wherein n = 1, 2, 3, 4 or 5.

**Table 1-1 Amino acid sequences of the anti-CD19 scFv contained in the bispecific antibody and amino acid sequences of its CDRs**

| | | |
|---|---|---|
| CD19 | | |
| SEQ ID NO: 1 | HCDR1 | SYWMN |
| SEQ ID NO: 2 | HCDR2 | QIWPGDGDTNYNGKFKG |
| SEQ ID NO: 3 | HCDR3 | RETTTVGRYYYAMDY |
| SEQ ID NO: 4 | LCDR1 | KASQSVDYDGDSYLN |
| SEQ ID NO: 5 | LCDR2 | DASNLVS |
| SEQ ID NO: 6 | LCDR3 | QQSTEDPWT |
| SEQ ID NO: 7 | VH | QVQLQQSGAELVRPGSSVKISCKASGYAFSS YWMNWVKQRPGQGLEWIGQIWPGDGDTNY NGKFKGKATLTADESSSTAYMQLSSLASEDS AVYFCARRETTTVGRYYYAMDYWGQGTTVT VSS |
| SEQ ID NO: 8 | VL | DIQLTQSPASLAVSLGQRATISCKASQSVDYD GDSYLNWYQQIPGQPPKLLIYDASNLVSGIPP RFSGSGSGTDFTLNIHPVEKVDAATYHCQQS TEDPWTFGGGTKLEIK |
| CD19 | | |
| SEQ ID NO: 9 | HCDR1 | SNWMH |
| SEQ ID NO: 10 | HCDR2 | EIDPSDSYTNYNQNFQG |
| SEQ ID NO: 11 | HCDR3 | GSNPYYYAMDY |
| SEQ ID NO: 12 | LCDR1 | SASSGVNYMH |
| SEQ ID NO: 13 | LCDR2 | DTSKLAS |
| SEQ ID NO: 14 | LCDR3 | HQRGSYT |
| SEQ ID NO: 15 | VH | QVQLVQPGAEVVKPGASVKLSCKTSGYTFTS NWMHWVKQAPGQGLEWIGEIDPSDSYTNYN QNFQGKAKLTVDKSTSTAYMEVSSLRSDDTA VYYCARGSNPYYYAMDYWGQGTSVTVSS |
| SEQ ID NO: 16 | VL | EIVLTQSPAIMSASPGERVTMTCSASSGVNYM HWYQQKPGTSPRRWIYDTSKLASGVPARFS GSGSGTDYSLTISSMEPEDAATYYCHQRGSY TFGGGTKLEIK |
| CD19 | | |
| SEQ ID NO: 17 | HCDR1 | TSGMGVG |
| SEQ ID NO: 18 | HCDR2 | HIWWDDDKRYNPALKS |
| SEQ ID NO: 19 | HCDR3 | MELWSYYFDY |
| SEQ ID NO: 20 | LCDR1 | SASSSVSYMH |
| SEQ ID NO: 13 | LCDR2 | DTSKLAS |
| SEQ ID NO: 21 | LCDR3 | FQGSVYPFT |
| SEQ ID NO: 22 | VH | QVQLQESGPGLVKPSQTLSLTCTVSGGSISTS GMGVGWIRQHPGKGLEWIGHIWWDDDKRYN PALKSRVTISVDTSKNQFSLKLSSVTAADTAVY YCARMELWSYYFDYWGQGTLVTVSS |
| SEQ ID NO: 23 | VL | EIVLTQSPATLSLSPGERATLSCSASSSVSYM HWYQQKPGQAPRLLIYDTSKLASGIPARFSGS GSGTDFTLTISSLEPEDVAVYYCFQGSVYPFT FGQGTKLEIK |
| CD19 | | |
| SEQ ID NO: 24 | HCDR1 | SSWMN |
| SEQ ID NO: 25 | HCDR2 | RIYPGDGDTNYNVKFKG |
| SEQ ID NO: 26 | HCDR3 | SGFITTVRDFDY |
| SEQ ID NO: 27 | LCDR1 | RASESVDTFGISFMN |
| SEQ ID NO: 28 | LCDR2 | EASNQGS |
| SEQ ID NO: 29 | LCDR3 | QQSKEVPFT |
| SEQ ID NO: 30 | VH | EVQLVESGGGLVQPGGSLRLSCAASGFTFSS SWMNWVRQAPGKGLEWVGRIYPGDGDTNY NVKFKGRFTISRDDSKNSLYLQMNSLKTEDTA VYYCARSGFITTVRDFDYWGQGTLVTVSS |
| SEQ ID NO: 31 | VL | EIVLTQSPDFQSVTPKEKVTITCRASESVDTFG ISFMNWFQQKPDQSPKLLIHEASNQGSGVPS RFSGSGSGTDFTLTINSLEAEDAATYYCQQSK EVPFTFGGGTKVEIK |
| CD19 | | |
| SEQ ID NO: 17 | HCDR1 | TSGMGVG |
| SEQ ID NO: 18 | HCDR2 | HIWWDDDKRYNPALKS |
| SEQ ID NO: 19 | HCDR3 | MELWSYYFDY |
| SEQ ID NO: 32 | LCDR1 | RASQSVSYMH |
| SEQ ID NO: 33 | LCDR2 | DASNRAS |
| SEQ ID NO: 21 | LCDR3 | FQGSVYPFT |
| SEQ ID NO: 34 | VH | QVTLRESGPALVKPTQTLTLTCTFSGFSLSTS GMGVGWIRQPPGKALEWLAHIWWDDDKRYN PALKSRLTISKDTSKNQVFLTMTNMDPVDTAT YYCARMELWSYYFDYWGQGTTVTVSS |
| SEQ ID NO: 35 | VL | ENVLTQSPATLSVTPGEKATITCRASQSVSYM HWYQQKPGQAPRLLIYDASNRASGVPSRFSG SGSGTDHTLTISSLEAEDAATYYCFQGSVYPF TFGQGTKLEIK |
| CD19 | | |
| SEQ ID NO: 17 | HCDR1 | TSGMGVG |
| SEQ ID NO: 18 | HCDR2 | HIWWDDDKRYNPALKS |
| SEQ ID NO: 19 | HCDR3 | MELWSYYFDY |
| SEQ ID NO: 32 | LCDR1 | RASQSVSYMH |
| SEQ ID NO: 33 | LCDR2 | DASNRAS |
| SEQ ID NO: 21 | LCDR3 | FQGSVYPFT |
| SEQ ID NO: 36 | VH | QVQLQESGPGLVKPTQTLSLTCTVSGGSISTS GMGVGWIRQHPGKGLEWIGHIWWDDDKRYN PALKSRVTISVDTSKNQVFLKLSSVTAADTAVY YCARMELWSYYFDYWGQGTTVTVSS |
| SEQ ID NO: 37 | VL | EIVLTQSPATLSLSPGERATITCRASQSVSYM HWYQQKPGQAPRLLIYDASNRASGVPSRFSG SGSGTDFTLTISSLEPEDAATYYCFQGSVYPF TFGQGTKLEIK |
| CD19 | | |
| SEQ ID NO: 17 | HCDR1 | TSGMGVG |
| SEQ ID NO: 18 | HCDR2 | HIWWDDDKRYNPALKS |
| SEQ ID NO: 19 | HCDR3 | MELWSYYFDY |
| SEQ ID NO: 20 | LCDR1 | SASSSVSYMH |
| SEQ ID NO: 13 | LCDR2 | DTSKLAS |
| SEQ ID NO: 21 | LCDR3 | FQGSVYPFT |
| SEQ ID NO: 38 | VH | QVQLQESGPALVKPTQTLTLTCTFSGFSLSTS GMGVGWIRQPPGKALEWLAHIWWDDDKRYN PALKSRLTISKDTSKNQVFLTMTNMDPVDTAT YYCARMELWSYYFDYWGQGTLVTVSS |
| SEQ ID NO: 39 | VL | ENVLTQSPATLSVTPGEKATLSCSASSSVSYM HWYQQKPGQAPRLLIYDTSKLASGIPARFSGS GSGTDHTLTISSLEAEDVAVYYCFQGSVYPFT FGQGTKLEIK |

The anti-CD3 scFv binds to an effector cell at an EC₅₀ value greater than about 10 nM, or greater than 20 nM, or greater than 40 nM, or greater than about 50 nM in an *in vitro* FACS binding assay; more preferably, the second single-chain Fv of the bispecific antibody is capable of binding to human CD3 and specifically binding to CD3 of a cynomolgus monkey or a rhesus monkey.

In a preferred example, amino acid sequences of VH and VL of the anti-CD3 scFv contained in the bispecific antibody are as shown in SEQ ID NO: 46 and SEQ ID NO: 47, respectively, and VH and VL are linked by (GGGGS)₃. The monoclonal antibody (named CD3-3) specifically binds to CD3 of the human and the cynomolgus monkey and has weak binding affinity to CD3.

The linker peptide linking the anti-CD19 scFv to the anti-CD3 scFv consists of a flexible peptide and a rigid peptide; preferably, an amino acid composition structure of the flexible peptide has a general formula of GₓS_{y}(GGGGS)_{z}, where x, y and z are integers greater than or equal to 0 and x + y + z ≥ 1. The rigid peptide is derived from a full-length sequence (as shown in SEQ ID NO: 48) consisting of amino acids 118 to 145 at the carboxyl terminus of natural human chorionic gonadotropin β-subunit or a truncated fragment thereof; preferably, the composition of the CTP rigid peptide is SSSSKAPPPS (CTP¹). Some preferred amino acid sequences of the linker peptide linking the anti-CD19 scFv to the anti-CD3 scFv are listed in Table 1-2.

**Table 1-2 Amino acid sequences of the linker peptide linking the anti-TAA scFv to the anti-CD3 scFv**

| | | |
|---|---|---|
| SEQ ID NO: 49 | G₂(GGGGS)₃CTP ₁ | GGGGGGSGGGGSGGGGSSSSSKAPPPS |
| SEQ ID NO: 50 | (GGGGS)₃CTP¹ | GGGGSGGGGSGGGGSSSSSKAPPPS |
| SEQ ID NO: 51 | GS(GGGGS)₂CTP¹ | GSGGGGSGGGGSSSSSKAPPPS |
| SEQ ID NO: 52 | (GGGGS)₁CTP⁴ | GGGGSSSSSKAPPPSLPSPSRLPGPSDTP ILPQ |

The Fc fragment is linked to the anti-CD3 scFv directly or by a linker peptide, and the linker peptide includes 1-20 amino acids, and preferably selected from the following amino acids: Gly(G), Ser(S), Ala(A) and Thr(T), more preferably selected from Gly(G) and Ser(S), most preferably, the linker peptide consists of (GGGGS)n, wherein n = 1, 2, 3 or 4.

The Fc fragment is preferably selected from heavy chain constant regions of human IgG1, IgG2, IgG3 and IgG4 and more particularly selected from heavy chain constant regions of human IgG1 or IgG4; and Fc is mutated to modify the properties of the bispecific antibody molecule, e.g., to show reduced affinity to at least one of human FcγRs (FcγRI, FcγRIIa or FcγRIIIa) and C1q, a reduced effector cell function, or a reduced complement function. In addition, the Fc fragment may also contain amino acid substitutions that change one or more other characteristics (such as an ability of binding to an FcRn receptor, the glycosylation of the antibody or the charge heterogeneity of the antibody).

Some amino acid sequences of the Fc fragment with one or more amino acid mutations are listed in Table 1-3.

**Table 1-3 Amino acid sequences of the Fc fragment from human IgG**

| **Amino acid sequence (EU numbering) of constant region of IgG1 Fc (L234A, L235A) mutant** | |
|---|---|
| SEQ ID NO: 53 | DKTHTCPPCP APEAAGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPGK |

| **Amino acid sequence (EU numbering) of constant region of IgG1 (L234A, L235A, T250Q, N297A, P331S, M428L, K447⁻) mutant** | |
|---|---|
| SEQ ID NO: 54 | DKTHTCPPCP APEAAGGPSV FLFPPKPKDQ LMISRTPEVT CVVVDVSHED PEVKFNWYVD GVEVHNAKTK PREEQYASTY RVVSVLTVLH QDWLNGKEYK CKVSNKALPA SIEKTISKAK GQPREPQVYT LPPSRDELTK NQVSLTCLVK GFYPSDIAVE WESNGQPENN YKTTPPVLDS DGSFFLYSKL TVDKSRWQQG NVFSCSVLHE ALHNHYTQKS LSLSPGK |

### 1.2 Construction of expression vector of bispecific antibody molecule

A gene encoding the bispecific antibody was synthesized by a conventional molecular biology method, and cDNA encoding the obtained fusion gene was separately inserted into corresponding restriction sites of a eukaryotic expression plasmid pCMAB2M modified by PCDNA3.1. The plasmid contains an early promoter of a cytomegalovirus, which is an enhancer required for the high-level expression of foreign genes in mammalian cells. The plasmid pCMAB2M also contains a selective marker to have kanamycin resistance in bacteria and G418 resistance in mammalian cells. In addition, when host cells are deficient in the expression of DHFR genes, the pCMAB2M expression vector contains mouse dihydrofolate reductase (DHFR) genes so that target genes and the DHFR genes can be co-amplified in the presence of methotrexate (MTX) (see U.S. Pat. No. US4399216).

### 1.3 Expression of the bispecific antibody molecule

The constructed expression plasmid was transfected into a mammalian host cell line to express the bispecific antibody. To achieve stable and high-level expression, a preferred host cell line is DHFR deficient CHO-cells (see U.S. Pat. No. US4818679). In this example, a CHO-derived cell strain DXB11 was selected as the host cell. A preferred method of transfection is electroporation, and other methods including calcium phosphate co-precipitation and lipofection may also be used. During electroporation, 50 µg of DNA of the expression vector plasmid were added to 5×10⁷ cells in a cuvette with a Gene Pulser electroporator (Bio-Rad Laboratories, Hercules, CA) set at an electric field of 300 V and a capacitance of 1500 µFd. Two days after transfection, the medium was replaced with a growth medium containing 0.6 mg/mL G418. Transfectants were subcloned by limiting dilution and the secretion rate of each cell line was determined through an ELISA. The cell strain that expressed the bispecific antibody at a high level was screened.

To achieve the higher-level expression of fusion proteins, DHFR genes inhibited by MTX should be used for co-amplification. The transfected fusion protein genes were co-amplified with the DHFR genes in growth media containing MTX with increased concentrations. Subclones with the positive expression of DHFR were subjected to limiting dilution with gradually increased pressure to screen transfectants capable of growing in media with MTX of up to 6 µM. The secretion rates of the transfectants were determined and a cell line with high foreign protein expression was screened. A cell line with a secretion rate greater than about 5 (preferably about 15) µg/10⁶ (million) cells/24 h was adaptively suspended using a serum-free medium. Cell supernatants were then collected and the bispecific antibody was separated and purified.

The purification process, stability and *in vitro* and *in vivo* biological functions of several bispecific antibodies are evaluated below.

### 1.4 Purification process and stability test of the bispecific antibody

### (a) Purification of a double-chain tetravalent bispecific antibody

The bispecific antibody against CD19 was purified by three-step chromatography. The three-step chromatography was respectively affinity chromatography, hydroxyapatite chromatography and anion exchange chromatography. (The protein purifier used in this example was AKTA pure 25 M from GE in the U.S. Reagents used in this example were purchased from Sinopharm Chemical Reagent Co., Ltd and had purity at an analytical grade).

In a first step, affinity chromatography was performed. Sample capture and concentration and the removal of partial pollutants were performed using AT Protein A Diamond from Bestchrom or other commercially available affinity media (such as MabSelect Sure from GE). First, chromatography columns were equilibrated with 3-5 column volumes (CVs) of an equilibration buffer (20 mM PB, 150 mM NaCl, pH 7.4) at a linear flow rate of 100-200 cm/h. The clarified fermentation broth was loaded at a linear flow rate of 100-200 cm/h with a load not higher than 20 mg/mL. After loading, the chromatography columns were equilibrated with 3-5 column volumes (CVs) of an equilibration buffer (20 mM PB, 150 mM NaCl, pH 7.4) at a linear flow rate of 100-200 cm/h to remove unbound components. The chromatography columns were rinsed with 3-5 column volumes of decontamination buffer 1 (50 mM NaAc-HAc, 1 M NaCl, pH 5.0) at a linear flow rate of 100-200 cm/h to remove partial pollutants. The chromatography columns were equilibrated with 3-5 column volumes (CVs) of decontamination buffer 2 (50 mM NaAc-HAc, pH 5.0) at a linear flow rate of 100-200 cm/h. The target product was eluted using an elution buffer (50 mM NaAc-HAc, pH 3.5) at a linear flow rate not higher than 100 cm/h and target peaks were collected.

In a second step, hydroxyapatite chromatography was performed. Intermediate purification was performed using CHT Type II from BIO-RAD or other commercially available hydroxyapatite media to reduce the content of polymers. After the target proteins were polymerized, the polymers and monomers differed in property such as charge characteristics and calcium ion chelation. The polymers and the monomers were separated with differences between charge characteristics. First, chromatography columns were equilibrated with 3-5 column volumes (CVs) of an equilibration buffer (20 mM PB, pH 7.4) at a linear flow rate of 100-200 cm/h. The target proteins separated through the affinity chromatography in the first step were loaded after the pH was adjusted to 7.0, with a load controlled to be less than 5 mg/mL. After loading, the chromatography columns were rinsed with 3-5 column volumes (CVs) of an equilibration buffer (20 mM PB, pH 7.0) at a linear flow rate of 100-200 cm/h. Finally, the target proteins were eluted using 10 column volumes (CVs) of an elution buffer (20 mM PB, 1M NaCl, pH 7.0) at a linear flow rate not higher than 100 cm/h with a gradient of 0-25%. Eluted fractions were collected and sent for SEC-HPLC, respectively. Target components with the percentage of monomers being greater than 95% were combined for chromatography in the next step.

In a third step, anion exchange chromatography was performed. Fine purification was performed by using Q-HP from Bestchrom or other commercially available anion exchange chromatography media (such as Q HP from GE, Toyopearl GigaCap Q-650 from TOSOH, DEAE Beads 6FF from Smart-Lifesciences, Generik MC-Q from Sepax Technologies, Inc, Fractogel EMD TMAE from Merck, and Q Ceramic HyperD F from Pall) to further remove pollutants such as HCP and DNA. First, chromatography columns were rinsed with 3-5 column volumes (CVs) of an equilibration buffer (20 mM PB, 0.15 M NaCl, pH 7.0) at a linear flow rate of 100-200 cm/h. The target proteins separated through hydroxyapatite chromatography in the second step were loaded and through-flows were collected. After loading, the chromatography columns were rinsed with 3-5 column volumes (CVs) of an equilibration buffer (20 mM PB, 0.15 M NaCl, pH 7.0) at a linear flow rate of 100-200 cm/h. The through-flow components were collected and sent for the detection of protein content, SEC-HPLC and electrophoresis.

The SEC-HPLC purity results and SDS-PAGE electrophoresis results of the samples are shown in FIG. 1-1 and FIG. 1-2, respectively. The SEC-HPLC results showed that the purity of the main peak of the bispecific antibody was more than 95% after three-step chromatography. The band pattern in the SDS-PAGE electrophoresis was as expected, where a band was shown at 160 KDa in the non-reducing electrophoresis and a clear single-chain band (80 KDa) was obtained after reduction.

### (b) Stability test of the bispecific antibody against CD19

The stability of proteins of the bispecific antibody against CD19 in citrates (20 mM citrate, 8% sucrose, 0.02% Tween-80) at different pH (5.5 and 6.0) was investigated and the effect of shaking on the stability of the sample was explored. The proteins of the bispecific antibody against CD19 were stored for two weeks under the condition of acceleration at 25°C for the evaluation of protein stability.

The proteins of the bispecific antibody against CD19 were transferred to citrate buffers of pH 5.5 and 6.0, separately. Samples were taken at different time points for detection and analysis. Detection items included SEC-HPLC and CE-SDS.

SEC-HPLC and CE-SDS test results of two preparations stored for 0-2 weeks at 25 °C are shown in Table 1-4 and Table 1-5. After the samples were placed still for two weeks, the proportions of the polymers, main peaks, shoulder peaks and fragments in the SEC-HPLC test results had no significant changes and differed little at different pH. There was no significant difference in SEC-HPLC results over two weeks in the citrate buffers of pH 5.5 and 6.0.

**Table 1-4 SEC-HPLC results in acceleration tests at 25°C**

| | SEC-polymer% | | | | SEC-main peak% | | | | SEC-shoulder peak% | | | | SEC-fragment% | | | |
|---|---|---|---|---|---|---|---|---|---|---|---|---|---|---|---|---|
| | T0 | 1d | 1W | 2W | T0 | 1d | 1W | 2W | T0 | 1d | 1W | 2W | T0 | 1d | 1W | 2W |
| pH 5.5 | 0.2 | 0.2 | 0.1 | 0.1 | 98.2 | 98.2 | 98.2 | 97.2 | 1.6 | 1.6 | 1.6 | 2.6 | 0 | 0 | 0 | 0.1 |
| pH 6.0 | 0.2 | 0.2 | 0.1 | 0.2 | 98.2 | 98.2 | 98.2 | 97.2 | 1.6 | 1.6 | 1.7 | 2.6 | 0 | 0 | 0 | 0 |

**Table 1-5 CE-SDS results in acceleration tests at 25 °C**

| | T0 | | 25 °C, 1W | | 25°C, 2W | |
|---|---|---|---|---|---|---|
| | Reducin g | Non-Reducin g | Reducing | Non-Reducing | Reducing | Non-Reducing |
| pH 5.5 | 99.5 | 99 | 98.5 | 98.3 | 99.4 | 97.9 |
| pH 6.0 | 99.4 | 98.7 | 98.6 | 98.6 | 99.2 | 97.2 |

The proteins of the bispecific antibody against CD19 were placed in two stability incubators, where the rotational speed of one stability incubator was set to 300 rpm and the rotational speed of the other stability incubator was set to 0 rpm, with other conditions remaining the same. The SEC-HPLC test results after three days under the condition of 300 rpm are shown in Table 1-6. The proportions of the polymers, main peaks, shoulder peaks and fragments are basically the same between the control group and the sample group. Therefore, the rotational speed of 300 rpm has no significant effect on the stability of the proteins of the bispecific antibody against CD19.

**Table 1-6 SEC-HPLC results of a control test of three days at 300 rpm**

| | SEC-polymer% | | SEC-main peak% | | SEC-shoulder peak% | | SEC-fragment% | |
|---|---|---|---|---|---|---|---|---|
| | 3d control | 3d | 3d control | 3d | 3d control | 3d | 3d control | 3d |
| pH 5.5 | 0.1 | 0.2 | 98.2 | 98.2 | 1.7 | 1.7 | 0 | 0 |
| pH 6.0 | 0.2 | 0.2 | 98.2 | 98.1 | 1.7 | 1.7 | 0 | 0 |

### Example 2 Evaluation of in vitro biological functions of the anti-CD 19×CD3 bispecific antibody

### 2.1 Determination of the binding activity of the bispecific antibody to effector cells and target cells (FACS)

### (a) Detection of the binding activity of the bispecific antibody to CD19-positive tumor cells Raji through flow cytometry

CD19-positive tumor cells Raji cells (from a cell bank of the Chinese Academy of Sciences in Shanghai) were cultured and collected through centrifugation. The collected cells were resuspended with 1% PBSB with the cell density being adjusted to 2×10⁶ cells/mL and then placed in a 96-well plate, 100 µL (2×10⁵ cells) per well. They were blocked at 4 °C for 0.5 h. After blocked, the cells were centrifuged and the supernatant was discarded. Diluted bispecific antibodies with a series of concentrations were added and incubated at 4°C for 1 h. The supernatant was removed through centrifugation. The 96-well plate was washed with PBS solution containing 1% BSA (PBSB) three times. A diluted goat anti-human IgG antibody labeled with AF647 was added and incubated at 4°C for 1h in dark. The supernatant was removed through centrifugation. The 96-well plate was washed twice with 1% PBSB, each well was resuspended with 100 µL of 1% paraformaldehyde (PF), and the signal strength was detected through flow cytometry. With the mean fluorescence intensity as the Y-axis and the antibody concentration as the X-axis, the EC₅₀ value of the bispecific antibody binding to tumor cells Raji was calculated by software GraphPad.

The results showed that all the bispecific antibodies with different structures had good binding activity to tumor cells with the overexpression of CD19. FIGS. 2-1 to 2-3 show the binding curves of the bispecific antibodies with different structures to tumor cells Raji. As shown in Table 2-1, the EC₅₀ value of all four bispecific molecules bound to Raji cells was at a nM level.

**Table 2-1 Determination of the abilities of anti-CD19×CD3 bispecific antibodies to bind to tumor cell Raji**

| | AB23P7 | AB23P8 | AB23P9 | AB23P10 |
|---|---|---|---|---|
| EC₅₀ (nM) | 1.393 | 1.924 | 2.600 | 2.678 |

### (b) Detection of the binding activity of the bispecific antibody to human T cells through FACS

PBMCs were prepared from human fresh blood through density gradient centrifugation. The PBMCs were resuspended in 1640 medium containing 10% heat-inactivated FBS. 2 µg/mL of CD3 antibody was added for activation for 24 h, and 250 IU/mL of IL-2 was added for amplification and culture for seven days. The amplified T cells were prepared and were detected to be positive for CD3 expression on the cell surface through flow cytometry. The method for preparing and determining the sample to be tested was the same as that in Example 2.1(a). The cells resuspended with 1% PF were loaded and the mean fluorescence intensity was analyzed by software GraphPad so that the EC₅₀ value of each bispecific antibody to bind to human T cells was calculated.

The results showed that each bispecific antibody had good binding activity to CIK (FIGS. 2-4 and 2-5). As shown in Table 2-2, the EC₅₀ value of AB23P7 was about 16 nM, the EC₅₀ value of AB23P8 was comparable to that of AB23P7, and the EC₅₀ value of AB23P9 and AB23P10 was about 50 nM and 30 nM, respectively.

**Table 2-2 Determination of abilities of anti-CD19×CD3 bispecific antibodies to bind to effector cells CIK**

| | AB23P7 | AB23P8 | AB23P9 | AB23P10 |
|---|---|---|---|---|
| EC₅₀ (nM) | 15.69 | 16.69 | 49.52 | 32.41 |

### (c) Detection of the cross-reactivity of the bispecific antibody with CD3 on the membrane of CIK cells of a cynomolgus monkey through the FACS

PBMCs were prepared from the fresh blood of the cynomolgus monkey through density gradient centrifugation. The PBMCs were resuspended in 1640 medium containing 10% heat-inactivated FBS. 2 µg/mL of OKT3 was added for activation for 24 h, and 250 IU/mL of IL-2 was added for amplification and culture for seven days. Cynomolgus monkey CIK cells were obtained for use. Human CIK cells and cynomolgus monkey CIK cells were collected through centrifugation. The method for preparing and determining the sample to be tested was the same as that in Example 2.1(a). The cells resuspended with a solution of 1% paraformaldehyde were loaded and the mean fluorescence intensity was analyzed by software GraphPad so that the EC₅₀ value of the bispecific antibody to bind to human CIK cells and cynomolgus monkey CIK cells was calculated.

As shown in FIG. 2-6, the bispecific antibodies AB23P7 and AB23P10 had substantially the same ability to bind to cynomolgus monkeyT cells. The EC₅₀ value of the bispecific antibodies AB23P7 and AB23P10 was detected through flow cytometry to be about 5.5 nM. The abilities of the two bispecific antibodies to bind to cynomolgus monkey T cells were stronger than those thereof to bind to human T cells. AB23P8 and AB23P9 can specifically bind to cynomolgus monkey T cells as AB23P7 and AB23P10.

### 2.2 Detection of abilities of bispecific antibodies to bind to antigens

The binding of bispecific antibodies to soluble CD3 and CD19 was detected by double antigen sandwich ELISA.

CD19 proteins (ACRO Biosystems, Cat. No. CD9-H5251) were diluted with PBS to a concentration of 1 µg/ml and added to 96-well plates, 100 µl per well. The plates were coated at 4 °C overnight. The plates were then blocked with 1% skimmed milk powder for 1 hour at room temperature. Each bispecific antibody was diluted with a 5-fold gradient for a total of 10 concentration gradients. The 96-well plates were then washed with PBST, and then the diluted bispecific antibodies were added. Control wells without antibodies were set. Incubated for 2 hours at room temperature. Unbound bispecific antibodies were washed away with PBST. Biotinylated CD3E&CD3D (ACRO Biosystem, Cat. No. CDD-H82W1) were mixed at 50 ng/ml with streptavdin HRP (BD, Cat. No. 554066), added in 96-well plates, 100 µl per well, and incubated for 1 hour at room temperature. 96-well plates were washed with PBST, and TMB was added to the plates, 100 µl per wells. Color development was performed at room temperature for 15 minutes, and then 0.2 M H₂SO₄ was added to stop the color development reaction. The light absorbance values at A450-620 nm were measured by a microplate reader. Analysis was performed by software GraphPa, and the EC₅₀ values for the binding of bispecific antibodies to two antigens were calculated.

The results show that each bispecific antibody bound specifically to both CD3 and CD19 molecules and exhibited good dose-dependence as the concentration of the antibodies changed (FIG. 2-7). The abilities of several bispecific antibodies to bind to soluble CD3 and CD19 are shown in Table 2-3, with EC₅₀ values ranging from 0.19 nM to 0.47 nM, and there was little difference between binding activities at both ends.

**Table 2-3 Detection of abilities of Anti-CD19xCD3 bispecific antibodies to bind to CD3 and CD19 molecules**

| | AB23P7 | AB23P8 | AB23P9 | AB23P1 0 |
|---|---|---|---|---|
| EC₅₀ (nM) | 0.2185 | 0.1925 | 0.2211 | 0.4704 |

### 2.3 Evaluation of the ability of the bispecific antibody to activate T cells in a reportor cell strain

Jurkat T cells (BPS Bioscience, Art. 60621) containing NFAT RE reportor can overexpress luciferase in the presence of both the bispecific antibody and target cells (Raji cells). The degree of activation of Jurkat T cells was quantified by detecting the activity of luciferase. The bispecific antibody blinatumomab (Blincyto®, Amgen) was used as a positive control. With the concentration of the bispecific antibody as the X-axis and a fluorescein signal as the Y-axis, four parameter curves were fitted.

The test results in FIGS. 2-8 and 2-9 show that Jurkat T cells are hardly activated in the absence of target cells that overexpress CD19 and T cells can be activated only in the presence of both the bispecific antibody and the target cells. The ability of each antibody to activate Jurkat T cells is shown in Table 2-4. The ability of each bispecific antibody to activate Jurkat T cells is almost comparable.

**Table 2-4 Determination of the abilities of anti-CD19×CD3 bispecific antibodies to activate a reporter gene cell line Jurkat T cells**

| | AB23P7 | AB23P8 | AB23P9 | AB23P1 0 | Blincyto |
|---|---|---|---|---|---|
| EC₅₀ (nM) | 1.080 | 1.123 | 0.8527 | 0.7093 | 2.714 |

### 2.4 Abilities of bispecific antibodies to mediate T cells to kill tumor cells

Normally cultured tumor cell lines, including Raji-Luc, NALM6 and Reh cells (all purchased from the cell bank of Chinese Academy of Sciences, Shanghai) were used as target cells, and cell suspensions were collected and centrifuged, added to 96-well cell culture plates after the cell density was adjusted to 2 × 10⁵ cells/ml, 100 µl per well, and cultured overnight. The antibodies were diluted according to the test design, and added to the cells, 50 µl per well, while wells without the addition of antibodies were supplemented with the same volume of the medium. Effector cells (human PBMCs or expanded CIK cells) whose number was five times larger than the number of target cells, were then added, 100 µl per well. Control wells were set, and wells without the addition of effector cells were supplemented with the same volume of the medium. After 48 hours of culture, Raji-Luc cells were detected by Steady-Glo Luciferase Assay System (Promega) and other cells were detected by CytoTox96 Non-Radio Cytotoxicity Assay (Promega). The analysis was performed with the detection results as the Y-axis and the bispecific antibody concentration as the X-axis through software GraphPad to calculate and compare the ability of each bispecific antibody to mediate the killing on Raji-luc cells.

The EC50 values of each bispecific antibody to mediate effector cells to kill tumor cells are shown in Tables 2-5 to 2-7. The results show that each bispecific antibody exhibited a very significant killing effect on tumor cells with high expression of CD19 in a dose-dependent manner, wherein EC₅₀ of each bispecific antibody reached the pM level.

**Table 9-5 EC₅₀ values of bispecific antibodies to mediate CIK to kill tumor cells**

| EC₅₀ (pM) | AB23P7 | AB23P8 | AB23P9 | AB23P10 | Blincyto |
|---|---|---|---|---|---|
| Raji-LUC | 0.6988 | 0.5861 | 0.1480 | 0.1280 | 0.5952 |
| | 0.2024 | - | - | 0.4834 | 5.654 |

| | | | | | |
|---|---|---|---|---|---|
| Note: - means that no detection is performed. | | | | | |

**Table 9-6 EC₅₀ values of bispecific antibodies to mediate PBMCs to kill tumor cells**

| EC50 (pM) | AB23P7 | AB23P8 | AB23P9 | AB23P10 | Blincyto |
|---|---|---|---|---|---|
| Raji-LUC | 1.225 | 1.025 | 1.014 | 0.9462 | 5.452 |
| | 1.254 | - | - | 1.254 | 21.22 |
| | - | - | - | 4.176 | 22.58 |

| | | | | | |
|---|---|---|---|---|---|
| Note: - means that no detection is performed. | | | | | |

**Table 9-7 EC₅₀ values of bispecific antibodies to mediate CIK to kill different tumor cells**

| EC50 (pM) | AB23P7 | AB23P10 | Blincyto |
|---|---|---|---|
| NALM6 | - | 4.402 | 77.29 |
| Reh | 1.709 | 1.640 | 11.87 |

| | | | |
|---|---|---|---|
| Note: - means that no detection is performed. | | | |

### Example 3 Evaluation of in vivo biological functions of the anti-CD19xCD3 bispecific antibody

### 3.1 Pharmacodynamic study of AB23P9 and AB23P10 (anti-CD19xCD3) in models constructed by transplanting subcutaneously human Burkkit's lymphoma Raji cells in vivo

Mouse transplanted tumor model of CD19-positive human Burkkit's lymphoma Raji cells were selected for pharmacodynamic study of the anti-CD19xCD3 bispecific antibody in inhibiting tumor growth *in vivo.*

The peripheral blood of a normal human was taken. Human PBMCs were separated through density gradient centrifugation, then resuspended in RPMI-1640 culture medium added with 10% inactivated FBS, and added with OKT3 at a final concentration of 1 µg/mL and human IL-2 at 250 IU/mL. After three days of culture, the mixture was centrifuged at 300 g for 5 min, and the medium was changed. The cells were resuspended in RPMI-1640 added with 10% inactivated FBS for cell culture and added with human IL-2 at 250 IU/mL. After that, fresh medium was then added every 2 days and CIK cells were collected on the tenth day of culture. Female NPG mice at the age of seven to eight weeks (purchased from Beijing Vitalstar Biotechnology Co., Ltd.) were selected and Raji cells in the logarithmic growth stage were collected. 5×10⁶ Raji cells and 5×10⁶ CIK cells were mixed and inoculated subcutaneously on the right back of each NPG mouse. One hour later, the mice were randomly divided into 7 groups with 6 mice in each group according to their weight, and they were intraperitoneally administered with corresponding drugs. All administration groups were administered twice a week, and the dosages at which the bispecific antibodies AB23P9 and AB23P10 were administered were 1 mg/kg, 0.1 mg/kg and 0.01 mg/kg. The day of administration was recorded as Day 0. The maximum diameter (D) and the minimum diameter (d) of the tumor were measured weekly with an electronic vernier caliper. The volume of the tumor was calculated using the following formula: volume (mm³) = [D×d²]/2. The tumor growth inhibition rate was calculated for each administration group using the following formula: TGI (%) = (1 - the volume of the administration group/the volume of the control group) × 100%.

As shown in FIG. 3-1, on Day 26 of administration, the mean tumor volume of the PBS control group was 1679.90 ± 359.05 mm³; the mean tumor volumes of AB23P9 administration groups at the dosages of 1 mg/kg, 0.1 mg/kg and 0.01 mg/kg were 7.39 ± 7.39 mm³, 56.84 ± 36.69 mm³ and 124.78 ± 35.03 mm³, respectively, and TGIs thereof were 99.56%, 96.62% and 92.57%, respectively, which were significantly different from that of the control group (*P<0.01*); the mean tumor volumes of AB23P10 administration groups at the dosages of 1 mg/kg, 0.1 mg/kg and 0.01 mg/kg were 0.00 ± 0.00 mm³, 0.00 ± 0.00 mm³ and 196.79 ± 146.03 mm³, respectively, and TGIs thereof were 100%, 100% and 88.29%, respectively, which were significantly different from that of the control group (*P<0.01*).

These above results suggest that the bispecific antibodies AB23P9 and AB23P10 inhibit the growth of tumor cells by activating human immune cells in animals and that AB23P9 and AB23P10 have substantially the same antitumor effect at the same dosage.

### 3.2 Pharmacodynamic study of Blincyto, AB23P9 and AB23P10 (anti-CD19×CD3) in models constructed by transplanting subcutaneously human Burkkit's lymphoma Daudi cells in vivo

Mouse transplanted tumor model of CD19-positive human Burkkit's lymphoma Daudi cells were selected for pharmacodynamic study of the anti-CD19×CD3 bispecific antibody in inhibiting tumor growth *in vivo.*

Human PBMCs were separated by the method of Example 3.1 and similarly resuspended twice. A fresh medium was added every 2 days during the cells were resuspended and cultured for the second time, and CIK cells were collected on day 8. Female NPG mice at the age of seven to eight weeks were selected and Daudi cells (from a cell bank of the Chinese Academy of Sciences) in the logarithmic growth stage were collected. 5×10⁶ Daudi cells and 1×10⁶ CIK cells were mixed and inoculated subcutaneously on the right back of each NPG mouse. One hour later, the mice were randomly divided into 8 groups with 5 mice in each group according to their weight.

They were intraperitoneally administered with corresponding drugs. All administration groups were administered twice weekly. Blincyto was administered at a dosage of 1 mg/kg, and bispecific antibodies AB23P9 and AB23P10 were administered were at a dosages of 1 mg/kg, 0.1 mg/kg and 0.01 mg/kg. The day of administration was recorded as Day 0. The maximum diameter (D) and the minimum diameter (d) of the tumor were measured weekly with an electronic vernier caliper. The volume of the tumor was calculated using the following formula: volume (mm³) = [D×d²]/2. The tumor growth inhibition rate was calculated for each administration group using the following formula: TGI (%) = (1 - the volume of the administration group/the volume of the control group) × 100%.

As shown in FIG. 3-2, on Day 25 of administration, the mean tumor volume of the PBS control group was 1903.03 ± 727.61 mm³; the mean tumor volume of the Blincyto administration group at the dosage of 1 mg/kg was 0.00 ± 0.00 mm³ and the TGI thereof was 100%, which was significantly different from that of the control group (*P<0.05*); the mean tumor volumes of AB23P9 administration groups at the dosages of 1 mg/kg, 0.1 mg/kg and 0.01 mg/kg were 0.00 ± 0.00 mm³, 261.13 ± 178.17 mm³ and 520.87 ± 133.26 mm³, respectively, and TGIs thereof were 100%, 86.28% and 72.63%, respectively, where compared with the control group, the AB23P9 administration group at the dosage of 1 mg/kg had a significant difference (*P<0.05*) and the other two groups had antitumor effects with no significant differences; the mean tumor volumes of AB23P10 administration groups at the dosages of 1 mg/kg, 0.1 mg/kg and 0.01 mg/kg were 0.00 ± 0.00 mm³, 58.46 ± 36.67 mm³ and 599.28 ± 199.80 mm³, respectively, and TGIs thereof were 100%, 96.93% and 68.51%, respectively, where compared with the control group, the AB23P10 administration groups at the dosages of 1 mg/kg and 0.1 mg/kg had significantly differences (*P<0.05*) and the AB23P10 administration group at the dosage of 0.01 mg/kg had an antitumor effect with no significant difference.

These results suggest that the bispecific antibodies Blincyto, AB23P9 and AB23P10 inhibit the growth of tumor cells by activating human immune cells in animals. Blincyto, AB23P9 and AB23P10 have substantially the same antitumor effect at the same dosage of 1 mg/kg. There is no difference between AB23P9 and AB23P10 at the same dosage, and the antitumor effects of AB23P9 and AB23P10 exhibit certain dosage-dependence.

### Example 4 Evaluation of the safety of the anti-CD19xCD3 bispecific antibody

### 4.1 Evaluation of the toxicity of the bispecific antibody to normal cynomolgus monkeys

Adult cynomolgus monkeys (Guangzhou Xiangguan Biotechnology Co., Ltd.) at the age of 3-4 years and with the weight of 3-4 kg were divided into two groups, one male and one female in each group. The two groups were an AB23P7 administration group and an AB23P10 administration group, respectively. The samples were diluted with 0.9% saline and administered four times in total with a peristaltic pump at an intravenous drip of 20 mL/kg/h on Day 1 (D1), Day 4 (D4), Day 8 (D8) and Day 11 (D11). The drug dosages are listed in Table 4-1. The cynomolgus monkeys were weighted weekly. The concentrations and volumes of AB23P7 and AB23P10 administered are shown in Table 4-1.

**Table 4-1 Administration scheme for the evaluation of acute toxicity to cynomolgus monkeys**

| Administration | First | Second | Third | Fourth | Observation |
|---|---|---|---|---|---|
| Administration | D1 | D4 | D8 | D11 | D12-D18 |
| AB23P7 | 50 µg/Kg | 50 µg/Kg | 300 | 1000 | / |
| AB23P10 | 50 µg/Kg | 50 µg/Kg | 300 | 1000 | / |
| Concentration | 5 µg/mL | 5 µg/mL | 30 µg/mL | 100 µg/mL | / |
| Volume | 10 | 10 | 10 mL/Kg | 10 mL/Kg | / |

As shown in Table 4-2, fecal softening occurred with two female cynomolgus monkeys after administration, where the AB23P7 administration group had fecal softening at a higher frequency and watery feces, and the AB23P10 administration group returned to a normal state immediately after the first administration. The other side effects observed, such as anorexia and vomiting, disappeared the day after occurrence.

**Table 4-2 Daily observations of cynomolgus monkeys**

| Test Drug | Sex | Daily Clinical Observations | | |
|---|---|---|---|---|
| AB23P7 | ♂ | K2: D11 | | |
| | ♀ | F4: D3\4\6\11\12\13\15 | | F5: D14 |
| AB23P10 | ♂ | | | |
| | ♀ | F4: D1 | K2 & F11: D1 | |

| | | | | |
|---|---|---|---|---|
| Note: F4: fecal softening; F5: watery feces; K2: anorexia; F11: vomiting; blank: no side effects. | | | | |

The changes in body temperature of cynomolgus monkeys are shown in Table 4-3. The body temperature was monitored on the day of each administration. It can be seen that the body temperature in each group has no large changes and fluctuates within a normal physiological value range.

**Table 4-3 Body temparature monitoring of cynomolgus monkeys (°C)**

| Test Object | Sex | D-1 | D1 | | | | D4 | | D8 | | D11 | | D18 |
|---|---|---|---|---|---|---|---|---|---|---|---|---|---|
| | | | 2h | 6h | 24h | 48h | 0h | 24h | 0h | 24h | 0h | 24h | |
| AB23P7 | ♂ | 38.99 | 39.92 | 40.39 | 39.07 | 39.25 | 39.44 | 39.26 | 39.34 | 39.38 | 39.19 | 39.40 | 39.13 |
| | ♀ | 38.27 | 38.52 | 39.29 | 38.21 | 38.21 | 38.26 | 38.42 | 38.88 | 38.89 | 38.83 | 39.02 | 38.59 |
| AB23P10 | ♂ | 39.26 | 39.21 | 39.18 | 39.58 | 39.56 | 39.18 | 38.82 | 38.55 | 39.27 | 39.47 | 39.57 | 39.19 |
| | ♀ | 37.46 | 38.32 | 38.61 | 37.41 | 37.82 | 37.90 | 37.46 | 37.85 | 38.14 | 38.22 | 38.99 | 37.86 |

As shown in Table 4-4, the body weight of test animals had no significant changes as a whole. Compared with the body weight on the fourth day before administration (recorded as D-4), merely male cynomolgus monkeys in the AB23P7 administration group decreased significantly in body weight, but the body weight decreased by less than 10% and stopped decreasing after the drug administration was stopped.

**Table 4-4 Body weight monitoring of cynomolgus monkeys (kg)**

| Test Object | Sex | D | D1 | D4 | D8 | D11 | D15 | D18 |
|---|---|---|---|---|---|---|---|---|
| AB23P7 | ♂ | 3.75 | 3.50 | 3.57 | 3.64 | 3.44 | 3.48 | 3.46 |
| | ♀ | 2.80 | 2.73 | 2.78 | 2.79 | 2.78 | 2.79 | 2.79 |
| AB23P10 | ♂ | 2.51 | 2.57 | 2.52 | 2.58 | 2.59 | 2.57 | 2.61 |
| | ♀ | 2.42 | 2.36 | 2.47 | 2.41 | 2.39 | 2.43 | 2.40 |

After the cynomolgus monkeys were administered, blood was taken to collect serum and the content of cytokines in serum was determined. The results are shown in FIGS. 4-1A to 4-1F. After the first administration, IL-2, IL-6 and IFN-γ all increased and returned to normal within 24 h. After the subsequent administrations at increased dosages, cytokines remained at normal levels after the immune systems of the cynomolgus monkeys adaptted. This indicates that the tolerance of cynomolgus monkeys to the test drugs exhibits a process of adaptation from low to high. Different degrees of diarrhea observed in the test process are a common side effect in the treatment with immunological drugs and the cynomolgus monkeys return to normal after the drug administration is stopped. The cynomolgus monkeys had no serious side effects at the dosage of up to 1 mg/kg, which indicates that cynomolgus monkeys have good tolerance to two candidate drugs. The results of in vivo pharmacodynamic tests show that AB23P7 and AB23P10 at low dosages show good antitumor effects, which indicates that the candidate drugs have a relatively wide treatment window and high safety.

### 4.2 In vivo pharmacokinetic test of the bispecific antibody in cynomolgus monkeys

Cynomolgus monkeys (Guangzhou Xiangguan Biotechnology Co., Ltd.) with the weight of 3-4 kg were divided into two groups, one male and one female in each group. The first group was administered with AB23P7 and the second group was administered with AB23P10, at the dosage of 50 µg/kg. The blood sampling time points were 30 min, 1 h, 3 h, 6 h, 8 h, 24 h, 48 h and 72 h, respectively, 8 time points in total. Blood was taken and serum was collected and frozen at -80 °C. The concentration of the drug in serum was determined through the ELISA.

Plates were coated with human CD19 antigen (Acrobiosystems, CD9-H 5258) at a concentration of 0.5 µg/mL. AB23P7 and AB23P10 were formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled goat anti-human IgG antibody was used at a concentration of 1: 5000 for detection, and developed with TMB. The pharmacokinetic parameters were calculated using PKSolver software. Specific parameters are shown in Table 4-5.

The results showed that the half-life of AB23P7 in cynomolgus monkeys was 15.85±5.3 h, the half-life of AB23P10 in cynomolgus monkeys was 15.9±1.8 h, and the two bispecific antibodies exhibited a consistent half-life in cynomolgus monkeys.

**Table 4-5 Pharmacokinetic parameters of bispecific antibodies in cynomolgus monkeys**

| Abbreviation | Unit | AB23P7 | | AB23P10 | |
|---|---|---|---|---|---|
| | | No1 | No2 | No3 | No4 |
| AUC (0-∞) | µg/L*h | 11377.39 | 8004.526 | 19407.642 | 11957.544 |
| AUMC (0-∞) | | 158331.8 | 124677.2 | 405708.07 6 | 197818.22 2 |
| MRT (0-∞) | h | 13.916 | 15.576 | 20.905 | 16.543 |
| VRT (0-∞) | h^2 | 261.551 | 563.011 | 625.843 | 407.778 |
| t_{1/2}z | h | 12.117 | 19.643 | 17.17 | 14.628 |
| Tmax | h | 0.5 | 0.5 | 0.5 | 0.5 |
| CLz | L/h/kg | 0.004 | 0.006 | 0.003 | 0.004 |
| Vz | L/kg | 0.077 | 0.177 | 0.064 | 0.088 |
| Cmax | µg/L | 1030.217 | 876.42 | 1216.021 | 1012.578 |

All the publications mentioned in the present disclosure are incorporated herein by reference as if each publication is separately incorporated herein by reference. In addition, it should be understood that those skilled in the art, who have read the disclosure, can make various changes or modifications to the present disclosure, and these equivalent forms fall within the scope of the appended claims.

## Claims

1. A bispecific antibody, which is a tetravalent homodimer formed by two identical polypeptide chains that bind to each other by a covalent bond, wherein each of the polypeptide chains comprises a first single-chain Fv that specifically binds to tumor-associated antigen CD19, a second single-chain Fv that specifically binds to effector cell antigen CD3 and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions comprising CDC, ADCC and ADCP.

2. The bispecific antibody according to claim 1, wherein the first single-chain Fv comprises a VH domain and a VL domain that are linked by a linker peptide which has an amino acid sequence of (GGGGX)ₙ, wherein X comprises Ser or Ala, preferably Ser, and n is a natural number from 1 to 5, preferably 3.

3. The bispecific antibody according to claim 1, wherein the tumor-associated antigen is CD19 comprising, but not limited to, any variant, isotype, derivative and species homolog of CD19; preferably, CD19 is derived from a human, a cynomolgus monkey or a rhesus monkey.

4. The bispecific antibody according to claim 1, wherein the first single-chain Fv specifically binds to CD19 and contains CDR1, CDR2 and CDR3 selected from the group consisting of:
(i) HCDR1, HCDR2 and HCDR3, comprised in a VH domain, that are as shown in SEQ ID NOs: 1, 2 and 3, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 1, 2 and 3; and LCDR1, LCDR2 and LCDR3, comprised in a VL domain, that are as shown in SEQ ID NOs: 4, 5 and 6, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 4, 5 and 6;
(ii) HCDR1, HCDR2 and HCDR3, comprised in a VH domain, that are as shown in SEQ ID NOs: 9, 10 and 11, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 9, 10 and 11; and LCDR1, LCDR2 and LCDR3, comprised in a VL domain, that are as shown in SEQ ID NOs: 12, 13 and 14, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 12, 13 and 14;
(iii) HCDR1, HCDR2 and HCDR3, comprised in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, comprised in a VL domain, that are as shown in SEQ ID NOs: 20, 13 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 20, 13 and 21;
(iv) HCDR1, HCDR2 and HCDR3, comprised in a VH domain, that are as shown in SEQ ID NOs: 24, 25 and 26, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 24, 25 and 26; and LCDR1, LCDR2 and LCDR3, comprised in a VL domain, that are as shown in SEQ ID NOs: 27, 28 and 29, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 27, 28 and 29; and
(v) HCDR1, HCDR2 and HCDR3, comprised in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, comprised in a VL domain, that are as shown in SEQ ID NOs: 32, 33 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 32, 33 and 21.

5. The bispecific antibody according to claim 1 or 4, wherein the first single-chain Fv specifically binds to CD19 and is selected from the group consisting of:
(i) a VH domain comprising an amino acid sequence as shown in SEQ ID NO: 7 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 7; and a VL domain comprising an amino acid sequence as shown in SEQ ID NO: 8 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 8;
(ii) a VH domain comprising an amino acid sequence as shown in SEQ ID NO: 15 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 15; and a VL domain comprising an amino acid sequence as shown in SEQ ID NO: 16 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 16;
(iii) a VH domain comprising an amino acid sequence as shown in SEQ ID NO: 22 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 22; and a VL domain comprising an amino acid sequence as shown in SEQ ID NO: 23 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 23;
(iv) a VH domain comprising an amino acid sequence as shown in SEQ ID NO: 30 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 30; and a VL domain comprising an amino acid sequence as shown in SEQ ID NO: 31 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 31;
(v) a VH domain comprising an amino acid sequence as shown in SEQ ID NO: 34 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 34; and a VL domain comprising an amino acid sequence as shown in SEQ ID NO: 35 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 35;
(vi) a VH domain comprising an amino acid sequence as shown in SEQ ID NO: 36 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 36; and a VL domain comprising an amino acid sequence as shown in SEQ ID NO: 37 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 37; and
(vii) a VH domain comprising an amino acid sequence as shown in SEQ ID NO: 38 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 38; and a VL domain comprising an amino acid sequence as shown in SEQ ID NO: 39 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 39.

6. A monoclonal antibody or an antigen-binding fragment thereof that specifically binds to CD19, comprising CDR1, CDR2 and CDR3 contained in the first single-chain Fv of the bispecific antibody according to claim 4, which are selected from the group consisting of:
(i) HCDR1, HCDR2 and HCDR3, comprised in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, comprised in a VL domain, that are as shown in SEQ ID NOs: 32, 33 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 32, 33 and 21; and
(ii) HCDR1, HCDR2 and HCDR3, comprised in a VH domain, that are as shown in SEQ ID NOs: 17, 18 and 19, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 17, 18 and 19; and LCDR1, LCDR2 and LCDR3, comprised in a VL domain, that are as shown in SEQ ID NOs: 20, 13 and 21, respectively or have sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 20, 13 and 21;
preferably, the monoclonal antibody is a humanized antibody;
preferably, the antigen-binding fragment is selected from scFv, Fab, Fab', (Fab')2, an Fv fragment or Fv linked by a disulfide bond (dsFv).

7. The monoclonal antibody or the antigen-binding fragment thereof according to claim 6, comprising a VH domain and a VL domain selected from the group consisting of:
(i) a VH domain comprising an amino acid sequence as shown in SEQ ID NO: 36 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 36; and a VL domain comprising an amino acid sequence as shown in SEQ ID NO: 37 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 37; and
(ii) a VH domain comprising an amino acid sequence as shown in SEQ ID NO: 38 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 38; and a VL domain comprising an amino acid sequence as shown in SEQ ID NO: 39 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 39.

8. The bispecific antibody according to claim 1, wherein the second single-chain Fv comprises a VH domain and a VL domain that are linked by a linker peptide which has an amino acid sequence of (GGGGX)n, wherein X comprises Ser or Ala, preferably Ser, and n is a natural number from 1 to 5, preferably 3.

9. The bispecific antibody according to claim 1 or 8, wherein the second single-chain Fv binds to an effector cell at an EC₅₀ value greater than about 10 nM, or greater than 20 nM, or greater than 40 nM, or greater than about 50 nM in an *in vitro* binding affinity assay; more preferably, the second single-chain Fv of the bispecific antibody is capable of binding to human CD3 and specifically binding to CD3 of a cynomolgus monkey or a rhesus monkey.

10. The bispecific antibody according to claim 9, wherein the second single-chain Fv specifically binds to CD3; the VH domain of the second single-chain Fv contains HCDR1, HCDR2 and HCDR3 as shown in SEQ ID NOs: 40, 41 and 42, respectively or having sequences that are at least 80%, 85%, 90%, 92%, 95%, 97%, 98%, 99% or more similar to or have one or more amino acid substitutions than SEQ ID NOs: 40, 41 and 42; and the VL domain of the second single-chain Fv contains LCDR1, LCDR2 and LCDR3 as shown in SEQ ID NOs: 43, 44 and 45, respectively or having sequences that are at least 80%, 85%, 90%, 92%, 95%, 97%, 98%, 99% or more similar to or have one or more amino acid substitutions than SEQ ID NOs: 43, 44 and 45.

11. The bispecific antibody according to claim 10, wherein the second single-chain Fv specifically binds to CD3; the VH domain of the second single-chain Fv contains an amino acid sequence as shown in SEQ ID NO: 46 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 46; and the VL domain of the second single-chain Fv contains an amino acid sequence as shown in SEQ ID NO: 47 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 47.

12. The bispecific antibody according to claim 1, wherein the linker peptide that links the first single-chain Fv to the second single-chain Fv consists of a flexible peptide and a rigid peptide; wherein the flexible peptide comprises two or more amino acids, and preferably selected from the following amino acids: Gly(G), Ser(S), Ala(A) and Thr(T); more preferably, the flexible peptide comprises G and S residues; most preferably, an amino acid composition structure of the flexible peptide has a general formula of GxSy(GGGGS)z, wherein x, y and z are integers greater than or equal to 0 and x + y + z ≥ 1; the rigid peptide is derived from a full-length sequence consisting of amino acids 118 to 145 at carboxyl terminus of natural human chorionic gonadotropin β-subunit or a truncated fragment thereof; preferably, the rigid peptide comprises SSSSKAPPPS.

13. The bispecific antibody according to claim 12, wherein the linker peptide contains an amino acid sequence as shown in SEQ ID NO: 49.

14. The bispecific antibody according to claim 1, wherein the linker peptide that links the Fc fragment to the second single-chain Fv comprises 1-20 amino acids, and preferably selected from the following amino acids: Gly(G), Ser(S), Ala(A) and Thr(T); preferably Gly(G) and Ser(S); more preferably, the linker peptide consists of (GGGGS)n, wherein n = 1, 2, 3 or 4.

15. The bispecific antibody according to claim 1 or 14, wherein the Fc fragment comprises a hinge region, a CH2 domain and a CH3 domain from a human immunoglobulin heavy chain constant region; preferably, the Fc fragment is selected from heavy chain constant regions of human IgG1, IgG2, IgG3, IgG4, IgM, IgA1, IgA2, IgD and IgE; more preferably, the Fc fragment is selected from heavy chain constant regions of human IgG1, IgG2, IgG3 and IgG4; further preferably, the Fc fragment is selected from a heavy chain constant region of human IgG1 or IgG4; and the Fc fragment has one or more amino acid substitutions, deletions or additions than a natural sequence from which the Fc fragment is derived.

16. The bispecific antibody according to claim 15, wherein the Fc fragment comprises amino acid substitutions, deletions or additions with reduced or eliminated effector functions (ADCP, ADCC and CDC effects).

17. The bispecific antibody according to claim 16, wherein the Fc fragment comprises amino acid substitutions L234A/L235A/P331 S that are determined according to an EU numbering system.

18. The bispecific antibody according to claim 16 or 17, wherein the Fc fragment further comprises amino acid substitutions, deletions or additions with one or more of the following properties:
(i) enhanced binding affinity to a neonatal receptor (FcRn);
(ii) reduced or eliminated glycosylation; and
(iii) reduced or eliminated charge heterogeneity.

19. The bispecific antibody according to claim 18, wherein the Fc fragment further comprises one or more of amino acid substitutions, deletions or additions as follows:
(i) amino acid substitutions M428L, T250Q/M428L, M428L/N434S or M252Y/S254T/T256E determined according to the EU numbering system;
(ii) an amino acid substitution N297A determined according to the EU numbering system; and
(iii) an amino acid deletion K447 determined according to the EU numbering system.

20. The bispecific antibody according to claim 18, wherein the Fc fragment has an amino acid sequence as shown in SEQ ID NO: 54 that has six amino acid substitutions or replacements L234A/L235A/N297A/P331 S/T250Q/M428L determined according to the EU numbering system and a deleted or removed K447 determined according to the EU numbering system compared to the natural sequence from which the Fc fragment is derived.

21. The bispecific antibody according to claim 1, wherein the bispecific antibody binds to human CD19 and CD3 and has an amino acid sequence comprising:
group (a):
(i) a sequence as shown in SEQ ID NO: 55;
(ii) a sequence having one or more substitutions, deletions or additions (such as 1, 2, 3, 4 or 5 substitutions, deletions or additions) relative to the sequence as shown in SEQ ID NO: 55; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 55;
group (b):
(i) a sequence as shown in SEQ ID NO: 57;
(ii) a sequence having one or more substitutions, deletions or additions (such as 1, 2, 3, 4 or 5 substitutions, deletions or additions) relative to the sequence as shown in SEQ ID NO: 57; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 57;
group (c):
(i) a sequence as shown in SEQ ID NO: 59;
(ii) a sequence having one or more substitutions, deletions or additions (such as 1, 2, 3, 4 or 5 substitutions, deletions or additions) relative to the sequence as shown in SEQ ID NO: 59; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 59;
group (d):
(i) a sequence as shown in SEQ ID NO: 61;
(ii) a sequence having one or more substitutions, deletions or additions (such as 1, 2, 3, 4 or 5 substitutions, deletions or additions) relative to the sequence as shown in SEQ ID NO: 61; or
(iii) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 61.

22. A DNA molecule encoding the bispecific antibody according to any one of claims 1 to 5 and 8 to 21.

23. The DNA molecule according to claim 22, which has a nucleotide sequence as shown in SEQ ID NO: 56, 58, 60 or 62.

24. A vector, comprising the DNA molecule according to claim 22 or 23.

25. A host cell, comprising the vector according to claim 24, wherein the host cell comprises a prokaryotic cell, a yeast or a mammalian cell, preferably a CHO cell.

26. A pharmaceutical composition, comprising the bispecific antibody according to any one of claims 1 to 5 and 8 to 21 or the DNA molecule according to claim 22 or the nucleotide sequence according to claim 23 and a pharmaceutically acceptable excipient, carrier or diluent;
preferably, the pharmaceutical composition further comprises an additional pharmaceutically active agent;
preferably, the additional pharmaceutically active agent is a drug for treating an immune-related disease;
preferably, the additional pharmaceutically active agent is a drug having antitumor activity;
preferably, the additional pharmaceutically active agent is a drug for treating an autoimmune disease or an inflammatory disease;
preferably, the additional pharmaceutically active agent is a drug for treating a disease or disorder related to transplant rejection;
preferably, the antibody and the additional pharmaceutically active agent are provided as separate components or as components of a same composition.

27. The pharmaceutical composition according to claim 26, further comprising a pharmaceutically active agent administered before, after or while the pharmaceutical composition is administered to a subject, wherein the pharmaceutically active agent is selected from an antibody, an antibody fragment, a drug, an enzyme, a cytotoxic agent, a toxin, an antibiotic, a hormone, an immunomodulator, a cytokine, a chemokine or a radioisotope.

28. The pharmaceutical composition according to claim 27, wherein the drug is selected from the group consisting of cyclophosphamide, nimustine, amethopterin, fluorouracil, capecitabine, gemcitabine, tigio, pemetrexed, fludarabine, doxorubicin, bleomycin, vinorelbine, paclitaxel, docetaxel, irinotecan, tamoxifen, letrozole, exemestane, fulvestrant, goserelin, medroxyprogesterone, cisplatin, carboplatin, oxaliplatin, nedaplatin, oxaliplatin, asparaginase, lobaplatin, etoposide, vincristine, irinotecan, tegafur, dacarbazine, mitomycin, teniposide, pirarubicin, mitoxantrone, vindesine, raltitrexed, methotrexate, cisplatin bleomycin sulfate, carmustine, chlorambucil, cyclophosphamide hydroxyurea and daunomycin.

29. The pharmaceutical composition according to claim 27, wherein the toxin is selected from the group consisting of ricin, abrin, α toxin, saponin, ribonuclease (RNase), DNase I, staphylococcal enterotoxin-A, pokeweed antiviral protein, gelonin, diphtheria toxin, Pseudomonas exotoxin and Pseudomonas endotoxin.

30. The pharmaceutical composition according to claim 27, wherein the immunomodulator is selected from the group consisting of a cytokine, a chemokine, a stem cell growth factor, a lymphotoxin, a colony-stimulating factor (CSF), an interleukin (IL), an erythropoietin, a platelet growth factor, a tumor necrosis factor (TNF), a granulocyte-colony stimulating factor (G-CSF), a granulocyte macrophage-colony stimulating factor (GM-CSF), interferon-a, interferon-β, interferon-γ or interferon-A, TGF-α, TGF-β, interleukin-1 (IL-1), IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-23, IL-25, LIF, FLT-3, a vascular endothelial growth factor, a thrombospondin and an endostatin.

31. The pharmaceutical composition according to any one of claims 26 to 30, wherein one or more additional therapies are applied, wherein the additional therapies are selected from the group consisting of a surgery, chemotherapy, radiotherapy, immunotherapy, gene therapy, DNA therapy, RNA therapy, nano therapy, viral therapy, adjuvant therapy and a combination thereof.

32. A method for enhancing or stimulating an immune response or function, comprising administering a therapeutically effective amount of the bispecific antibody according to any one of claims 1 to 5 and 8 to 21 or the DNA molecule according to claim 22 or the nucleotide sequence according to claim 23 or the pharmaceutical composition according to any one of claims 26 to 30 to an individual.

33. A method for treating, preventing or ameliorating an immune disorder or disease in a cell, tissue, organ or animal, comprising administering a therapeutically effective amount of the bispecific antibody according to any one of claims 1 to 5 and 8 to 21 or the DNA molecule according to claim 22 or the nucleotide sequence according to claim 23 or the pharmaceutical composition according to any one of claims 26 to 30 to an individual.

34. A method for preventing/treating, delaying development of, or reducing/inhibiting recurrence of a disease including an immune-related disease, a tumor, an autoimmune disease, an inflammatory disease or a transplant rejection-related disease or disorder, comprising administering an effective amount of the bispecific antibody according to any one of claims 1 to 5 and 8 to 21 or the DNA molecule according to claim 22 or the nucleotide sequence according to claim 23 or the pharmaceutical composition according to any one of claims 26 to 30 to an individual suffering from the disease or disorder.

35. The method according to any one of claims 32 to 34, wherein one or more additional therapies are applied, wherein the additional therapies are selected from the group consisting of a surgery, chemotherapy, radiotherapy, immunotherapy, gene therapy, DNA therapy, RNA therapy, nanotherapy, viral therapy, adjuvant therapy and a combination thereof.

36. A method for preparing the bispecific antibody according to any one of claims 1 to 5 and 8 to 21, comprising: (a) obtaining a fusion gene of the bispecific antibody, and constructing an expression vector of the bispecific antibody; (b) transfecting the expression vector into a host cell by a genetic engineering method; (c) culturing the host cell under conditions that allow the bispecific antibody to be produced; (d) separating and purifying the bispecific antibody;
wherein the expression vector in step (a) is one or more selected from a plasmid, a bacterium and a virus; preferably, the expression vector is a pCDNA3.4 vector;
wherein the host cell into which the constructed vector is transfected by the genetic engineering method in step (b) comprises a prokaryotic cell, a yeast cell or a mammalian cell, such as a CHO cell, an NS0 cell or another mammalian cell, preferably a CHO cell; and
wherein the bispecific antibody is separated and purified in step (d) by a conventional immunoglobulin purification method comprising protein A affinity chromatography and ion exchange, hydrophobic chromatography or molecular sieve.

37. Use of the bispecific antibody according to any one of claims 1 to 5 and 8 to 21 or the DNA molecule according to claim 22 or the nucleotide sequence according to claim 23 for preparing a drug for:
(1) treating an immune-related disease in a subject (such as a human);
(2) treating a tumor in a subject (such as a human);
(3) treating an autoimmune disease or an inflammatory disease in a subject (such as a human); or
(4) treating a transplant rejection-related disease or disorder in a subject (such as a human).

38. Use of the bispecific antibody according to any one of claims 1 to 5 and 8 to 21 or the DNA molecule according to claim 22 or the nucleotide sequence according to claim 23 for preparing a drug for treating, preventing or alleviating a tumor, wherein the tumor comprises acute myeloid leukemia (AML), chronic myeloid leukemia (CML), B acute lymphocytic leukemia (B-ALL), B chronic lymphocytic leukemia (B-CLL), B-cell lymphoma (BCL), T-cell lymphoma (TCL) (such as skin), myelodysplastic syndrome (MDS), small lymphocytic lymphoma (SLL), hairy cell leukemia (HCL), marginal zone lymphoma (MZL) (such as extranodal or splenic), follicular lymphoma (FL) (such as pediatric or gastrointestinal), B-cell prolymphocytic leukemia (B-PLL), mantle cell lymphoma (MCL), lymphoplasmacytic lymphoma (LPL)/Waldenstrom's macroglobulinemia (WM), lymphoblastic leukemia (ALL) (such as B cell), lymphoblastic lymphoma (LBL) (such as B cell), plasmablastic lymphoma (PBL) (such as B cell), Hodgkin's lymphoma, non-Hodgkin's lymphoma, diffuse large B-cell lymphoma (DLBCL) (for example, primary or inflammation-related), Burkitt's lymphoma (BL), multiple myeloma, anaplastic large-cell lymphoma and HIV-related lymphoma.

39. Use of the bispecific antibody according to any one of claims 1 to 5 and 8 to 21 or the DNA molecule according to claim 22 or the nucleotide sequence according to claim 23 for preparing a drug for treating, preventing or alleviating an autoimmune or inflammatory disease, wherein the autoimmune or inflammatory disease is selected from rheumatoid arthritis (RA), osteoarthritis, reactive arthritis, systemic lupus erythematosus (SLE), Crohn's disease, multiple sclerosis, scleroderma, psoriasis, psoriatic arthritis, ulcerative colitis (such as chronic), insulin-dependent diabetes (such as juvenile), thyroiditis (such as chronic), hyperthyroidism, asthma, allergic diseases, sarcoidosis, autoimmune hemolytic anemia, pernicious anemia, graft-versus-host disease, dermatomyositis, chronic hepatitis, microscopic renal vasculitis, chronic active hepatitis, uveitis, intestinal synovitis, autoimmune intestinal disease, idiopathic leukopenia, autoimmune glomerulonephritis, autoimmune hemolytic anemia, autoimmune hepatitis, interstitial pneumonia, chronic pemphigus, pemphigus vulgaris, arteritis, polyarteritis nodosa and ankylosing spondylitis.

40. Use of the bispecific antibody according to any one of claims 1 to 5 and 8 to 21 or the DNA molecule according to claim 22 or the nucleotide sequence according to claim 23 for preparing a drug for treating, preventing or alleviating a disease or disorder associated with transplant rejection, wherein the transplant rejection comprises acute, hyperacute or chronic transplant rejection and the transplant rejection comprises rejection to an organ, tissue or cell transplant which comprises, but is not limited to, blood transfusion, a vascular transplant, an epithelial transplant, an endothelial transplant, a muscle transplant, a connective tissue transplant, a joint transplant, a heart transplant, a lung transplant, a liver transplant, a kidney transplant, a pancreas transplant, a skin transplant, an intestinal transplant, a corneal transplant, a bone transplant, a bone marrow transplant, a graft-versus-host disease or a host-versus-graft disease.

41. A method for detecting the presence of cancer in a mammal, comprising:
(a) contacting a sample comprising one or more cells derived from the mammal with the bispecific antibody according to any one of claims 1 to 5 and 8 to 21 or the DNA molecule according to claim 22 or the nucleotide sequence according to claim 23 to form a complex; and
(b) detecting the complex, wherein the presence of the complex indicates the presence of cancer in the mammal.

42. A kit, comprising the bispecific antibody according to any one of claims 1 to 5 and 8 to 21 or the DNA molecule according to claim 22 or the nucleotide sequence according to claim 23.


SEQUENCE LISTING

<110>  AMPSOURCE BIOPHARMA SHANGHAI INC.

<120>  HOMODIMER-TYPE BISPECIFIC ANTIBODY TARGETING CD19 AND CD3, AND
       PREPARATION METHOD THEREFOR AND APPLICATION THEREOF

<130>  A4659EP

<140>  PCT/CN2019/114808
<141>  2019-10-31

<150>  CN201811294887.4
<151>  2018-11-01

<160>  63

<170>  PatentIn version 3.5

<210>  1
<211>  5
<212>  PRT
<213>  Artificial Sequence

<220>
<223>  anti-CD19 antibody 1_HCDR1

<400>  1

Ser Tyr Trp Met Asn
1               5


<210>  2
<211>  17
<212>  PRT
<213>  Artificial Sequence

<220>
<223>  anti-CD19 antibody 1_HCDR2

<400>  2

Gln Ile Trp Pro Gly Asp Gly Asp Thr Asn Tyr Asn Gly Lys Phe Lys
1               5                   10                  15


Gly



<210>  3
<211>  15
<212>  PRT
<213>  Artificial Sequence

<220>
<223>  anti-CD19 antibody 1_HCDR3

<400>  3

Arg Glu Thr Thr Thr Val Gly Arg Tyr Tyr Tyr Ala Met Asp Tyr
1               5                   10                  15

<210>  4
<211>  15
<212>  PRT
<213>  Artificial Sequence

<220>
<223>  anti-CD19 antibody 1_LCDR1

<400>  4

Lys Ala Ser Gln Ser Val Asp Tyr Asp Gly Asp Ser Tyr Leu Asn
1               5                   10                  15


<210>  5
<211>  7
<212>  PRT
<213>  Artificial Sequence

<220>
<223>  anti-CD19 antibody 1_LCDR2

<400>  5

Asp Ala Ser Asn Leu Val Ser
1               5


<210>  6
<211>  9
<212>  PRT
<213>  Artificial Sequence

<220>
<223>  anti-CD19 antibody 1_LCDR3

<400>  6

Gln Gln Ser Thr Glu Asp Pro Trp Thr
1               5


<210>  7
<211>  124
<212>  PRT
<213>  Artificial Sequence

<220>
<223>  anti-CD19 antibody 1_VH

<400>  7

Gln Val Gln Leu Gln Gln Ser Gly Ala Glu Leu Val Arg Pro Gly Ser
1               5                   10                  15


Ser Val Lys Ile Ser Cys Lys Ala Ser Gly Tyr Ala Phe Ser Ser Tyr
            20                  25                  30


Trp Met Asn Trp Val Lys Gln Arg Pro Gly Gln Gly Leu Glu Trp Ile

35                  40                  45

Gly Gln Ile Trp Pro Gly Asp Gly Asp Thr Asn Tyr Asn Gly Lys Phe
    50                  55                  60

Lys Gly Lys Ala Thr Leu Thr Ala Asp Glu Ser Ser Ser Thr Ala Tyr
65                  70                  75                  80

Met Gln Leu Ser Ser Leu Ala Ser Glu Asp Ser Ala Val Tyr Phe Cys
                85                  90                  95

Ala Arg Arg Glu Thr Thr Thr Val Gly Arg Tyr Tyr Tyr Ala Met Asp
            100                 105                 110

Tyr Trp Gly Gln Gly Thr Thr Val Thr Val Ser Ser
        115                 120

<210>  8
<211>  111
<212>  PRT
<213>  Artificial Sequence

<220>
<223>  anti-CD19 antibody 1_VL

<400>  8

Asp Ile Gln Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Leu Asn Trp Tyr Gln Gln Ile Pro Gly Gln Pro Pro
        35                  40                  45

Lys Leu Leu Ile Tyr Asp Ala Ser Asn Leu Val Ser Gly Ile Pro Pro
    50                  55                  60

Arg Phe Ser Gly Ser Gly Ser Gly Thr Asp Phe Thr Leu Asn Ile His
65                  70                  75                  80

Pro Val Glu Lys Val Asp Ala Ala Thr Tyr His Cys Gln Gln Ser Thr
                85                  90                  95

Glu Asp Pro Trp Thr Phe Gly Gly Gly Thr Lys Leu Glu Ile Lys
            100                 105                 110

<210>  9
<211>  5

<212>  PRT
<213>  Artificial Sequence

<220>
<223>  anti-CD19 antibody 2_HCDR1

<400>  9

Ser Asn Trp Met His
1               5


<210>  10
<211>  17
<212>  PRT
<213>  Artificial Sequence

<220>
<223>  anti-CD19 antibody 2_HCDR2

<400>  10

Glu Ile Asp Pro Ser Asp Ser Tyr Thr Asn Tyr Asn Gln Asn Phe Gln
1               5                   10                  15


Gly



<210>  11
<211>  11
<212>  PRT
<213>  Artificial Sequence

<220>
<223>  anti-CD19 antibody 2_HCDR3

<400>  11

Gly Ser Asn Pro Tyr Tyr Tyr Ala Met Asp Tyr
1               5                   10


<210>  12
<211>  10
<212>  PRT
<213>  Artificial Sequence

<220>
<223>  anti-CD19 antibody 2_LCDR1

<400>  12

Ser Ala Ser Ser Gly Val Asn Tyr Met His
1               5                   10


<210>  13
<211>  7
<212>  PRT
<213>  Artificial Sequence

<220>
<223>  anti-CD19 antibody 2, 3, 7_LCDR2

<400>  13

Asp Thr Ser Lys Leu Ala Ser
1               5


<210>  14
<211>  7
<212>  PRT
<213>  Artificial Sequence

<220>
<223>  anti-CD19 antibody 2_LCDR3

<400>  14

His Gln Arg Gly Ser Tyr Thr
1               5


<210>  15
<211>  120
<212>  PRT
<213>  Artificial Sequence

<220>
<223>  anti-CD19 antibody 2_VH

<400>  15

Gln Val Gln Leu Val Gln Pro Gly Ala Glu Val Val Lys Pro Gly Ala
1               5                   10                  15

Ser Val Lys Leu Ser Cys Lys Thr Ser Gly Tyr Thr Phe Thr Ser Asn
            20                  25                  30

Trp Met His Trp Val Lys Gln Ala Pro Gly Gln Gly Leu Glu Trp Ile
        35                  40                  45

Gly Glu Ile Asp Pro Ser Asp Ser Tyr Thr Asn Tyr Asn Gln Asn Phe
    50                  55                  60

Gln Gly Lys Ala Lys Leu Thr Val Asp Lys Ser Thr Ser Thr Ala Tyr
65                  70                  75                  80

Met Glu Val Ser Ser Leu Arg Ser Asp Asp Thr Ala Val Tyr Tyr Cys
                85                  90                  95

Ala Arg Gly Ser Asn Pro Tyr Tyr Tyr Ala Met Asp Tyr Trp Gly Gln
            100                 105                 110

Gly Thr Ser Val Thr Val Ser Ser
        115                 120

<210>  16
<211>  104
<212>  PRT
<213>  Artificial Sequence

<220>
<223>  anti-CD19 antibody 2_VL

<400>  16

Glu Ile Val Leu Thr Gln Ser Pro Ala Ile Met Ser Ala Ser Pro Gly
1               5                   10                  15

Glu Arg Val Thr Met Thr Cys Ser Ala Ser Ser Gly Val Asn Tyr Met
            20                  25                  30

His Trp Tyr Gln Gln Lys Pro Gly Thr Ser Pro Arg Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Ser Leu Thr Ile Ser Ser Met Glu Pro Glu
65                  70                  75                  80

Asp Ala Ala Thr Tyr Tyr Cys His Gln Arg Gly Ser Tyr Thr Phe Gly
                85                  90                  95

Gly Gly Thr Lys Leu Glu Ile Lys
            100


<210>  17
<211>  7
<212>  PRT
<213>  Artificial Sequence

<220>
<223>  anti-CD19 antibody 3, 5, 6, 7_HCDR1

<400>  17

Thr Ser Gly Met Gly Val Gly
1               5


<210>  18
<211>  16
<212>  PRT
<213>  Artificial Sequence

<220>
<223>  anti-CD19 antibody 3, 5, 6, 7_HCDR2

<400>  18

His Ile Trp Trp Asp Asp Asp Lys Arg Tyr Asn Pro Ala Leu Lys Ser
1               5                   10                  15

<210>  19
<211>  10
<212>  PRT
<213>  Artificial Sequence

<220>
<223>  anti-CD19 antibody 3, 5, 6, 7_HCDR3

<400>  19

Met Glu Leu Trp Ser Tyr Tyr Phe Asp Tyr
1               5                   10

<210>  20
<211>  10
<212>  PRT
<213>  Artificial Sequence

<220>
<223>  anti-CD19 antibody 3, 7_LCDR1

<400>  20

Ser Ala Ser Ser Ser Val Ser Tyr Met His
1               5                   10

<210>  21
<211>  9
<212>  PRT
<213>  Artificial Sequence

<220>
<223>  anti-CD19 antibody 3, 5, 6, 7_LCDR3

<400>  21

Phe Gln Gly Ser Val Tyr Pro Phe Thr
1               5

<210>  22
<211>  120
<212>  PRT
<213>  Artificial Sequence

<220>
<223>  anti-CD19 antibody 3_VH

<400>  22

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Thr Ser
            20                  25                  30

Gly Met Gly Val Gly Trp Ile Arg Gln His Pro Gly Lys Gly Leu Glu
        35                  40                  45

Trp Ile Gly His Ile Trp Trp Asp Asp Asp Lys Arg Tyr Asn Pro Ala
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Met Glu Leu Trp Ser Tyr Tyr Phe Asp Tyr Trp Gly Gln
            100                 105                 110

Gly Thr Leu Val Thr Val Ser Ser
        115                 120

<210>  23
<211>  106
<212>  PRT
<213>  Artificial Sequence

<220>
<223>  anti-CD19 antibody 3_VL

<400>  23

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Val Ala Val Tyr Tyr Cys Phe Gln Gly Ser Val Tyr Pro Phe Thr
                85                  90                  95

Phe Gly Gln Gly Thr Lys Leu Glu Ile Lys
            100                 105

<210>  24
<211>  5
<212>  PRT
<213>  Artificial Sequence

<220>
<223>  anti-CD19 antibody 4_HCDR1

<400>  24

Ser Ser Trp Met Asn
1               5


<210>  25
<211>  17
<212>  PRT
<213>  Artificial Sequence

<220>
<223>  anti-CD19 antibody 4_HCDR2

<400>  25

Arg Ile Tyr Pro Gly Asp Gly Asp Thr Asn Tyr Asn Val Lys Phe Lys
1               5                   10                  15


Gly



<210>  26
<211>  12
<212>  PRT
<213>  Artificial Sequence

<220>
<223>  anti-CD19 antibody 4_HCDR3

<400>  26

Ser Gly Phe Ile Thr Thr Val Arg Asp Phe Asp Tyr
1               5                   10


<210>  27
<211>  15
<212>  PRT
<213>  Artificial Sequence

<220>
<223>  anti-CD19 antibody 4_LCDR1

<400>  27

Arg Ala Ser Glu Ser Val Asp Thr Phe Gly Ile Ser Phe Met Asn
1               5                   10                  15


<210>  28
<211>  7

<212>  PRT
<213>  Artificial Sequence

<220>
<223>  anti-CD19 antibody 4_LCDR2

<400>  28

Glu Ala Ser Asn Gln Gly Ser
1               5


<210>  29
<211>  9
<212>  PRT
<213>  Artificial Sequence

<220>
<223>  anti-CD19 antibody 4_LCDR3

<400>  29

Gln Gln Ser Lys Glu Val Pro Phe Thr
1               5


<210>  30
<211>  121
<212>  PRT
<213>  Artificial Sequence

<220>
<223>  anti-CD19 antibody 4_VH

<400>  30

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ser
            20                  25                  30

Trp Met Asn Trp Val Arg Gln Ala Pro Gly Lys Gly Leu Glu Trp Val
        35                  40                  45

Gly Arg Ile Tyr Pro Gly Asp Gly Asp Thr Asn Tyr Asn Val Lys Phe
    50                  55                  60

Lys Gly Arg Phe Thr Ile Ser Arg Asp Asp Ser Lys Asn Ser Leu Tyr
65                  70                  75                  80

Leu Gln Met Asn Ser Leu Lys Thr Glu Asp Thr Ala Val Tyr Tyr Cys
                85                  90                  95

Ala Arg Ser Gly Phe Ile Thr Thr Val Arg Asp Phe Asp Tyr Trp Gly
            100                 105                 110

Gln Gly Thr Leu Val Thr Val Ser Ser
        115                 120

<210>  31
<211>  111
<212>  PRT
<213>  Artificial Sequence

<220>
<223>  anti-CD19 antibody 4_VL

<400>  31

Glu Ile Val Leu Thr Gln Ser Pro Asp Phe Gln Ser Val Thr Pro Lys
1               5                   10                  15

Glu Lys Val Thr Ile Thr Cys Arg Ala Ser Glu Ser Val Asp Thr Phe
            20                  25                  30

Gly Ile Ser Phe Met Asn Trp Phe Gln Gln Lys Pro Asp Gln Ser Pro
        35                  40                  45

Lys Leu Leu Ile His Glu Ala Ser Asn Gln Gly Ser Gly Val Pro Ser
    50                  55                  60

Arg Phe Ser Gly Ser Gly Ser Gly Thr Asp Phe Thr Leu Thr Ile Asn
65                  70                  75                  80

Ser Leu Glu Ala Glu Asp Ala Ala Thr Tyr Tyr Cys Gln Gln Ser Lys
                85                  90                  95

Glu Val Pro Phe Thr Phe Gly Gly Gly Thr Lys Val Glu Ile Lys
            100                 105                 110

<210>  32
<211>  10
<212>  PRT
<213>  Artificial Sequence

<220>
<223>  anti-CD19 antibody 5, 6_LCDR1

<400>  32

Arg Ala Ser Gln Ser Val Ser Tyr Met His
1               5                   10

<210>  33
<211>  7
<212>  PRT
<213>  Artificial Sequence

<220>

<223>  anti-CD19 antibody 5, 6_LCDR2

<400>  33

Asp Ala Ser Asn Arg Ala Ser
1               5


<210>  34
<211>  120
<212>  PRT
<213>  Artificial Sequence

<220>
<223>  anti-CD19 antibody 5_VH

<400>  34

Gln Val Thr Leu Arg Glu Ser Gly Pro Ala Leu Val Lys Pro Thr Gln
1               5                   10                  15


Thr Leu Thr Leu Thr Cys Thr Phe Ser Gly Phe Ser Leu Ser Thr Ser
            20                  25                  30


Gly Met Gly Val Gly Trp Ile Arg Gln Pro Pro Gly Lys Ala Leu Glu
        35                  40                  45


Trp Leu Ala His Ile Trp Trp Asp Asp Asp Lys Arg Tyr Asn Pro Ala
    50                  55                  60


Leu Lys Ser Arg Leu Thr Ile Ser Lys Asp Thr Ser Lys Asn Gln Val
65                  70                  75                  80


Phe Leu Thr Met Thr Asn Met Asp Pro Val Asp Thr Ala Thr Tyr Tyr
                85                  90                  95


Cys Ala Arg Met Glu Leu Trp Ser Tyr Tyr Phe Asp Tyr Trp Gly Gln
            100                 105                 110


Gly Thr Thr Val Thr Val Ser Ser
        115                 120


<210>  35
<211>  106
<212>  PRT
<213>  Artificial Sequence

<220>
<223>  anti-CD19 antibody 5_VL

<400>  35

Glu Asn Val Leu Thr Gln Ser Pro Ala Thr Leu Ser Val Thr Pro Gly
1               5                   10                  15

Glu Lys Ala Thr Ile Thr Cys Arg Ala Ser Gln Ser Val Ser Tyr Met
            20                  25                  30

His Trp Tyr Gln Gln Lys Pro Gly Gln Ala Pro Arg Leu Leu Ile Tyr
        35                  40                  45

Asp Ala Ser Asn Arg Ala Ser Gly Val Pro Ser Arg Phe Ser Gly Ser
    50                  55                  60

Gly Ser Gly Thr Asp His Thr Leu Thr Ile Ser Ser Leu Glu Ala Glu
65                  70                  75                  80

Asp Ala Ala Thr Tyr Tyr Cys Phe Gln Gly Ser Val Tyr Pro Phe Thr
                85                  90                  95

Phe Gly Gln Gly Thr Lys Leu Glu Ile Lys
            100                 105

<210>  36
<211>  120
<212>  PRT
<213>  Artificial Sequence

<220>
<223>  anti-CD19 antibody 6_VH

<400>  36

Gln Val Gln Leu Gln Glu Ser Gly Pro Gly Leu Val Lys Pro Thr Gln
1               5                   10                  15

Thr Leu Ser Leu Thr Cys Thr Val Ser Gly Gly Ser Ile Ser Thr Ser
            20                  25                  30

Gly Met Gly Val Gly Trp Ile Arg Gln His Pro Gly Lys Gly Leu Glu
        35                  40                  45

Trp Ile Gly His Ile Trp Trp Asp Asp Asp Lys Arg Tyr Asn Pro Ala
    50                  55                  60

Leu Lys Ser Arg Val Thr Ile Ser Val Asp Thr Ser Lys Asn Gln Val
65                  70                  75                  80

Phe Leu Lys Leu Ser Ser Val Thr Ala Ala Asp Thr Ala Val Tyr Tyr
                85                  90                  95

Cys Ala Arg Met Glu Leu Trp Ser Tyr Tyr Phe Asp Tyr Trp Gly Gln
            100                 105                 110

Gly Thr Thr Val Thr Val Ser Ser
        115                 120

<210>  37
<211>  106
<212>  PRT
<213>  Artificial Sequence

<220>
<223>  anti-CD19 antibody 6_VL

<400>  37

Glu Ile Val Leu Thr Gln Ser Pro Ala Thr Leu Ser Leu Ser Pro Gly
1               5                   10                  15

Glu Arg Ala Thr Ile Thr Cys Arg Ala Ser Gln Ser Val Ser Tyr Met
            20                  25                  30

His Trp Tyr Gln Gln Lys Pro Gly Gln Ala Pro Arg Leu Leu Ile Tyr
        35                  40                  45

Asp Ala Ser Asn Arg Ala Ser Gly Val Pro Ser Arg Phe Ser Gly Ser
    50                  55                  60

Gly Ser Gly Thr Asp Phe Thr Leu Thr Ile Ser Ser Leu Glu Pro Glu
65                  70                  75                  80

Asp Ala Ala Thr Tyr Tyr Cys Phe Gln Gly Ser Val Tyr Pro Phe Thr
                85                  90                  95

Phe Gly Gln Gly Thr Lys Leu Glu Ile Lys
            100                 105

<210>  38
<211>  120
<212>  PRT
<213>  Artificial Sequence

<220>
<223>  anti-CD19 antibody 7_VH

<400>  38

Gln Val Gln Leu Gln Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Gly Trp Ile Arg Gln Pro Pro Gly Lys Ala Leu Glu
        35                  40                  45

Trp Leu Ala His Ile Trp Trp Asp Asp Asp Lys Arg Tyr Asn Pro Ala
    50                  55                  60

Leu Lys Ser Arg Leu Thr Ile Ser Lys Asp Thr Ser Lys Asn Gln Val
65                  70                  75                  80

Phe Leu Thr Met Thr Asn Met Asp Pro Val Asp Thr Ala Thr Tyr Tyr
                85                  90                  95

Cys Ala Arg Met Glu Leu Trp Ser Tyr Tyr Phe Asp Tyr Trp Gly Gln
            100                 105                 110

Gly Thr Leu Val Thr Val Ser Ser
        115                 120

<210>  39
<211>  106
<212>  PRT
<213>  Artificial Sequence

<220>
<223>  anti-CD19 antibody 7_VL

<400>  39

Glu Asn Val Leu Thr Gln Ser Pro Ala Thr Leu Ser Val Thr Pro Gly
1               5                   10                  15

Glu Lys Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp His Thr Leu Thr Ile Ser Ser Leu Glu Ala Glu
65                  70                  75                  80

Asp Val Ala Val Tyr Tyr Cys Phe Gln Gly Ser Val Tyr Pro Phe Thr
                85                  90                  95

Phe Gly Gln Gly Thr Lys Leu Glu Ile Lys
            100                 105

<210>  40
<211>  5
<212>  PRT
<213>  Artificial Sequence

<220>
<223>  CD3-3_HCDR1

<400>  40

Thr Tyr Ala Met Asn
1               5


<210>  41
<211>  19
<212>  PRT
<213>  Artificial Sequence

<220>
<223>  CD3-3_HCDR2

<400>  41

Arg Ile Arg Ser Lys Tyr Asn Asn Tyr Ala Thr Tyr Tyr Ala Asp Ser
1               5                   10                  15


Val Lys Asp


<210>  42
<211>  14
<212>  PRT
<213>  Artificial Sequence

<220>
<223>  CD3-3_HCDR3

<400>  42

His Gly Asn Phe Gly Asn Ser Tyr Val Ser Trp Phe Ala Tyr
1               5                   10


<210>  43
<211>  14
<212>  PRT
<213>  Artificial Sequence

<220>
<223>  CD3-3_LCDR1

<400>  43

Arg Ser Ser Thr Gly Ala Val Thr Thr Ser Asn Tyr Ala Asn
1               5                   10


<210>  44
<211>  7
<212>  PRT
<213>  Artificial Sequence

<220>
<223>  CD3-3_LCDR2

<400>  44

Gly Thr Asn Lys Arg Ala Pro
1               5


<210>  45
<211>  9
<212>  PRT
<213>  Artificial Sequence

<220>
<223>  CD3-3_LCDR3

<400>  45

Ala Leu Trp Tyr Ser Asn Leu Trp Val
1               5


<210>  46
<211>  125
<212>  PRT
<213>  Artificial Sequence

<220>
<223>  CD3-3_VH

<400>  46

Glu Val Gln Leu Leu Glu Ser Gly Gly Gly Leu Val Gln Pro Gly Gly
1               5                   10                  15


Ser Leu Lys Leu Ser Cys Ala Ala Ser Gly Phe Thr Phe Asn Thr Tyr
            20                  25                  30


Ala Met Asn Trp Val Arg Gln Ala Pro Gly Lys Gly Leu Glu Trp Val
        35                  40                  45


Ala Arg Ile Arg Ser Lys Tyr Asn Asn Tyr Ala Thr Tyr Tyr Ala Asp
    50                  55                  60


Ser Val Lys Asp Arg Phe Thr Ile Ser Arg Asp Asp Ser Lys Asn Thr
65                  70                  75                  80


Ala Tyr Leu Gln Met Asn Asn Leu Lys Thr Glu Asp Thr Ala Val Tyr
                85                  90                  95


Tyr Cys Val Arg His Gly Asn Phe Gly Asn Ser Tyr Val Ser Trp Phe
            100                 105                 110


Ala Tyr Trp Gly Gln Gly Thr Leu Val Thr Val Ser Ser
        115                 120                 125

<210>  47
<211>  109
<212>  PRT
<213>  Artificial Sequence

<220>
<223>  CD3-3_VL

<400>  47

Glu Leu Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105


<210>  48
<211>  28
<212>  PRT
<213>  Homo sapiens

<400>  48

Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25


<210>  49
<211>  27
<212>  PRT
<213>  Artificial Sequence

<220>
<223>  G2(GGGGS)3CTP1

<400>  49

Gly Gly Gly Gly Gly Gly Ser Gly Gly Gly Gly Ser Gly Gly Gly Gly
1               5                   10                  15

Ser Ser Ser Ser Ser Lys Ala Pro Pro Pro Ser
            20                  25

<210>  50
<211>  25
<212>  PRT
<213>  Artificial Sequence

<220>
<223>  (GGGGS)3CTP1

<400>  50

Gly Gly Gly Gly Ser Gly Gly Gly Gly Ser Gly Gly Gly Gly Ser Ser
1               5                   10                  15

Ser Ser Ser Lys Ala Pro Pro Pro Ser
            20                  25

<210>  51
<211>  22
<212>  PRT
<213>  Artificial Sequence

<220>
<223>  GS(GGGGS)2CTP1

<400>  51

Gly Ser Gly Gly Gly Gly Ser Gly Gly Gly Gly Ser Ser Ser Ser Ser
1               5                   10                  15

Lys Ala Pro Pro Pro Ser
            20

<210>  52
<211>  33
<212>  PRT
<213>  Artificial Sequence

<220>
<223>  (GGGGS)1CTP4

<400>  52

Gly Gly Gly Gly Ser Ser Ser Ser Ser Lys Ala Pro Pro Pro Ser Leu
1               5                   10                  15

Pro Ser Pro Ser Arg Leu Pro Gly Pro Ser Asp Thr Pro Ile Leu Pro
            20                  25                  30

Gln

<210>  53
<211>  227
<212>  PRT
<213>  Artificial Sequence

<220>
<223>  IgG1 Fc(L234A,L235A) mutant constant region amino acid sequence

<400>  53

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  54
<211>  226
<212>  PRT
<213>  Artificial Sequence

<220>
<223>  IgG1(L234A,L235A, T250Q,N297A,P331S,M428L,K447-) mutant constant
       region amino acid sequence

<400>  54

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Gln Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Ala Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Ser Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
        180                 185                 190

Asp Lys Ser Arg Trp Gln Gln Gly Asn Val Phe Ser Cys Ser Val Leu
    195                 200                 205

His Glu Ala Leu His Asn His Tyr Thr Gln Lys Ser Leu Ser Leu Ser
    210                 215                 220

Pro Gly
225

<210>  55
<211>  743
<212>  PRT
<213>  Artificial Sequence

<220>
<223>  AB23P7 amino acid sequence

<400>  55

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Thr Ser
            20                  25                  30

Gly Met Gly Val Gly Trp Ile Arg Gln His Pro Gly Lys Gly Leu Glu
        35                  40                  45

Trp Ile Gly His Ile Trp Trp Asp Asp Asp Lys Arg Tyr Asn Pro Ala
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Met Glu Leu Trp Ser Tyr Tyr Phe Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Glu Ile Val Leu Thr Gln Ser Pro Ala
    130                 135                 140

Thr Leu Ser Leu Ser Pro Gly Glu Arg Ala Thr Leu Ser Cys Ser Ala
145                 150                 155                 160

Ser Ser Ser Val Ser Tyr Met His Trp Tyr Gln Gln Lys Pro Gly Gln
                165                 170                 175

Ala Pro Arg Leu Leu Ile Tyr Asp Thr Ser Lys Leu Ala Ser Gly Ile
            180                 185                 190

Pro Ala Arg Phe Ser Gly Ser Gly Ser Gly Thr Asp Phe Thr Leu Thr
        195                 200                 205

Ile Ser Ser Leu Glu Pro Glu Asp Val Ala Val Tyr Tyr Cys Phe Gln
    210                 215                 220

Gly Ser Val Tyr Pro Phe Thr Phe Gly Gln Gly Thr Lys Leu Glu Ile
225                 230                 235                 240

Lys Gly Gly Gly Gly Gly Gly Ser Gly Gly Gly Gly Ser Gly Gly Gly
                245                 250                 255

Gly Ser Ser Ser Ser Ser Lys Ala Pro Pro Pro Ser Glu Val Gln Leu
            260                 265                 270

Leu Glu Ser Gly Gly Gly Leu Val Gln Pro Gly Gly Ser Leu Lys Leu
        275                 280                 285

Ser Cys Ala Ala Ser Gly Phe Thr Phe Asn Thr Tyr Ala Met Asn Trp
    290                 295                 300

Val Arg Gln Ala Pro Gly Lys Gly Leu Glu Trp Val Ala Arg Ile Arg
305                 310                 315                 320

Ser Lys Tyr Asn Asn Tyr Ala Thr Tyr Tyr Ala Asp Ser Val Lys Asp
                325                 330                 335

Arg Phe Thr Ile Ser Arg Asp Asp Ser Lys Asn Thr Ala Tyr Leu Gln
            340                 345                 350

Met Asn Asn Leu Lys Thr Glu Asp Thr Ala Val Tyr Tyr Cys Val Arg
        355                 360                 365

His Gly Asn Phe Gly Asn Ser Tyr Val Ser Trp Phe Ala Tyr Trp Gly
    370                 375                 380

Gln Gly Thr Leu Val Thr Val Ser Ser Gly Gly Gly Gly Ser Gly Gly
385                 390                 395                 400

Gly Gly Ser Gly Gly Gly Gly Ser Glu Leu Val Val Thr Gln Glu Pro
                405                 410                 415

Ser Leu Thr Val Ser Pro Gly Gly Thr Val Thr Leu Thr Cys Arg Ser
            420                 425                 430

Ser Thr Gly Ala Val Thr Thr Ser Asn Tyr Ala Asn Trp Val Gln Gln
        435                 440                 445

Lys Pro Gly Gln Ala Pro Arg Gly Leu Ile Gly Gly Thr Asn Lys Arg
    450                 455                 460

Ala Pro Gly Thr Pro Ala Arg Phe Ser Gly Ser Leu Leu Gly Gly Lys
465                 470                 475                 480

Ala Ala Leu Thr Leu Ser Gly Val Gln Pro Glu Asp Glu Ala Glu Tyr
                485                 490                 495

Tyr Cys Ala Leu Trp Tyr Ser Asn Leu Trp Val Phe Gly Gly Gly Thr
            500                 505                 510

Lys Leu Thr Val Leu Asp Lys Thr His Thr Cys Pro Pro Cys Pro Ala
        515                 520                 525

Pro Glu Ala Ala Gly Gly Pro Ser Val Phe Leu Phe Pro Pro Lys Pro
    530                 535                 540

Lys Asp Gln Leu Met Ile Ser Arg Thr Pro Glu Val Thr Cys Val Val
545                 550                 555                 560

Val Asp Val Ser His Glu Asp Pro Glu Val Lys Phe Asn Trp Tyr Val
                565                 570                 575

Asp Gly Val Glu Val His Asn Ala Lys Thr Lys Pro Arg Glu Glu Gln
            580                 585                 590

Tyr Ala Ser Thr Tyr Arg Val Val Ser Val Leu Thr Val Leu His Gln
        595                 600                 605

Asp Trp Leu Asn Gly Lys Glu Tyr Lys Cys Lys Val Ser Asn Lys Ala
    610                 615                 620

Leu Pro Ala Ser Ile Glu Lys Thr Ile Ser Lys Ala Lys Gly Gln Pro
625                 630                 635                 640

Arg Glu Pro Gln Val Tyr Thr Leu Pro Pro Ser Arg Asp Glu Leu Thr

645                 650                 655

Lys Asn Gln Val Ser Leu Thr Cys Leu Val Lys Gly Phe Tyr Pro Ser
        660                 665                 670

Asp Ile Ala Val Glu Trp Glu Ser Asn Gly Gln Pro Glu Asn Asn Tyr
    675                 680                 685

Lys Thr Thr Pro Pro Val Leu Asp Ser Asp Gly Ser Phe Phe Leu Tyr
    690                 695                 700

Ser Lys Leu Thr Val Asp Lys Ser Arg Trp Gln Gln Gly Asn Val Phe
705                 710                 715                 720

Ser Cys Ser Val Leu His Glu Ala Leu His Asn His Tyr Thr Gln Lys
                725                 730                 735

Ser Leu Ser Leu Ser Pro Gly
        740

<210>  56
<211>  2307
<212>  DNA
<213>  Artificial Sequence

<220>
<223>  AB23P7 nucleotide sequence

<400>  56
actagtcgcc accatgagga gcctcggggc cctgctcttg ctgctgagcg cctgcctggc      60

ggtgagcgct caggtgcagc tgcaggagag cggaccagga ctggtgaagc catcccagac     120

cctgagcctg acctgtacag tgtctggcgg cagcatctct acatccggaa tgggagtggg     180

atggatcagg cagcacccag gcaagggcct ggaatggatc ggccatatct ggtgggacga     240

tgacaagaga tacaatcctg ccctgaagtc ccgcgtgaca atctctgtgg acacctccaa     300

gaaccagttt agcctgaagc tgtcttccgt gacagccgct gataccgccg tgtattactg     360

cgctagaatg gagctgtgga gctattactt cgactactgg ggccagggca cactggtgac     420

cgtgagctcc ggtggtggtg gttccggcgg cggcggttct ggcggcggcg gcagcgagat     480

cgtgctgaca cagtcccctg ccaccctgag cctgtctcca ggagagaggg ccaccctgtc     540

ctgttccgcc tccagctctg tgagctacat gcattggtat cagcagaagc caggccaggc     600

tcccaggctg ctgatctacg atacttctaa actggcctct ggcatccctg ctcggttttc     660

tggctccggc agcggcacag acttcaccct gacaatctcc agcctggagc cagaggatgt     720

ggccgtgtat tactgcttcc agggcagcgt gtatcccttc acatttggcc agggcactaa     780

actggagatc aagggtggcg gcggtggagg atccggcggt ggaggtagcg gcggaggcgg     840

tagctccagc tctagtaaag ctccccctcc ttccgaggtg cagctgctgg agtccggagg 900

aggactggtg cagccaggag gctccctgaa gctgagctgt gctgcctctg gctttacctt 960

caacacatat gccatgaatt gggtgcggca ggctccaggc aagggactgg agtgggtggc 1020

taggatcagg tctaagtaca acaattatgc cacctactat gctgattccg tgaaggacag 1080

gttcaccatc tcccgcgacg atagcaagaa cacagcctac ctgcagatga acaatctgaa 1140

gaccgaggat accgccgtgt actactgcgt gagacatggc aactttggca atagctacgt 1200

gtcctggttc gcttactggg gacagggcac cctggtcaca gtgagctctg gaggaggagg 1260

atctggagga ggaggctccg gaggaggagg aagcgagctg gtggtgaccc aggagccatc 1320

tctgacagtg tcccccggcg gcacagtgac cctgacatgt agatccagca ccggcgccgt 1380

gaccacatcc aactacgcta attgggtgca gcagaagcca ggacaggctc caaggggact 1440

gatcggagga accaacaaga gggctcctgg aacaccagct cggtttagcg gatctctgct 1500

gggaggcaag gctgccctga ccctgtccgg agtgcagcca gaggatgagg ccgagtatta 1560

ttgcgctctg tggtatagca atctgtgggt gttcggagga ggaaccaagc tgacagtgct 1620

ggacaagacc catacatgcc caccatgccc tgcccctgaa gccgccggag gaccttccgt 1680

gttcctgttc cctcccaagc caaaagatca gctgatgatc tctagaaccc ccgaagtcac 1740

ctgcgtggtc gtcgacgtgt cccatgagga ccctgaagtc aagttcaact ggtacgtgga 1800

cggtgtcgaa gtccacaacg ccaagaccaa gcctagggag gagcagtatg ccagcacata 1860

ccgggtggtg tctgtgctga ccgtgctgca tcaggattgg ctgaatggca aggaatataa 1920

atgtaaggtg agcaataagg ctctgccggc tagcattgaa aaaaccattt ccaaggctaa 1980

gggccagccc agggagcctc aggtctacac cctgcctcca tctagagatg aactgaccaa 2040

aaaccaggtg agcctgactt gcctggtcaa aggcttctac cccagcgaca ttgccgtgga 2100

gtgggagtct aatggccagc ctgaaaataa ctacaaaact acccctcctg tgctggactc 2160

tgatggctcc ttctttctgt actctaaact gaccgtggac aagtctcgct ggcagcaggg 2220

taacgtgttt tcttgctccg tgctgcacga ggctctgcat aaccattaca cccagaagag 2280

cctgtctctg tccccaggat agaattc 2307

<210> 57
<211> 743
<212> PRT
<213> Artificial Sequence

<220>
<223> AB23P8 amino acid sequence

<400> 57

Gln Val Thr Leu Arg Glu Ser Gly Pro Ala Leu Val Lys Pro Thr Gln

1               5                   10                  15

Thr Leu Thr Leu Thr Cys Thr Phe Ser Gly Phe Ser Leu Ser Thr Ser
            20                  25                  30

Gly Met Gly Val Gly Trp Ile Arg Gln Pro Pro Gly Lys Ala Leu Glu
        35                  40                  45

Trp Leu Ala His Ile Trp Trp Asp Asp Asp Lys Arg Tyr Asn Pro Ala
    50                  55                  60

Leu Lys Ser Arg Leu Thr Ile Ser Lys Asp Thr Ser Lys Asn Gln Val
65                  70                  75                  80

Phe Leu Thr Met Thr Asn Met Asp Pro Val Asp Thr Ala Thr Tyr Tyr
                85                  90                  95

Cys Ala Arg Met Glu Leu Trp Ser Tyr Tyr Phe Asp Tyr Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Glu Asn Val Leu Thr Gln Ser Pro Ala
    130                 135                 140

Thr Leu Ser Val Thr Pro Gly Glu Lys Ala Thr Ile Thr Cys Arg Ala
145                 150                 155                 160

Ser Gln Ser Val Ser Tyr Met His Trp Tyr Gln Gln Lys Pro Gly Gln
                165                 170                 175

Ala Pro Arg Leu Leu Ile Tyr Asp Ala Ser Asn Arg Ala Ser Gly Val
            180                 185                 190

Pro Ser Arg Phe Ser Gly Ser Gly Ser Gly Thr Asp His Thr Leu Thr
        195                 200                 205

Ile Ser Ser Leu Glu Ala Glu Asp Ala Ala Thr Tyr Tyr Cys Phe Gln
    210                 215                 220

Gly Ser Val Tyr Pro Phe Thr Phe Gly Gln Gly Thr Lys Leu Glu Ile
225                 230                 235                 240

Lys Gly Gly Gly Gly Gly Gly Ser Gly Gly Gly Gly Ser Gly Gly Gly
                245                 250                 255

Gly Ser Ser Ser Ser Ser Lys Ala Pro Pro Pro Ser Glu Val Gln Leu
                260                 265                 270

Leu Glu Ser Gly Gly Gly Leu Val Gln Pro Gly Gly Ser Leu Lys Leu
            275                 280                 285

Ser Cys Ala Ala Ser Gly Phe Thr Phe Asn Thr Tyr Ala Met Asn Trp
    290                 295                 300

Val Arg Gln Ala Pro Gly Lys Gly Leu Glu Trp Val Ala Arg Ile Arg
305                 310                 315                 320

Ser Lys Tyr Asn Asn Tyr Ala Thr Tyr Tyr Ala Asp Ser Val Lys Asp
                325                 330                 335

Arg Phe Thr Ile Ser Arg Asp Asp Ser Lys Asn Thr Ala Tyr Leu Gln
            340                 345                 350

Met Asn Asn Leu Lys Thr Glu Asp Thr Ala Val Tyr Tyr Cys Val Arg
        355                 360                 365

His Gly Asn Phe Gly Asn Ser Tyr Val Ser Trp Phe Ala Tyr Trp Gly
    370                 375                 380

Gln Gly Thr Leu Val Thr Val Ser Ser Gly Gly Gly Gly Ser Gly Gly
385                 390                 395                 400

Gly Gly Ser Gly Gly Gly Gly Ser Glu Leu Val Val Thr Gln Glu Pro
                405                 410                 415

Ser Leu Thr Val Ser Pro Gly Gly Thr Val Thr Leu Thr Cys Arg Ser
            420                 425                 430

Ser Thr Gly Ala Val Thr Thr Ser Asn Tyr Ala Asn Trp Val Gln Gln
        435                 440                 445

Lys Pro Gly Gln Ala Pro Arg Gly Leu Ile Gly Gly Thr Asn Lys Arg
    450                 455                 460

Ala Pro Gly Thr Pro Ala Arg Phe Ser Gly Ser Leu Leu Gly Gly Lys
465                 470                 475                 480

Ala Ala Leu Thr Leu Ser Gly Val Gln Pro Glu Asp Glu Ala Glu Tyr
                485                 490                 495

Tyr Cys Ala Leu Trp Tyr Ser Asn Leu Trp Val Phe Gly Gly Gly Thr
            500                 505                 510

Lys Leu Thr Val Leu Asp Lys Thr His Thr Cys Pro Pro Cys Pro Ala
        515                 520                 525

Pro Glu Ala Ala Gly Gly Pro Ser Val Phe Leu Phe Pro Pro Lys Pro
    530                 535                 540

Lys Asp Gln Leu Met Ile Ser Arg Thr Pro Glu Val Thr Cys Val Val
545                 550                 555                 560

Val Asp Val Ser His Glu Asp Pro Glu Val Lys Phe Asn Trp Tyr Val
                565                 570                 575

Asp Gly Val Glu Val His Asn Ala Lys Thr Lys Pro Arg Glu Glu Gln
            580                 585                 590

Tyr Ala Ser Thr Tyr Arg Val Val Ser Val Leu Thr Val Leu His Gln
        595                 600                 605

Asp Trp Leu Asn Gly Lys Glu Tyr Lys Cys Lys Val Ser Asn Lys Ala
    610                 615                 620

Leu Pro Ala Ser Ile Glu Lys Thr Ile Ser Lys Ala Lys Gly Gln Pro
625                 630                 635                 640

Arg Glu Pro Gln Val Tyr Thr Leu Pro Pro Ser Arg Asp Glu Leu Thr
                645                 650                 655

Lys Asn Gln Val Ser Leu Thr Cys Leu Val Lys Gly Phe Tyr Pro Ser
            660                 665                 670

Asp Ile Ala Val Glu Trp Glu Ser Asn Gly Gln Pro Glu Asn Asn Tyr
        675                 680                 685

Lys Thr Thr Pro Pro Val Leu Asp Ser Asp Gly Ser Phe Phe Leu Tyr
    690                 695                 700

Ser Lys Leu Thr Val Asp Lys Ser Arg Trp Gln Gln Gly Asn Val Phe
705                 710                 715                 720

Ser Cys Ser Val Leu His Glu Ala Leu His Asn His Tyr Thr Gln Lys
                725                 730                 735

Ser Leu Ser Leu Ser Pro Gly
            740

<210>  58
<211>  2307

<212>  DNA
<213>  Artificial Sequence

<220>
<223>  AB23P8 nucleotide sequence

<400>  58
actagtcgcc accatgagga gcctcggggc cctgctcttg ctgctgagcg cctgcctggc      60

ggtgagcgct caggtgaccc tgagggagtc cggacctgcc ctggtgaagc caacacagac     120

cctgacactg acctgtacct tctccggctt ctctctgtcc accagcggaa tgggagtggg     180

atggatcagg cagccacctg gcaaggccct ggagtggctg gctcacatct ggtgggacga     240

tgacaagaga tacaacccag ctctgaagtc tcgcctgacc atcagcaagg atacatctaa     300

gaaccaggtg tttctgacca tgacaaatat ggaccccgtg gacaccgcca catactattg     360

cgctcgcatg gagctgtggt cctactattt cgactactgg ggccagggta caaccgtcac     420

cgtgagctct ggtggtggtg gttccggcgg cggcggttct ggcggcggcg gcagcgagaa     480

cgtgctgaca cagtctccag ccaccctgtc cgtgacacct ggagagaagg ccaccatcac     540

atgtagagct tctcagtccg tgagctacat gcactggtat cagcagaagc caggacaggc     600

tccaaggctg ctgatctacg acgcctccaa tagggctagc ggcgtgcctt ctcggttttc     660

tggttctggc agcggcaccg atcataccct gacaatctcc agcctggagg ctgaagatgc     720

tgctacatac tattgtttcc agggcagcgt gtatcccttc accttcggcc agggtaccaa     780

gctggagatc aagggtggcg gcggtggagg atccggcggt ggaggtagcg gcggaggcgg     840

tagctccagc tctagtaaag ctccccctcc ttccgaggtg cagctgctgg agtccggagg     900

aggactggtg cagccaggag gctccctgaa gctgagctgt gctgcctctg gctttacctt     960

caacacatat gccatgaatt gggtgcggca ggctccaggc aagggactgg agtgggtggc    1020

taggatcagg tctaagtaca acaattatgc cacctactat gctgattccg tgaaggacag    1080

gttcaccatc tcccgcgacg atagcaagaa cacagcctac ctgcagatga acaatctgaa    1140

gaccgaggat accgccgtgt actactgcgt gagacatggc aactttggca atagctacgt    1200

gtcctggttc gcttactggg gacagggcac cctggtcaca gtgagctctg gaggaggagg    1260

atctggagga ggaggctccg gaggaggagg aagcgagctg gtggtgaccc aggagccatc    1320

tctgacagtg tcccccggcg gcacagtgac cctgacatgt agatccagca ccggcgccgt    1380

gaccacatcc aactacgcta attgggtgca gcagaagcca ggacaggctc caaggggact    1440

gatcggagga accaacaaga gggctcctgg aacaccagct cggtttagcg gatctctgct    1500

gggaggcaag gctgccctga ccctgtccgg agtgcagcca gaggatgagg ccgagtatta    1560

ttgcgctctg tggtatagca atctgtgggt gttcggagga ggaaccaagc tgacagtgct    1620

ggacaagacc catacatgcc caccatgccc tgcccctgaa gccgccggag gaccttccgt    1680

gttcctgttc cctcccaagc caaaagatca gctgatgatc tctagaaccc ccgaagtcac 1740

ctgcgtggtc gtcgacgtgt cccatgagga ccctgaagtc aagttcaact ggtacgtgga 1800

cggtgtcgaa gtccacaacg ccaagaccaa gcctagggag gagcagtatg ccagcacata 1860

ccgggtggtg tctgtgctga ccgtgctgca tcaggattgg ctgaatggca aggaatataa 1920

atgtaaggtg agcaataagg ctctgccggc tagcattgaa aaaaccattt ccaaggctaa 1980

gggccagccc agggagcctc aggtctacac cctgcctcca tctagagatg aactgaccaa 2040

aaaccaggtg agcctgactt gcctggtcaa aggcttctac cccagcgaca ttgccgtgga 2100

gtgggagtct aatggccagc ctgaaaataa ctacaaaact acccctcctg tgctggactc 2160

tgatggctcc ttctttctgt actctaaact gaccgtggac aagtctcgct ggcagcaggg 2220

taacgtgttt tcttgctccg tgctgcacga ggctctgcat aaccattaca cccagaagag 2280

cctgtctctg tccccaggat agaattc 2307

<210> 59
<211> 743
<212> PRT
<213> Artificial Sequence

<220>
<223> AB23P9 amino acid sequence

<400> 59

Gln Val Gln Leu Gln Glu Ser Gly Pro Gly Leu Val Lys Pro Thr Gln
1               5                   10                  15

Thr Leu Ser Leu Thr Cys Thr Val Ser Gly Gly Ser Ile Ser Thr Ser
            20                  25                  30

Gly Met Gly Val Gly Trp Ile Arg Gln His Pro Gly Lys Gly Leu Glu
        35                  40                  45

Trp Ile Gly His Ile Trp Trp Asp Asp Asp Lys Arg Tyr Asn Pro Ala
    50                  55                  60

Leu Lys Ser Arg Val Thr Ile Ser Val Asp Thr Ser Lys Asn Gln Val
65                  70                  75                  80

Phe Leu Lys Leu Ser Ser Val Thr Ala Ala Asp Thr Ala Val Tyr Tyr
                85                  90                  95

Cys Ala Arg Met Glu Leu Trp Ser Tyr Tyr Phe Asp Tyr Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Glu Ile Val Leu Thr Gln Ser Pro Ala
    130                 135                 140

Thr Leu Ser Leu Ser Pro Gly Glu Arg Ala Thr Ile Thr Cys Arg Ala
145                 150                 155                 160

Ser Gln Ser Val Ser Tyr Met His Trp Tyr Gln Gln Lys Pro Gly Gln
                165                 170                 175

Ala Pro Arg Leu Leu Ile Tyr Asp Ala Ser Asn Arg Ala Ser Gly Val
            180                 185                 190

Pro Ser Arg Phe Ser Gly Ser Gly Ser Gly Thr Asp Phe Thr Leu Thr
        195                 200                 205

Ile Ser Ser Leu Glu Pro Glu Asp Ala Ala Thr Tyr Tyr Cys Phe Gln
    210                 215                 220

Gly Ser Val Tyr Pro Phe Thr Phe Gly Gln Gly Thr Lys Leu Glu Ile
225                 230                 235                 240

Lys Gly Gly Gly Gly Gly Gly Ser Gly Gly Gly Gly Ser Gly Gly Gly
                245                 250                 255

Gly Ser Ser Ser Ser Ser Lys Ala Pro Pro Pro Ser Glu Val Gln Leu
            260                 265                 270

Leu Glu Ser Gly Gly Gly Leu Val Gln Pro Gly Gly Ser Leu Lys Leu
        275                 280                 285

Ser Cys Ala Ala Ser Gly Phe Thr Phe Asn Thr Tyr Ala Met Asn Trp
    290                 295                 300

Val Arg Gln Ala Pro Gly Lys Gly Leu Glu Trp Val Ala Arg Ile Arg
305                 310                 315                 320

Ser Lys Tyr Asn Asn Tyr Ala Thr Tyr Tyr Ala Asp Ser Val Lys Asp
                325                 330                 335

Arg Phe Thr Ile Ser Arg Asp Asp Ser Lys Asn Thr Ala Tyr Leu Gln
            340                 345                 350

Met Asn Asn Leu Lys Thr Glu Asp Thr Ala Val Tyr Tyr Cys Val Arg
        355                 360                 365

His Gly Asn Phe Gly Asn Ser Tyr Val Ser Trp Phe Ala Tyr Trp Gly

370                 375                 380

Gln Gly Thr Leu Val Thr Val Ser Ser Gly Gly Gly Gly Ser Gly Gly
385                 390                 395                 400

Gly Gly Ser Gly Gly Gly Gly Ser Glu Leu Val Val Thr Gln Glu Pro
                405                 410                 415

Ser Leu Thr Val Ser Pro Gly Gly Thr Val Thr Leu Thr Cys Arg Ser
            420                 425                 430

Ser Thr Gly Ala Val Thr Thr Ser Asn Tyr Ala Asn Trp Val Gln Gln
        435                 440                 445

Lys Pro Gly Gln Ala Pro Arg Gly Leu Ile Gly Gly Thr Asn Lys Arg
    450                 455                 460

Ala Pro Gly Thr Pro Ala Arg Phe Ser Gly Ser Leu Leu Gly Gly Lys
465                 470                 475                 480

Ala Ala Leu Thr Leu Ser Gly Val Gln Pro Glu Asp Glu Ala Glu Tyr
                485                 490                 495

Tyr Cys Ala Leu Trp Tyr Ser Asn Leu Trp Val Phe Gly Gly Gly Thr
            500                 505                 510

Lys Leu Thr Val Leu Asp Lys Thr His Thr Cys Pro Pro Cys Pro Ala
        515                 520                 525

Pro Glu Ala Ala Gly Gly Pro Ser Val Phe Leu Phe Pro Pro Lys Pro
    530                 535                 540

Lys Asp Gln Leu Met Ile Ser Arg Thr Pro Glu Val Thr Cys Val Val
545                 550                 555                 560

Val Asp Val Ser His Glu Asp Pro Glu Val Lys Phe Asn Trp Tyr Val
                565                 570                 575

Asp Gly Val Glu Val His Asn Ala Lys Thr Lys Pro Arg Glu Glu Gln
            580                 585                 590

Tyr Ala Ser Thr Tyr Arg Val Val Ser Val Leu Thr Val Leu His Gln
        595                 600                 605

Asp Trp Leu Asn Gly Lys Glu Tyr Lys Cys Lys Val Ser Asn Lys Ala
    610                 615                 620

Leu Pro Ala Ser Ile Glu Lys Thr Ile Ser Lys Ala Lys Gly Gln Pro
625                 630                 635                 640

Arg Glu Pro Gln Val Tyr Thr Leu Pro Pro Ser Arg Asp Glu Leu Thr
                645                 650                 655

Lys Asn Gln Val Ser Leu Thr Cys Leu Val Lys Gly Phe Tyr Pro Ser
            660                 665                 670

Asp Ile Ala Val Glu Trp Glu Ser Asn Gly Gln Pro Glu Asn Asn Tyr
        675                 680                 685

Lys Thr Thr Pro Pro Val Leu Asp Ser Asp Gly Ser Phe Phe Leu Tyr
    690                 695                 700

Ser Lys Leu Thr Val Asp Lys Ser Arg Trp Gln Gln Gly Asn Val Phe
705                 710                 715                 720

Ser Cys Ser Val Leu His Glu Ala Leu His Asn His Tyr Thr Gln Lys
                725                 730                 735

Ser Leu Ser Leu Ser Pro Gly
            740

<210>  60
<211>  2307
<212>  DNA
<213>  Artificial Sequence

<220>
<223>  AB23P9 nucleotide sequence

<400>  60
actagtcgcc accatgagga gcctcggggc cctgctcttg ctgctgagcg cctgcctggc      60

ggtgagcgct caggtgcagc tgcaggagag cggaccagga ctggtgaagc caacccagac     120

cctgagcctg acctgtacag tgtctggcgg cagcatctct acatccggaa tgggagtggg     180

atggatcagg cagcacccag gcaagggcct ggaatggatc ggccatatct ggtgggacga     240

tgacaagaga tacaatcctg ccctgaagtc ccgcgtgaca atctctgtgg acacctccaa     300

gaaccaggtc ttcctgaagc tgtcttccgt gacagccgct gataccgccg tgtattactg     360

cgctagaatg gagctgtgga gctattactt cgactactgg ggccagggca caaccgtgac     420

cgtgagctcc ggtggtggtg gttccggcgg cggcggttct ggcggcggcg gcagcgagat     480

cgtgctgaca cagtcccctg ccaccctgag cctgtctcca ggagagaggg ccaccatcac     540

ctgtagggcc tcccagtctg tgagctacat gcattggtat cagcagaagc caggccaggc     600

tcccaggctg ctgatctacg atgcttctaa tcgggcctct ggcgtgcctt cccggttttc     660

tggctccggc agcggcacag acttcaccct gacaatctcc agcctggagc cagaggatgc 720

ggccacctat tactgcttcc agggcagcgt gtatcccttc acatttggcc agggcactaa 780

actggagatc aagggtggcg gcggtggagg atccggcggt ggaggtagcg gcggaggcgg 840

tagctccagc tctagtaaag ctccccctcc ttccgaggtg cagctgctgg agtccggagg 900

aggactggtg cagccaggag gctccctgaa gctgagctgt gctgcctctg gctttacctt 960

caacacatat gccatgaatt gggtgcggca ggctccaggc aagggactgg agtgggtggc 1020

taggatcagg tctaagtaca acaattatgc cacctactat gctgattccg tgaaggacag 1080

gttcaccatc tcccgcgacg atagcaagaa cacagcctac ctgcagatga acaatctgaa 1140

gaccgaggat accgccgtgt actactgcgt gagacatggc aactttggca atagctacgt 1200

gtcctggttc gcttactggg gacagggcac cctggtcaca gtgagctctg gaggaggagg 1260

atctggagga ggaggctccg gaggaggagg aagcgagctg gtggtgaccc aggagccatc 1320

tctgacagtg tcccccggcg gcacagtgac cctgacatgt agatccagca ccggcgccgt 1380

gaccacatcc aactacgcta attgggtgca gcagaagcca ggacaggctc caaggggact 1440

gatcggagga accaacaaga gggctcctgg aacaccagct cggtttagcg gatctctgct 1500

gggaggcaag gctgccctga ccctgtccgg agtgcagcca gaggatgagg ccgagtatta 1560

ttgcgctctg tggtatagca atctgtgggt gttcggagga ggaaccaagc tgacagtgct 1620

ggacaagacc catacatgcc caccatgccc tgcccctgaa gccgccggag gaccttccgt 1680

gttcctgttc cctcccaagc caaaagatca gctgatgatc tctagaaccc ccgaagtcac 1740

ctgcgtggtc gtcgacgtgt cccatgagga ccctgaagtc aagttcaact ggtacgtgga 1800

cggtgtcgaa gtccacaacg ccaagaccaa gcctagggag gagcagtatg ccagcacata 1860

ccgggtggtg tctgtgctga ccgtgctgca tcaggattgg ctgaatggca aggaatataa 1920

atgtaaggtg agcaataagg ctctgccggc tagcattgaa aaaaccattt ccaaggctaa 1980

gggccagccc agggagcctc aggtctacac cctgcctcca tctagagatg aactgaccaa 2040

aaaccaggtg agcctgactt gcctggtcaa aggcttctac cccagcgaca ttgccgtgga 2100

gtgggagtct aatggccagc ctgaaaataa ctacaaaact acccctcctg tgctggactc 2160

tgatggctcc ttctttctgt actctaaact gaccgtggac aagtctcgct ggcagcaggg 2220

taacgtgttt tcttgctccg tgctgcacga ggctctgcat aaccattaca cccagaagag 2280

cctgtctctg tccccaggat agaattc 2307

<210> 61
<211> 743
<212> PRT
<213> Artificial Sequence

<220>

<223>  AB23P10 amino acid sequence

<400>  61

Gln Val Gln Leu Gln Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Gly Trp Ile Arg Gln Pro Pro Gly Lys Ala Leu Glu
        35                  40                  45

Trp Leu Ala His Ile Trp Trp Asp Asp Asp Lys Arg Tyr Asn Pro Ala
    50                  55                  60

Leu Lys Ser Arg Leu Thr Ile Ser Lys Asp Thr Ser Lys Asn Gln Val
65                  70                  75                  80

Phe Leu Thr Met Thr Asn Met Asp Pro Val Asp Thr Ala Thr Tyr Tyr
                85                  90                  95

Cys Ala Arg Met Glu Leu Trp Ser Tyr Tyr Phe Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Glu Asn Val Leu Thr Gln Ser Pro Ala
    130                 135                 140

Thr Leu Ser Val Thr Pro Gly Glu Lys Ala Thr Leu Ser Cys Ser Ala
145                 150                 155                 160

Ser Ser Ser Val Ser Tyr Met His Trp Tyr Gln Gln Lys Pro Gly Gln
                165                 170                 175

Ala Pro Arg Leu Leu Ile Tyr Asp Thr Ser Lys Leu Ala Ser Gly Ile
            180                 185                 190

Pro Ala Arg Phe Ser Gly Ser Gly Ser Gly Thr Asp His Thr Leu Thr
        195                 200                 205

Ile Ser Ser Leu Glu Ala Glu Asp Val Ala Val Tyr Tyr Cys Phe Gln
    210                 215                 220

Gly Ser Val Tyr Pro Phe Thr Phe Gly Gln Gly Thr Lys Leu Glu Ile
225                 230                 235                 240

Lys Gly Gly Gly Gly Gly Gly Ser Gly Gly Gly Gly Ser Gly Gly Gly
                245                 250                 255

Gly Ser Ser Ser Ser Ser Lys Ala Pro Pro Pro Ser Glu Val Gln Leu
            260                 265                 270

Leu Glu Ser Gly Gly Gly Leu Val Gln Pro Gly Gly Ser Leu Lys Leu
        275                 280                 285

Ser Cys Ala Ala Ser Gly Phe Thr Phe Asn Thr Tyr Ala Met Asn Trp
    290                 295                 300

Val Arg Gln Ala Pro Gly Lys Gly Leu Glu Trp Val Ala Arg Ile Arg
305                 310                 315                 320

Ser Lys Tyr Asn Asn Tyr Ala Thr Tyr Tyr Ala Asp Ser Val Lys Asp
                325                 330                 335

Arg Phe Thr Ile Ser Arg Asp Asp Ser Lys Asn Thr Ala Tyr Leu Gln
            340                 345                 350

Met Asn Asn Leu Lys Thr Glu Asp Thr Ala Val Tyr Tyr Cys Val Arg
        355                 360                 365

His Gly Asn Phe Gly Asn Ser Tyr Val Ser Trp Phe Ala Tyr Trp Gly
    370                 375                 380

Gln Gly Thr Leu Val Thr Val Ser Ser Gly Gly Gly Gly Ser Gly Gly
385                 390                 395                 400

Gly Gly Ser Gly Gly Gly Gly Ser Glu Leu Val Val Thr Gln Glu Pro
                405                 410                 415

Ser Leu Thr Val Ser Pro Gly Gly Thr Val Thr Leu Thr Cys Arg Ser
            420                 425                 430

Ser Thr Gly Ala Val Thr Thr Ser Asn Tyr Ala Asn Trp Val Gln Gln
        435                 440                 445

Lys Pro Gly Gln Ala Pro Arg Gly Leu Ile Gly Gly Thr Asn Lys Arg
    450                 455                 460

Ala Pro Gly Thr Pro Ala Arg Phe Ser Gly Ser Leu Leu Gly Gly Lys
465                 470                 475                 480

Ala Ala Leu Thr Leu Ser Gly Val Gln Pro Glu Asp Glu Ala Glu Tyr
                485                 490                 495

Tyr Cys Ala Leu Trp Tyr Ser Asn Leu Trp Val Phe Gly Gly Gly Thr
            500                 505                 510

Lys Leu Thr Val Leu Asp Lys Thr His Thr Cys Pro Pro Cys Pro Ala
        515                 520                 525

Pro Glu Ala Ala Gly Gly Pro Ser Val Phe Leu Phe Pro Pro Lys Pro
    530                 535                 540

Lys Asp Gln Leu Met Ile Ser Arg Thr Pro Glu Val Thr Cys Val Val
545                 550                 555                 560

Val Asp Val Ser His Glu Asp Pro Glu Val Lys Phe Asn Trp Tyr Val
                565                 570                 575

Asp Gly Val Glu Val His Asn Ala Lys Thr Lys Pro Arg Glu Glu Gln
            580                 585                 590

Tyr Ala Ser Thr Tyr Arg Val Val Ser Val Leu Thr Val Leu His Gln
        595                 600                 605

Asp Trp Leu Asn Gly Lys Glu Tyr Lys Cys Lys Val Ser Asn Lys Ala
    610                 615                 620

Leu Pro Ala Ser Ile Glu Lys Thr Ile Ser Lys Ala Lys Gly Gln Pro
625                 630                 635                 640

Arg Glu Pro Gln Val Tyr Thr Leu Pro Pro Ser Arg Asp Glu Leu Thr
                645                 650                 655

Lys Asn Gln Val Ser Leu Thr Cys Leu Val Lys Gly Phe Tyr Pro Ser
            660                 665                 670

Asp Ile Ala Val Glu Trp Glu Ser Asn Gly Gln Pro Glu Asn Asn Tyr
        675                 680                 685

Lys Thr Thr Pro Pro Val Leu Asp Ser Asp Gly Ser Phe Phe Leu Tyr
    690                 695                 700

Ser Lys Leu Thr Val Asp Lys Ser Arg Trp Gln Gln Gly Asn Val Phe
705                 710                 715                 720

Ser Cys Ser Val Leu His Glu Ala Leu His Asn His Tyr Thr Gln Lys
                725                 730                 735

Ser Leu Ser Leu Ser Pro Gly

            740

<210> 62
<211> 2307
<212> DNA
<213> Artificial Sequence

<220>
<223> AB23P10 nucleotide sequence

<400> 62
actagtcgcc accatgagga gcctcggggc cctgctcttg ctgctgagcg cctgcctggc 60

ggtgagcgct caggtgcagc tgcaggagtc cggacctgcc ctggtgaagc caacacagac 120

cctgacactg acctgtacct tctccggctt ctctctgtcc accagcggaa tgggagtggg 180

atggatcagg cagccacctg gcaaggccct ggagtggctg gctcacatct ggtgggacga 240

tgacaagaga tacaacccag ctctgaagtc tcgcctgacc atcagcaagg atacatctaa 300

gaaccaggtg tttctgacca tgacaaatat ggaccccgtg gacaccgcca catactattg 360

cgctcgcatg gagctgtggt cctactattt cgactactgg ggccagggta cactggtcac 420

cgtgagctct ggtggtggtg gttccggcgg cggcggttct ggcggcggcg gcagcgagaa 480

cgtgctgaca cagtctccag ccaccctgtc cgtgacacct ggagagaagg ccaccctgtc 540

ctgttccgct tcttcttccg tgagctacat gcactggtat cagcagaagc caggacaggc 600

tccaaggctg ctgatctacg acacctccaa gctggctagc ggcattcctg ctcggttttc 660

tggttctggc agcggcaccg atcataccct gacaatctcc agcctggagg ctgaagatgt 720

ggctgtctac tattgtttcc agggcagcgt gtatcccttc accttcggcc agggtaccaa 780

gctggagatc aagggtggcg gcggtggagg atccggcggt ggaggtagcg gcggaggcgg 840

tagctccagc tctagtaaag ctccccctcc ttccgaggtg cagctgctgg agtccggagg 900

aggactggtg cagccaggag gctccctgaa gctgagctgt gctgcctctg gctttacctt 960

caacacatat gccatgaatt gggtgcggca ggctccaggc aagggactgg agtgggtggc 1020

taggatcagg tctaagtaca acaattatgc cacctactat gctgattccg tgaaggacag 1080

gttcaccatc tcccgcgacg atagcaagaa cacagcctac ctgcagatga acaatctgaa 1140

gaccgaggat accgccgtgt actactgcgt gagacatggc aactttggca atagctacgt 1200

gtcctggttc gcttactggg gacagggcac cctggtcaca gtgagctctg gaggaggagg 1260

atctggagga ggaggctccg gaggaggagg aagcgagctg gtggtgaccc aggagccatc 1320

tctgacagtg tcccccggcg gcacagtgac cctgacatgt agatccagca ccggcgccgt 1380

gaccacatcc aactacgcta attgggtgca gcagaagcca ggacaggctc caaggggact 1440

gatcggagga accaacaaga gggctcctgg aacaccagct cggtttagcg gatctctgct 1500

gggaggcaag gctgccctga ccctgtccgg agtgcagcca gaggatgagg ccgagtatta 1560

ttgcgctctg tggtatagca atctgtgggt gttcggagga ggaaccaagc tgacagtgct 1620

ggacaagacc catacatgcc caccatgccc tgcccctgaa gccgccggag gaccttccgt 1680

gttcctgttc cctcccaagc caaaagatca gctgatgatc tctagaaccc ccgaagtcac 1740

ctgcgtggtc gtcgacgtgt cccatgagga ccctgaagtc aagttcaact ggtacgtgga 1800

cggtgtcgaa gtccacaacg ccaagaccaa gcctagggag gagcagtatg ccagcacata 1860

ccgggtggtg tctgtgctga ccgtgctgca tcaggattgg ctgaatggca aggaatataa 1920

atgtaaggtg agcaataagg ctctgccggc tagcattgaa aaaaccattt ccaaggctaa 1980

gggccagccc agggagcctc aggtctacac cctgcctcca tctagagatg aactgaccaa 2040

aaaccaggtg agcctgactt gcctggtcaa aggcttctac cccagcgaca ttgccgtgga 2100

gtgggagtct aatggccagc ctgaaaataa ctacaaaact acccctcctg tgctggactc 2160

tgatggctcc ttctttctgt actctaaact gaccgtggac aagtctcgct ggcagcaggg 2220

taacgtgttt tcttgctccg tgctgcacga ggctctgcat aaccattaca cccagaagag 2280

cctgtctctg tccccaggat agaattc 2307

<210> 63
<211> 107
<212> PRT
<213> Artificial Sequence

<220>
<223> anti-CD19 antibody_VL

<400> 63

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Val Ala Val Tyr Tyr Cys Phe Gln Gly Ser Val Tyr Pro Phe Thr
                85                  90                  95

Phe Gly Gln Gly Thr Lys Leu Glu Ile Lys Arg

100                                    105